# EUROPEAN PATENT APPLICATION

(11) **EP 1 273 657 A1**
(43) Date of publication of application: **08.01.2003**
(21) Application number: 01915696.7
(22) Date of filing: 22.03.2001
(51) Int. Cl.: C12N 15/12, C07K 14/47, C07K 16/18, C12Q 1/68, A61K 38/17, A61K 31/711, A61K 48/00, A61K 45/00, A61P 3/10, A61P 3/04, A61P 35/00, A61P 9/10, A61P 3/06, A61P 25/00, A61P 43/00, A61K 39/395, G01N 33/53, G01N 33/50, G01N 33/15

(54) **NOVEL PROTEIN, PROCESS FOR PRODUCING THE SAME AND USE THEREOF**

(30) Priority: 24.03.2000 JP 2000088595
(71) Applicant: Takeda Chemical Industries, Ltd., Osaka-shi, Osaka 541-0045 (JP)
(72) Inventor: TANIYAMA, Yoshio, Tsukuba-shi, Ibaraki 305-0821 (JP); KITA, Shunbun, Nishinomiya-shi, Hyogo 662-0823 (JP); SATOMI, Tomoko, Tsukuba-shi, Ibaraki 305-0035 (JP)
(74) Representative: Lewin, John Harvey
(86) International application number: JP0102279
(87) International publication number: WO01070974

(57) **Abstract**

The present invention relates to a novel SSD(sterol-sensing domain)-containing protein derived from human liver, human testis and human brain, or a salt thereof, and a DNA encoding the same. Studies on the tissue-specificity of the protein expression, the expression change depending the intracellular cholesterol level, production of the transformant, and the site-specific expression in brain regions of a patient with Alzheimer's disease are disclosed.

SEQUENCE LISTING
<110> Takeda Chemical Industries, Ltd.
<120> Novel Protein, its production and use
<130> 2703WOOP
<150> JP2000-088595
<151> 2000-03-24
<160> 59
<210> 1
<211> 21
<212> DNA
<213> Artificial Sequence
<220>
<223>
<400> 1
ccagtcaggc cagggttgtc a 21
<210> 2
<211> 22
<212> DNA
<213> Artificial Sequence
<220>
<223>
<400> 2
cacacggctg caggagtcat ag 22
<210> 3

<211> 21
<212> DNA
<213> Artificial Sequence
<220>
<223>
<400> 3
cctctacacc ggccccaaca c                                                21
<210> 4
<211> 22
<212> DNA
<213> Artificial Sequence
<220>
<223>
<400> 4
gggcccctag gaagaagcag at                                               22
<210> 5
<211> 23
<212> DNA
<213> Artificial Sequence
<220>
<223>
<400> 5
ccatgggctt cttctcctac ttg                                              23

<210> 6

<211> 24

<212> DNA

<213> Artificial Sequence

<220>

<223>

<400> 6

tcaaggggca gtcacaagga agat                                              24

<210> 7

<211> 3999

<212> DNA

<213> Human

<400> 7
   atggcggagg ccggcctgag gggctggctg ctgtgggccc tgctcctgcg cttggcccag    60
   agtgagcctt acacaaccat ccaccagcct ggctactgcg ccttctatga cgaatgtggg   120
   aagaacccag agctgtctgg aagcctcatg acactctcca acgtgtcctg cctgtccaac   180
   acgccggccc gcaagatcac aggtgatcac ctgatcctat tacagaagat ctgcccccgc   240
   ctctacaccg gccccaacac ccaagcctgc tgctccgcca agcagctggt atcactggaa   300
   gcgagtctgt cgatcaccaa ggccctcctc acccgctgcc cagcctgctc tgacaatttt   360
   gtgaacctgc actgccacaa cacgtgcagc cccaatcaga gcctcttcat caatgtgacc   420
   cgcgtggccc agctaggggc tggacaactc ccagctgtgg tggcctatga ggccttctac   480
   cagcatagct ttgccgagca gagctatgac tcctgcagcc gtgtgcgcgt ccctgcagct   540
   gccacgctgg ctgtgggcac catgtgtggc gtgtatggct ctgccctttg caatgcccag   600
   cgctggctca acttccaggg agacacaggc aatggtctgg ccccactgga catcaccttc 660
   cacctcttgg agcctggcca ggccgtgggg agtgggattc agcctctgaa tgagggggtt 720
   gcacgttgca atgagtccca aggtgacgac gtggcgacct gctcctgcca agactgtgct 780
   gcatcctgtc ctgccatagc ccgcccccag gccctcgact ccaccttcta cctgggccag 840
   atgccgggca gtctggtcct catcatcatc ctctgctctg tcttcgctgt ggtcaccatc 900
   ctgcttgtgg gattccgtgt ggcccccgcc agggacaaaa gcaagatggt ggaccccaag 960
   aagggcacca gcctctctga caagctcagc ttctccaccc acaccctcct tggccagttc 1020
   ttccagggct ggggcacgtg ggtggcttcg tggcctctga ccatcttggt gctatctgtc 1080
   atcccggtgg tggccttggc agcgggcctg gtctttacag aactcactac ggaccccgtg 1140
   gagctgtggt cggcccccaa cagccaagcc cggagtgaga aagctttcca tgaccagcat 1200
   ttcggcccct tcttccgaac caaccaggtg atcctgacgg ctcctaaccg gtccagctac 1260
   aggtatgact ctctgctgct ggggcccaag aacttcagcg gaatcctgga cctggacttg 1320
   ctgctggagc tgctagagct gcaggagagg ctgcggcacc tccaggtatg gtcgcccgaa 1380
   gcacagcgca acatctccct gcaggacatc tgctacgccc ccctcaatcc ggacaatacc 1440
   agtctctacg actgctgcat caacagcctc ctgcagtatt tccagaacaa ccgcacgctc 1500
   ctgctgctca cagccaacca gacactgatg gggcagacct cccaagtcga ctggaaggac 1560
   cattttctgt actgtgccaa tgccccgctc accttcaagg atggcacagc cctggccctg 1620
   agctgcatgg ctgactacgg ggcccctgtc ttccccttcc ttgccattgg ggggtacaaa 1680
   ggaaaggact attctgaggc agaggccctg atcatgacgt tctccctcaa caattaccct 1740
   gccggggacc cccgtctggc ccaggccaag ctgtgggagg aggccttctt agaggaaatg 1800
   cgagccttcc agcgtcggat ggctggcatg ttccaggtca cgttcatggc tgagcgctct 1860
   ctggaagacg agatcaatcg caccacagct gaagacctgc ccatctttgc caccagctac 1920
   attgtcatat tcctgtacat ctctctggcc ctgggcagct attccagctg gagccgagtg 1980
   atggtggact ccaaggccac gctgggcctc ggcggggtgg ccgtggtcct gggagcagtc 2040
   atggctgcca tgggcttctt ctcctacttg ggtatccgct cctccctggt catcctgcaa 2100
   gtggttcctt tcctggtgct gtccgtgggg gctgataaca tcttcatctt tgttctcgag 2160
   taccagaggc tgccccggag gcctggggag ccacgagagg tccacattgg gcgagcccta 2220
   ggcagggtgg ctcccagcat gctgttgtgc agcctctctg aggccatctg cttcttccta 2280
   ggggccctga cccccatgcc agctgtgcgg acctttgccc tgacctctgg ccttgcagtg 2340
   atccttgact tcctcctgca gatgtcagcc tttgtggccc tgctctccct ggacagcaag 2400
   aggcaggagg cctcccggtt ggacgtctgc tgctgtgtca agccccagga gctgcccccg 2460
   cctggccagg gagaggggct cctgcttggc ttcttccaaa aggcttatgc ccccttcctg 2520
   ctgcactgga tcactcgagg tgttgtgctg ctgctgtttc tcgccctgtt cggagtgagc 2580
   ctctactcca tgtgccacat cagcgtggga ctggaccagg agctggccct gcccaaggac 2640
   tcgtacctgc ttgactattt cctctttctg aaccgctact tcgaggtggg ggccccggtg 2700
   tactttgtta ccaccttggg ctacaacttc tccagcgagg ctgggatgaa tgccatctgc 2760
   tccagtgcag gctgcaacaa cttctccttc acccagaaga tccagtatgc cacagagttc 2820
   cctgagcagt cttacctggc catccctgcc tcctcctggg tggatgactt cattgactgg 2880
   ctgaccccgt cctcctgctg ccgcctttat atatctggcc ccaataagga caagttctgc 2940
   ccctcgaccg tcaactctct gaactgccta aagaactgca tgagcatcac gatgggctct 3000
   gtgaggccct cggtggagca gttccataag tatcttccct ggttcctgaa cgaccggccc 3060
   aacatcaaat gtcccaaagg cggcctggca gcatacagca cctctgtgaa cttgacttca 3120
   gatggccagg ttttagcctc caggttcatg gcctatcaca agcccctgaa aaactcacag 3180
   gattacacag aagctctgcg ggcagctcga gagctggcag ccaacatcac tgctgacctg 3240
   cggaaagtgc ctggaacaga cccggctttt gaggtcttcc cctacacgat caccaatgtg 3300
   ttttatgagc agtacctgac catcctccct gaggggctct tcatgctcag cctctgcctt 3360
   gtgcccacct tcgctgtctc ctgcctcctg ctgggcctgg acctgcgctc cggcctcctc 3420
   aacctgctct ccattgtcat gatcctcgtg gacactgtcg gcttcatggc cctgtggggc 3480
   atcagttaca atgctgtgtc cctcatcaac ctggtctcgg cggtgggcat gtctgtggag 3540
   tttgtgtccc acattacccg ctcctttgcc atcagcacca agcccacctg gctggagagg 3600
   gccaaagagg ccaccatctc tatgggaagt gcggtgtttg caggtgtggc catgaccaac 3660
   ctgcctggca tccttgtcct gggcctcgcc aaggcccagc tcattcagat cttcttcttc 3720
   cgcctcaacc tcctgatcac tctgctgggc ctgctgcatg gcttggtctt cctgcccgtc 3780
   atcctcagct acgtggggcc tgacgttaac ccggctctgg cactggagca gaagcgggct 3840
   gaggaggcgg tggcagcagt catggtggcc tcttgcccaa atcacccctc ccgagtctcc 3900
   acagctgaca acatctatgt caaccacagc tttgaaggtt ctatcaaagg tgctggtgcc 3960
   atcagcaact tcttgcccaa caatgggcgg cagttctga 3999
<210> 8
<211> 194
<212> PRT
<213> Human
<400> 8
Phe Met Ala Glu Arg Ser Leu Glu Asp Glu Ile Asn Arg Thr Thr Ala
                5                   10                  15
Glu Asp Leu Pro Ile Phe Ala Thr Ser Tyr Ile Val Ile Phe Leu Tyr
            20                  25                  30
Ile Ser Leu Ala Leu Gly Ser Tyr Ser Ser Trp Ser Arg Val Met Val
        35                  40                  45
Asp Ser Lys Ala Thr Leu Gly Leu Gly Gly Val Ala Val Val Leu Gly

    50                  55                  60

Ala Val Met Ala Ala Met Gly Phe Phe Ser Tyr Leu Gly Ile Arg Ser

65                  70                  75                  80

Ser Leu Val Ile Leu Gln Val Val Pro Phe Leu Val Leu Ser Val Gly

                85                  90                  95

Ala Asp Asn Ile Phe Ile Phe Val Leu Glu Tyr Gln Arg Leu Pro Arg

            100                 105                 110

Arg Pro Gly Glu Pro Arg Glu Val His Ile Gly Arg Ala Leu Gly Arg

        115                 120                 125

Val Ala Pro Ser Met Leu Leu Cys Ser Leu Ser Glu Ala Ile Cys Phe

    130                 135                 140

Phe Leu Gly Ala Leu Thr Pro Met Pro Ala Val Arg Thr Phe Ala Leu

145                 150                 155                 160

Thr Ser Gly Leu Ala Val Ile Leu Asp Phe Leu Leu Gln Met Ser Ala

                165                 170                 175

Phe Val Ala Leu Leu Ser Leu Asp Ser Lys Arg Gln Glu Ala Ser Arg

            180                 185                 190

Leu Asp

<210> 9

<211> 1332

<212> PRT

<213> Human

<400> 9

Met Ala Glu Ala Gly Leu Arg Gly Trp Leu Leu Trp Ala Leu Leu Leu
                5                   10                  15
Arg Leu Ala Gln Ser Glu Pro Tyr Thr Thr Ile His Gln Pro Gly Tyr
            20                  25                  30
Cys Ala Phe Tyr Asp Glu Cys Gly Lys Asn Pro Glu Leu Ser Gly Ser
        35                  40                  45
Leu Met Thr Leu Ser Asn Val Ser Cys Leu Ser Asn Thr Pro Ala Arg
    50                  55                  60
Lys Ile Thr Gly Asp His Leu Ile Leu Leu Gln Lys Ile Cys Pro Arg
65                  70                  75                  80
Leu Tyr Thr Gly Pro Asn Thr Gln Ala Cys Cys Ser Ala Lys Gln Leu
                85                  90                  95
Val Ser Leu Glu Ala Ser Leu Ser Ile Thr Lys Ala Leu Leu Thr Arg
            100                 105                 110
Cys Pro Ala Cys Ser Asp Asn Phe Val Asn Leu His Cys His Asn Thr
        115                 120                 125
Cys Ser Pro Asn Gln Ser Leu Phe Ile Asn Val Thr Arg Val Ala Gln
    130                 135                 140
Leu Gly Ala Gly Gln Leu Pro Ala Val Val Ala Tyr Glu Ala Phe Tyr
145                 150                 155                 160
Gln His Ser Phe Ala Glu Gln Ser Tyr Asp Ser Cys Ser Arg Val Arg
                165                 170                 175
Val Pro Ala Ala Ala Thr Leu Ala Val Gly Thr Met Cys Gly Val Tyr

            180                 185                 190
Gly Ser Ala Leu Cys Asn Ala Gln Arg Trp Leu Asn Phe Gln Gly Asp
        195                 200                 205
Thr Gly Asn Gly Leu Ala Pro Leu Asp Ile Thr Phe His Leu Leu Glu
    210                 215                 220
Pro Gly Gln Ala Val Gly Ser Gly Ile Gln Pro Leu Asn Glu Gly Val
225                 230                 235                 240
Ala Arg Cys Asn Glu Ser Gln Gly Asp Asp Val Ala Thr Cys Ser Cys
                245                 250                 255
Gln Asp Cys Ala Ala Ser Cys Pro Ala Ile Ala Arg Pro Gln Ala Leu
            260                 265                 270
Asp Ser Thr Phe Tyr Leu Gly Gln Met Pro Gly Ser Leu Val Leu Ile
        275                 280                 285
Ile Ile Leu Cys Ser Val Phe Ala Val Val Thr Ile Leu Leu Val Gly
    290                 295                 300
Phe Arg Val Ala Pro Ala Arg Asp Lys Ser Lys Met Val Asp Pro Lys
305                 310                 315                 320
Lys Gly Thr Ser Leu Ser Asp Lys Leu Ser Phe Ser Thr His Thr Leu
                325                 330                 335
Leu Gly Gln Phe Phe Gln Gly Trp Gly Thr Trp Val Ala Ser Trp Pro
            340                 345                 350
Leu Thr Ile Leu Val Leu Ser Val Ile Pro Val Val Ala Leu Ala Ala
        355                 360                 365

Gly Leu Val Phe Thr Glu Leu Thr Thr Asp Pro Val Glu Leu Trp Ser
    370                 375                 380
Ala Pro Asn Ser Gln Ala Arg Ser Glu Lys Ala Phe His Asp Gln His
385                 390                 395                 400
Phe Gly Pro Phe Phe Arg Thr Asn Gln Val Ile Leu Thr Ala Pro Asn
                405                 410                 415
Arg Ser Ser Tyr Arg Tyr Asp Ser Leu Leu Leu Gly Pro Lys Asn Phe
            420                 425                 430
Ser Gly Ile Leu Asp Leu Asp Leu Leu Leu Glu Leu Leu Glu Leu Gln
        435                 440                 445
Glu Arg Leu Arg His Leu Gln Val Trp Ser Pro Glu Ala Gln Arg Asn
    450                 455                 460
Ile Ser Leu Gln Asp Ile Cys Tyr Ala Pro Leu Asn Pro Asp Asn Thr
465                 470                 475                 480
Ser Leu Tyr Asp Cys Cys Ile Asn Ser Leu Leu Gln Tyr Phe Gln Asn
                485                 490                 495
Asn Arg Thr Leu Leu Leu Leu Thr Ala Asn Gln Thr Leu Met Gly Gln
            500                 505                 510
Thr Ser Gln Val Asp Trp Lys Asp His Phe Leu Tyr Cys Ala Asn Ala
        515                 520                 525
Pro Leu Thr Phe Lys Asp Gly Thr Ala Leu Ala Leu Ser Cys Met Ala
    530                 535                 540
Asp Tyr Gly Ala Pro Val Phe Pro Phe Leu Ala Ile Gly Gly Tyr Lys

545                 550                 555                 560

Gly Lys Asp Tyr Ser Glu Ala Glu Ala Leu Ile Met Thr Phe Ser Leu

                565                 570                 575

Asn Asn Tyr Pro Ala Gly Asp Pro Arg Leu Ala Gln Ala Lys Leu Trp

            580                 585                 590

Glu Glu Ala Phe Leu Glu Glu Met Arg Ala Phe Gln Arg Arg Met Ala

        595                 600                 605

Gly Met Phe Gln Val Thr Phe Met Ala Glu Arg Ser Leu Glu Asp Glu

    610                 615                 620

Ile Asn Arg Thr Thr Ala Glu Asp Leu Pro Ile Phe Ala Thr Ser Tyr

625                 630                 635                 640

Ile Val Ile Phe Leu Tyr Ile Ser Leu Ala Leu Gly Ser Tyr Ser Ser

                645                 650                 655

Trp Ser Arg Val Met Val Asp Ser Lys Ala Thr Leu Gly Leu Gly Gly

            660                 665                 670

Val Ala Val Val Leu Gly Ala Val Met Ala Ala Met Gly Phe Phe Ser

        675                 680                 685

Tyr Leu Gly Ile Arg Ser Ser Leu Val Ile Leu Gln Val Val Pro Phe

    690                 695                 700

Leu Val Leu Ser Val Gly Ala Asp Asn Ile Phe Ile Phe Val Leu Glu

705                 710                 715                 720

Tyr Gln Arg Leu Pro Arg Arg Pro Gly Glu Pro Arg Glu Val His Ile

                725                 730                 735

Gly Arg Ala Leu Gly Arg Val Ala Pro Ser Met Leu Leu Cys Ser Leu
            740                 745                 750
Ser Glu Ala Ile Cys Phe Phe Leu Gly Ala Leu Thr Pro Met Pro Ala
        755                 760                 765
Val Arg Thr Phe Ala Leu Thr Ser Gly Leu Ala Val Ile Leu Asp Phe
    770                 775                 780
Leu Leu Gln Met Ser Ala Phe Val Ala Leu Leu Ser Leu Asp Ser Lys
785                 790                 795                 800
Arg Gln Glu Ala Ser Arg Leu Asp Val Cys Cys Cys Val Lys Pro Gln
                805                 810                 815
Glu Leu Pro Pro Pro Gly Gln Gly Glu Gly Leu Leu Leu Gly Phe Phe
            820                 825                 830
Gln Lys Ala Tyr Ala Pro Phe Leu Leu His Trp Ile Thr Arg Gly Val
        835                 840                 845
Val Leu Leu Leu Phe Leu Ala Leu Phe Gly Val Ser Leu Tyr Ser Met
    850                 855                 860
Cys His Ile Ser Val Gly Leu Asp Gln Glu Leu Ala Leu Pro Lys Asp
865                 870                 875                 880
Ser Tyr Leu Leu Asp Tyr Phe Leu Phe Leu Asn Arg Tyr Phe Glu Val
                885                 890                 895
Gly Ala Pro Val Tyr Phe Val Thr Thr Leu Gly Tyr Asn Phe Ser Ser
            900                 905                 910
Glu Ala Gly Met Asn Ala Ile Cys Ser Ser Ala Gly Cys Asn Asn Phe

        915                 920                 925

Ser Phe Thr Gln Lys Ile Gln Tyr Ala Thr Glu Phe Pro Glu Gln Ser

    930                 935                 940

Tyr Leu Ala Ile Pro Ala Ser Ser Trp Val Asp Asp Phe Ile Asp Trp

945                 950                 955                 960

Leu Thr Pro Ser Ser Cys Cys Arg Leu Tyr Ile Ser Gly Pro Asn Lys

                965                 970                 975

Asp Lys Phe Cys Pro Ser Thr Val Asn Ser Leu Asn Cys Leu Lys Asn

            980                 985                 990

Cys Met Ser Ile Thr Met Gly Ser Val Arg Pro Ser Val Glu Gln Phe

        995                 1000                1005

His Lys Tyr Leu Pro Trp Phe Leu Asn Asp Arg Pro Asn Ile Lys Cys

    1010                1015                1020

Pro Lys Gly Gly Leu Ala Ala Tyr Ser Thr Ser Val Asn Leu Thr Ser

1025                1030                1035                1040

Asp Gly Gln Val Leu Ala Ser Arg Phe Met Ala Tyr His Lys Pro Leu

                1045                1050                1055

Lys Asn Ser Gln Asp Tyr Thr Glu Ala Leu Arg Ala Ala Arg Glu Leu

            1060                1065                1070

Ala Ala Asn Ile Thr Ala Asp Leu Arg Lys Val Pro Gly Thr Asp Pro

        1075                1080                1085

Ala Phe Glu Val Phe Pro Tyr Thr Ile Thr Asn Val Phe Tyr Glu Gln

    1090                1095                1100

Tyr Leu Thr Ile Leu Pro Glu Gly Leu Phe Met Leu Ser Leu Cys Leu
1105                1110                1115                1120
Val Pro Thr Phe Ala Val Ser Cys Leu Leu Leu Gly Leu Asp Leu Arg
                1125                1130                1135
Ser Gly Leu Leu Asn Leu Leu Ser Ile Val Met Ile Leu Val Asp Thr
            1140                1145                1150
Val Gly Phe Met Ala Leu Trp Gly Ile Ser Tyr Asn Ala Val Ser Leu
        1155                1160                1165
Ile Asn Leu Val Ser Ala Val Gly Met Ser Val Glu Phe Val Ser His
    1170                1175                1180
Ile Thr Arg Ser Phe Ala Ile Ser Thr Lys Pro Thr Trp Leu Glu Arg
1185                1190                1195                1200
Ala Lys Glu Ala Thr Ile Ser Met Gly Ser Ala Val Phe Ala Gly Val
                1205                1210                1215
Ala Met Thr Asn Leu Pro Gly Ile Leu Val Leu Gly Leu Ala Lys Ala
            1220                1225                1230
Gln Leu Ile Gln Ile Phe Phe Phe Arg Leu Asn Leu Leu Ile Thr Leu
        1235                1240                1245
Leu Gly Leu Leu His Gly Leu Val Phe Leu Pro Val Ile Leu Ser Tyr
    1250                1255                1260
Val Gly Pro Asp Val Asn Pro Ala Leu Ala Leu Glu Gln Lys Arg Ala
1265                1270                1275                1280
Glu Glu Ala Val Ala Ala Val Met Val Ala Ser Cys Pro Asn His Pro

1285                    1290                    1295

Ser Arg Val Ser Thr Ala Asp Asn Ile Tyr Val Asn His Ser Phe Glu

1300                    1305                    1310

Gly Ser Ile Lys Gly Ala Gly Ala Ile Ser Asn Phe Leu Pro Asn Asn

1315                    1320                    1325

Gly Arg Gln Phe

    1330

<210> 10

<211> 23

<212> DNA

<213> Artificial Sequence

<220>

<223>

<400> 10

aaggcggcca ccccattcag gat                                              23

<210> 11

<211> 20

<212> DNA

<213> Artificial Sequence

<220>

<223>

<400> 11

tatgaagtgc gcaggacgtt                                                  20

<210> 12

<211> 19

<212> DNA

<213> Artificial Sequence

<220>

<223>

<400> 12

tgcgggcagg ggaatctta 19

<210> 13

<211> 26

<212> DNA

<213> Artificial Sequence

<220>

<223>

<400> 13

cttcttctgc atcatcgccc catttg 26

<210> 14

<211> 22

<212> DNA

<213> Artificial Sequence

<220>

<223>

<400> 14

ccaaaggtgc aagtgtccag ag 22
<210> 15
<211> 3264
<212> DNA
<213> Human
<400> 15
atggaccacc caggcttccg ggagttctgc tggaagcccc acgaggtgct caaggatctg 60
ccgctgggct cctactccta ctgctcgccc cccagctcgc tcatgaccta cttttttccc 120
accgagaggg gcggcaagat ctactatgac ggcatgggcc aggacctggc ggacatccgg 180
ggctccctgg agctggccat gactcaccct gagttctact ggtatgtgga tgagggcctc 240
tctgcagaca atctgaagag ctccctcctg cgcagtgaga tcctgtttgg agcacccctg 300
cccaactact actcagtaga tgaccgctgg gaggaacaac gggctaagtt tcagagcttc 360
gtggtcacct acgtggccat gctggccaag cagtctacca gcaaagtcca ggttctctat 420
ggggggacag acctgtttga ctatgaagtg cgcaggacgt tcaacaatga catgctcctg 480
gccttcatca gcagcagctg cattgctgcc ctggtctaca tcctcacctc ctgctcagtg 540
ttcctgtcct tctttgggat tgccagcatt ggtctcagct gcctggtggc cctcttcctg 600
taccacgtgg tctttggtat ccagtacttg ggcatcctga atggggtggc cgccttcgtg 660
atcgtgggca ttggtgtgga cgatgtcttt gtgttcatca acacctaccg ccaggccacc 720
cacctggaag acccacagct gcgcatgatc cacaccgtcc aaactgcagg caaggccacc 780
ttcttcacct ccctgaccac agccgccgcc tacgcagcta acgtcttctc ccagatccca 840
gccgtccacg actttggcct gttcatgtct ctcatcgtgt cctgttgctg gctggccgtg 900
cttgtcacca tgcctgcagc tctgggcctc tggagcctct acctggcacc actggagagc 960
tcctgccaga ccagctgcca ccagaattgc agccggaaga cctccctgca cttccccgga 1020

gacgtgtttg ccgctcccga gcaggttgga ggcagccctg cccagggccc cataccctac 1080
ctggatgatg acatcccctt gctggaggtc gaggaagagc cagtgtcact ggagctggga 1140
gacgtgtccc tggtgtctgt gtcccccgag ggtctgcagc cagcctccaa cacgggcagc 1200
cgcggccatc tcatcgtgca gctgcaggag ctgctgcacc actgggtcct gtggtcagcc 1260
gtcaagagcc gctgggtgat tgtggggctg ttcgtctcca tcctcatctt gtccctggtg 1320
ttcgccagcc ggctccgccc cgccagccgg gccccgctac tcttccggcc tgataccaac 1380
atccaggtgc tgctggacct caagtacaac ctgagcgccg agggcatctc ctgcatcacc 1440
tgttcaggtc tgttccagga gaagccccac agcctgcaga acaacatccg gacgtccctg 1500
gagaagaaga ggcgaggctc aggggtcccc tgggctagcc ggcctgaggc caccctgcag 1560
gatttcccag gcaccgtgta catctctaaa gtgaagagtc aaggccaccc cgctgtctac 1620
aggctctccc tcaatgccag cctgcctgct ccttggcagg ctgtgtcgcc tggggatgga 1680
gaggtgccct ccttccaggt gtatagagcg ccttttggta acttcaccaa gaagctgacc 1740
gcttgtatgt ctacagtagg gctgctccag gcggcgagcc cctcccgcaa gtggatgctg 1800
acgaccttgg cctgtgatgc caagcggggc tggaagtttg acttcagctt ctacgtggcc 1860
accaaggagc agcagcacac ccggaagctg tacttcgccc agtcccacaa gccccccttc 1920
cacgggcgcg tatgcatggc accccctggc tgcctgctta gctccagccc cgatgggcct 1980
accaaaggct tcttcttcgt gcctagtgag aaagtgccca aggcccgtct ctcagccacc 2040
ttcggcttca acccctgcgt gaacacgggc tgcgggaagc cggcggtgcg gccactagtg 2100
gataccgggg ccatggtctt tgtggtcttc ggcattattg gcgtcaaccg cactcggcag 2160
gtggacaacc acgtcattgg agacccgggt agtgttgtct acgacagcag ctttgacctc 2220
ttcaaggaaa ttgggcacct gtgtcacctc tgcaaggcca tcgcagccaa ctccgagctg 2280
gtgaagccgg gtggggccca gtgcctgcct tcaggctaca gcatctcctc cttcctgcag 2340
atgttgcacc ctgagtgcaa ggagctgccc gagcccaacc tgctcccggg gcagctgtcc 2400

cacggggcag tgggcgtcag ggagggccgc gtgcagtgga tctccatggc tttcgagtcg 2460
accacgtaca agggcaaatc ctccttccag acctactcgg actacctgcg ctgggagagc 2520
ttcctccagc agcagctgca ggccttgccc gagggctcag tcctgcgccg gggcttccag 2580
acctgcgagc actggaagca gatattcatg gaaatcgtag gggtgcagag cgccctgtgc 2640
ggcctggtgc tatccctgct catctgcgtg gccgcggtgg ccgtgttcac cacccacatc 2700
ctgctcctgc tgcccgtgct cctcagcatc ttgggcatcg tgtgcctggt ggtgaccatc 2760
atgtactgga gcggctggga gatgggggct gtggaagcca tctccctgtc catcctcgtt 2820
ggctcctccg tggattactg cgtccacctg gtcgagggct acctgctggc tggagagaac 2880
ctgccccccc accaggccga ggacgcccga acgcagcgcc agtggcgtac gctggaggcc 2940
gtgcggcacg tgggcgtggc catcgtctcc agtgccctca ccacggtcat cgccacagtg 3000
cccctcttct tctgcatcat cgccccattt gccaagttcg gcaagattgt ggcactcaac 3060
acgggcgtgt ccatcctcta cacgctgacc gtcagcaccg ccctgctggg catcatggcg 3120
cccagctctt tcactcggac ccggacttcc ttcctcaagg ccctgggtgc cgtgctgctg 3180
gcaggggccc tggggctggg tgcctgcctc gtgctcctgc agagcggcta taagattccc 3240
ctgcccgcag ggcctcccct atag 3264
<210> 16
<211> 200
<212> PRT
<213> Human
<400> 16
Lys Val Gln Val Leu Tyr Gly Gly Thr Asp Leu Phe Asp Tyr Glu Val
                5                   10                  15
Arg Arg Thr Phe Asn Asn Asp Met Leu Leu Ala Phe Ile Ser Ser Ser

            20                  25                  30
Cys Ile Ala Ala Leu Val Tyr Ile Leu Thr Ser Cys Ser Val Phe Leu
        35                  40                  45
Ser Phe Phe Gly Ile Ala Ser Ile Gly Leu Ser Cys Leu Val Ala Leu
    50                  55                  60
Phe Leu Tyr His Val Val Phe Gly Ile Gln Tyr Leu Gly Ile Leu Asn
65                  70                  75                  80
Gly Val Ala Ala Phe Val Ile Val Gly Ile Gly Val Asp Asp Val Phe
                85                  90                  95
Val Phe Ile Asn Thr Tyr Arg Gln Ala Thr His Leu Glu Asp Pro Gln
            100                 105                 110
Leu Arg Met Ile His Thr Val Gln Thr Ala Gly Lys Ala Thr Phe Phe
        115                 120                 125
Thr Ser Leu Thr Thr Ala Ala Ala Tyr Ala Ala Asn Val Phe Ser Gln
    130                 135                 140
Ile Pro Ala Val His Asp Phe Gly Leu Phe Met Ser Leu Ile Val Ser
145                 150                 155                 160
Cys Cys Trp Leu Ala Val Leu Val Thr Met Pro Ala Ala Leu Gly Leu
                165                 170                 175
Trp Ser Leu Tyr Leu Ala Pro Leu Glu Ser Ser Cys Gln Thr Ser Cys
            180                 185                 190
His Gln Asn Cys Ser Arg Lys Thr
        195                 200

<210> 17

<211> 1087

<212> PRT

<213> Human

<400> 17

Met Asp His Pro Gly Phe Arg Glu Phe Cys Trp Lys Pro His Glu Val

                5                   10                  15

Leu Lys Asp Leu Pro Leu Gly Ser Tyr Ser Tyr Cys Ser Pro Pro Ser

            20                  25                  30

Ser Leu Met Thr Tyr Phe Phe Pro Thr Glu Arg Gly Gly Lys Ile Tyr

        35                  40                  45

Tyr Asp Gly Met Gly Gln Asp Leu Ala Asp Ile Arg Gly Ser Leu Glu

    50                  55                  60

Leu Ala Met Thr His Pro Glu Phe Tyr Trp Tyr Val Asp Glu Gly Leu

65                  70                  75                  80

Ser Ala Asp Asn Leu Lys Ser Ser Leu Leu Arg Ser Glu Ile Leu Phe

                85                  90                  95

Gly Ala Pro Leu Pro Asn Tyr Tyr Ser Val Asp Asp Arg Trp Glu Glu

            100                 105                 110

Gln Arg Ala Lys Phe Gln Ser Phe Val Val Thr Tyr Val Ala Met Leu

        115                 120                 125

Ala Lys Gln Ser Thr Ser Lys Val Gln Val Leu Tyr Gly Gly Thr Asp

    130                 135                 140

Leu Phe Asp Tyr Glu Val Arg Arg Thr Phe Asn Asn Asp Met Leu Leu
145                 150                 155                 160
Ala Phe Ile Ser Ser Ser Cys Ile Ala Ala Leu Val Tyr Ile Leu Thr
                165                 170                 175
Ser Cys Ser Val Phe Leu Ser Phe Phe Gly Ile Ala Ser Ile Gly Leu
            180                 185                 190
Ser Cys Leu Val Ala Leu Phe Leu Tyr His Val Val Phe Gly Ile Gln
        195                 200                 205
Tyr Leu Gly Ile Leu Asn Gly Val Ala Ala Phe Val Ile Val Gly Ile
    210                 215                 220
Gly Val Asp Asp Val Phe Val Phe Ile Asn Thr Tyr Arg Gln Ala Thr
225                 230                 235                 240
His Leu Glu Asp Pro Gln Leu Arg Met Ile His Thr Val Gln Thr Ala
                245                 250                 255
Gly Lys Ala Thr Phe Phe Thr Ser Leu Thr Thr Ala Ala Ala Tyr Ala
            260                 265                 270
Ala Asn Val Phe Ser Gln Ile Pro Ala Val His Asp Phe Gly Leu Phe
        275                 280                 285
Met Ser Leu Ile Val Ser Cys Cys Trp Leu Ala Val Leu Val Thr Met
    290                 295                 300
Pro Ala Ala Leu Gly Leu Trp Ser Leu Tyr Leu Ala Pro Leu Glu Ser
305                 310                 315                 320
Ser Cys Gln Thr Ser Cys His Gln Asn Cys Ser Arg Lys Thr Ser Leu

                325                 330                 335
His Phe Pro Gly Asp Val Phe Ala Ala Pro Glu Gln Val Gly Gly Ser
            340                 345                 350
Pro Ala Gln Gly Pro Ile Pro Tyr Leu Asp Asp Asp Ile Pro Leu Leu
        355                 360                 365
Glu Val Glu Glu Glu Pro Val Ser Leu Glu Leu Gly Asp Val Ser Leu
    370                 375                 380
Val Ser Val Ser Pro Glu Gly Leu Gln Pro Ala Ser Asn Thr Gly Ser
385                 390                 395                 400
Arg Gly His Leu Ile Val Gln Leu Gln Glu Leu Leu His His Trp Val
                405                 410                 415
Leu Trp Ser Ala Val Lys Ser Arg Trp Val Ile Val Gly Leu Phe Val
            420                 425                 430
Ser Ile Leu Ile Leu Ser Leu Val Phe Ala Ser Arg Leu Arg Pro Ala
        435                 440                 445
Ser Arg Ala Pro Leu Leu Phe Arg Pro Asp Thr Asn Ile Gln Val Leu
    450                 455                 460
Leu Asp Leu Lys Tyr Asn Leu Ser Ala Glu Gly Ile Ser Cys Ile Thr
465                 470                 475                 480
Cys Ser Gly Leu Phe Gln Glu Lys Pro His Ser Leu Gln Asn Asn Ile
                485                 490                 495
Arg Thr Ser Leu Glu Lys Lys Arg Arg Gly Ser Gly Val Pro Trp Ala
            500                 505                 510

Ser Arg Pro Glu Ala Thr Leu Gln Asp Phe Pro Gly Thr Val Tyr Ile
        515                 520                 525
Ser Lys Val Lys Ser Gln Gly His Pro Ala Val Tyr Arg Leu Ser Leu
    530                 535                 540
Asn Ala Ser Leu Pro Ala Pro Trp Gln Ala Val Ser Pro Gly Asp Gly
545                 550                 555                 560
Glu Val Pro Ser Phe Gln Val Tyr Arg Ala Pro Phe Gly Asn Phe Thr
                565                 570                 575
Lys Lys Leu Thr Ala Cys Met Ser Thr Val Gly Leu Leu Gln Ala Ala
            580                 585                 590
Ser Pro Ser Arg Lys Trp Met Leu Thr Thr Leu Ala Cys Asp Ala Lys
        595                 600                 605
Arg Gly Trp Lys Phe Asp Phe Ser Phe Tyr Val Ala Thr Lys Glu Gln
    610                 615                 620
Gln His Thr Arg Lys Leu Tyr Phe Ala Gln Ser His Lys Pro Pro Phe
625                 630                 635                 640
His Gly Arg Val Cys Met Ala Pro Pro Gly Cys Leu Leu Ser Ser Ser
                645                 650                 655
Pro Asp Gly Pro Thr Lys Gly Phe Phe Phe Val Pro Ser Glu Lys Val
            660                 665                 670
Pro Lys Ala Arg Leu Ser Ala Thr Phe Gly Phe Asn Pro Cys Val Asn
        675                 680                 685
Thr Gly Cys Gly Lys Pro Ala Val Arg Pro Leu Val Asp Thr Gly Ala

690                 695                 700

Met Val Phe Val Val Phe Gly Ile Ile Gly Val Asn Arg Thr Arg Gln
705                 710                 715                 720

Val Asp Asn His Val Ile Gly Asp Pro Gly Ser Val Val Tyr Asp Ser
                725                 730                 735

Ser Phe Asp Leu Phe Lys Glu Ile Gly His Leu Cys His Leu Cys Lys
            740                 745                 750

Ala Ile Ala Ala Asn Ser Glu Leu Val Lys Pro Gly Gly Ala Gln Cys
        755                 760                 765

Leu Pro Ser Gly Tyr Ser Ile Ser Ser Phe Leu Gln Met Leu His Pro
    770                 775                 780

Glu Cys Lys Glu Leu Pro Glu Pro Asn Leu Leu Pro Gly Gln Leu Ser
785                 790                 795                 800

His Gly Ala Val Gly Val Arg Glu Gly Arg Val Gln Trp Ile Ser Met
                805                 810                 815

Ala Phe Glu Ser Thr Thr Tyr Lys Gly Lys Ser Ser Phe Gln Thr Tyr
            820                 825                 830

Ser Asp Tyr Leu Arg Trp Glu Ser Phe Leu Gln Gln Gln Leu Gln Ala
        835                 840                 845

Leu Pro Glu Gly Ser Val Leu Arg Arg Gly Phe Gln Thr Cys Glu His
    850                 855                 860

Trp Lys Gln Ile Phe Met Glu Ile Val Gly Val Gln Ser Ala Leu Cys
865                 870                 875                 880

Gly Leu Val Leu Ser Leu Leu Ile Cys Val Ala Ala Val Ala Val Phe
                885                 890                 895
Thr Thr His Ile Leu Leu Leu Leu Pro Val Leu Leu Ser Ile Leu Gly
            900                 905                 910
Ile Val Cys Leu Val Val Thr Ile Met Tyr Trp Ser Gly Trp Glu Met
        915                 920                 925
Gly Ala Val Glu Ala Ile Ser Leu Ser Ile Leu Val Gly Ser Ser Val
    930                 935                 940
Asp Tyr Cys Val His Leu Val Glu Gly Tyr Leu Leu Ala Gly Glu Asn
945                 950                 955                 960
Leu Pro Pro His Gln Ala Glu Asp Ala Arg Thr Gln Arg Gln Trp Arg
                965                 970                 975
Thr Leu Glu Ala Val Arg His Val Gly Val Ala Ile Val Ser Ser Ala
            980                 985                 990
Leu Thr Thr Val Ile Ala Thr Val Pro Leu Phe Phe Cys Ile Ile Ala
        995                 1000                1005
Pro Phe Ala Lys Phe Gly Lys Ile Val Ala Leu Asn Thr Gly Val Ser
    1010                1015                1020
Ile Leu Tyr Thr Leu Thr Val Ser Thr Ala Leu Leu Gly Ile Met Ala
1025                1030                1035                1040
Pro Ser Ser Phe Thr Arg Thr Arg Thr Ser Phe Leu Lys Ala Leu Gly
                1045                1050                1055
Ala Val Leu Leu Ala Gly Ala Leu Gly Leu Gly Ala Cys Leu Val Leu

            1060                1065                1070

Leu Gln Ser Gly Tyr Lys Ile Pro Leu Pro Ala Gly Ala Ser Leu

        1075                1080                1085

<210> 18

<211> 24

<212> DNA

<213> Artificial Sequence

<220>

<223>

<400> 18

ctggtgggca tactgggtgg actc 24

<210> 19

<211> 22

<212> DNA

<213> Artificial Sequence

<220>

<223>

<400> 19

gcctcctgcc tcttgctgtc ca 22

<210> 20

<211> 23

<212> DNA

<213> Artificial Sequence

<220>

<223>

<400> 20

ccatgggctt cttctcctac ttg 23

<210> 21

<211> 22

<212> DNA

<213> Artificial Sequence

<220>

<223>

<400> 21

gggcccctag gaagaagcag at 22

<210> 22

<211> 240

<212> DNA

<213> Human

<400> 22

ccatgggctt cttctcctac ttgggtatcc gctcctccct ggtcatcctg caagtggttc 60

ctttcctggt gctgtccgtg ggggctgata acatcttcat ctttgttctc gagtaccaga 120

ggctgccccg gaggcctggg gagccacgag aggtccacat tgggcgagcc ctaggcaggg 180

tggctcccag catgctgttg tgcagcctct ctgaggccat ctgcttcttc ctaggggccc 240

<210> 23

<211> 24

<212> DNA
<213> Artificial Sequence
<220>
<223>
<400> 23
ttgctgccct ggtctacatc ctca 24
<210> 24
<211> 23
<212> DNA
<213> Artificial Sequence
<220>
<223>
<400> 24
cagagctgca ggcatggtga caa 23
<210> 25
<211> 19
<212> DNA
<213> Artificial Sequence
<220>
<223>
<400> 25
gggtggccgc cttcgtgat 19
<210> 26

<211> 19

<212> DNA

<213> Artificial Sequence

<220>

<223>

<400> 26

gcacggccag ccagcaaca                                                    19

<210> 27

<211> 258

<212> DNA

<213> Human

<400> 27

gggtggccgc cttcgtgatc gtgggcattg gtgtggacga tgtctttgtg ttcatcaaca      60

cctaccgcca ggccacccac ctggaagacc cacagctgcg catgatccac accgtccaaa     120

ctgcaggcaa ggccaccttc ttcacctccc tgaccacagc cgccgcctac gcagctaacg     180

tcttctccca gatcccagcc gtccacgact ttggcctgtt catgtctctc atcgtgtcct     240

gttgctggct ggccgtgc                                                   258

<210> 28

<211> 582

<212> DNA

<213> Human

<400> 28

ttcatggctg agcgctctct ggaagacgag atcaatcgca ccacagctga agacctgccc      60

atctttgcca ccagctacat tgtcatattc ctgtacatct ctctggccct gggcagctat 120
tccagctgga gccgagtgat ggtggactcc aaggccacgc tgggcctcgg cggggtggcc 180
gtggtcctgg gagcagtcat ggctgccatg ggcttcttct cctacttggg tatccgctcc 240
tccctggtca tcctgcaagt ggttcctttc ctggtgctgt ccgtgggggc tgataacatc 300
ttcatctttg ttctcgagta ccagaggctg ccccggaggc ctggggagcc acgagaggtc 360
cacattgggc gagccctagg cagggtggct cccagcatgc tgttgtgcag cctctctgag 420
gccatctgct tcttcctagg ggccctgacc cccatgccag ctgtgcggac ctttgccctg 480
acctctggcc ttgcagtgat ccttgacttc ctcctgcaga tgtcagcctt tgtggccctg 540
ctctccctgg acagcaagag gcaggaggcc tcccggttgg ac 582
<210> 29
<211> 600
<212> DNA
<213> Human
<400> 29
aaagtccagg ttctctatgg ggggacagac ctgtttgact atgaagtgcg caggacgttc 60
aacaatgaca tgctcctggc cttcatcagc agcagctgca ttgctgccct ggtctacatc 120
ctcacctcct gctcagtgtt cctgtccttc tttgggattg ccagcattgg tctcagctgc 180
ctggtggccc tcttcctgta ccacgtggtc tttggtatcc agtacttggg catcctgaat 240
ggggtggccg ccttcgtgat cgtgggcatt ggtgtggacg atgtctttgt gttcatcaac 300
acctaccgcc aggccaccca cctggaagac ccacagctgc gcatgatcca caccgtccaa 360
actgcaggca aggccacctt cttcacctcc ctgaccacag ccgccgccta cgcagctaac 420
gtcttctccc agatcccagc cgtccacgac tttggcctgt tcatgtctct catcgtgtcc 480
tgttgctggc tggccgtgct tgtcaccatg cctgcagctc tgggcctctg gagcctctac 540

ctggcaccac tggagagctc ctgccagacc agctgccacc agaattgcag ccggaagacc 600
<210> 30
<211> 19
<212> DNA
<213> Artificial Sequence
<220>
<223>
<400> 30
cggagcgcaa cattttcac 19
<210> 31
<211> 24
<212> DNA
<213> Artificial Sequence
<220>
<223>
<400> 31
acaacactac ccgggtctcc aatg 24
<210> 32
<211> 1371
<212> DNA
<213> Human
<400> 32
atggaccacc caggcttccg ggagttctgc tggaagcccc acgaggtgct caaggatctg 60

ccgctgggct cctactccta ctgctcgccc cccagctcgc tcatgaccta cttttttccc 120
accgagaggg gcggcaagat ctactatgac ggcatgggcc aggacctggc ggacatccgg 180
ggctccctgg agctggccat gactcaccct gagttctact ggtatgtgga tgagggcctc 240
tctgcagaca atctgaagag ctccctcctg cgcagtgaga tcctgtttgg agcacccctg 300
cccaactact actcagtaga tgaccgctgg gaggaacaac gggctaagtt tcagagcttc 360
gtggtcacct acgtggccat gctggccaag cagtctacca gcaaagtcca ggttctctat 420
ggggggacag acctgtttga ctatgaagtg cgcaggacgt tcaacaatga catgctcctg 480
gccttcatca gcagcagctg cattgctgcc ctggtctaca tcctcacctc ctgctcagtg 540
ttcctgtcct tctttgggat tgccagcatt ggtctcagct gcctggtggc cctcttcctg 600
taccacgtgg tctttggtat ccagtacttg ggcatcctga atggggtggc cgccttcgtg 660
atcgtgggca ttggtgtgga cgatgtcttt gtgttcatca acacctaccg ccaggccacc 720
cacctggaag acccacagct gcgcatgatc cacaccgtcc aaactgcagg caaggccacc 780
ttcttcacct ccctgaccac agccgccgcc tacgcagcta acgtcttctc ccagatccca 840
gccgtccacg actttggcct gttcatgtct ctcatcgtgt cctgttgctg gctggccgtg 900
cttgtcacca tgcctgcagc tctgggcctc tggagcctct acctggcacc actggagagc 960
tcctgccaga ccagctgcca ccagaattgc agccggaaga cctccctgca cttccccgga 1020
gacgtgtttg ccactcccga gcaggttgga ggcagccctg cccagggccc cataccctac 1080
ctggatgatg acatcccctt gctggaggtc gaggaagagc cagtgtcact ggagctggga 1140
gacgtgtccc tggtgtctgt gtcccccgag ggtctgcagc cagcctccaa cacgggcagc 1200
cgcggccatc tcatcgtgca gctgcaggag ctgctgcacc actgggtcct gtggtcagcc 1260
gtcaagagcc gctgggtgat tgtggccggc tccgccccgc cagccgggcc ccgctactct 1320
tccggcctga taccaacatc caggtgctgc tggacctcaa gtacaacctg a 1371
<210> 33

<211> 1338

<212> DNA

<213> Human

<400> 33

atggaccacc caggcttccg ggagttctgc tggaagcccc acgaggtgct caaggatctg 60

ccgctgggct cctactccta ctgctcgccc cccagctcgc tcatgaccta ctttttcccc 120

accgagaggg gcggcaagat ctactatgac ggcatgggcc aggacctggc ggacatccgg 180

ggctccctgg agctggccat gactcaccct gagttctact ggtatgtgga tgagggcctc 240

tctgcagaca atctgaagag ctccctcctg cgcagtgaga tcctgtttgg agcacccctg 300

cccaactact actcagtaga tgaccgctgg gaggaacaac gggctaagtt tcagagcttc 360

gtggtcacct acgtggccat gctggccaag cagtctacca gcaaagtcca ggttctctat 420

ggggggacag acctgtttga ctatgaagtg cgcaggacgt tcaacaatga catgctcctg 480

gccttcatca gcagcagctg cattgctgcc ctggtctaca tcctcacctc ctgctcagtg 540

ttcctgtcct tctttgggat tgccagcatt ggtctcagct gcctggtggc cctcttcctg 600

taccacgtgg tctttggtat ccagtacttg ggcatcctga atggggtggc cgccttcgtg 660

atcgtgggca ttggtgtgga cgatgtcttt gtgttcatca acacctaccg ccaggccacc 720

cacctggaag acccacagct gcgcatgatc cacaccgtcc aaactgcagg caaggccacc 780

ttcttcacct ccctgaccac agccgccgcc tacgcagcta acgtcttctc ccagatccca 840

gccgtccacg actttggcct gttcatgtct ctcatcgtgt cctgttgctg gctggccgtg 900

cttgtcacca tgcctgcagc tctgggcctc tggagcctct acctggcacc actggagagc 960

tcctgccaga ccagctgcca ccagaattgc agccggaaga cctccctgca cttccccgga 1020

gacgtgtttg ccactcccga gcaggttgga ggcagccctg cccagggccc cataccctac 1080

ctggatgatg acatcccctt gctggaggtc gaggaagagc cagtgtcact ggagctggga 1140

gacgtgtccc tggtgtctgt gtcccccgag ggtctgcagc cagcctccaa cacgggcagc 1200
cgcggccatc tcatcgtgca gctgcaggag ctgctgcacc actgggtcct gtggtcagcc 1260
gtcaagagcc gctgggtgat tgtggtccgt ttagcactca gttcctattc tgtaattgag 1320
gatgagaacg gtggctag 1338
<210> 34
<211> 456
<212> PRT
<213> Human
<400> 34
Met Asp His Pro Gly Phe Arg Glu Phe Cys Trp Lys Pro His Glu Val
                5                   10                  15
Leu Lys Asp Leu Pro Leu Gly Ser Tyr Ser Tyr Cys Ser Pro Pro Ser
            20                  25                  30
Ser Leu Met Thr Tyr Phe Phe Pro Thr Glu Arg Gly Gly Lys Ile Tyr
        35                  40                  45
Tyr Asp Gly Met Gly Gln Asp Leu Ala Asp Ile Arg Gly Ser Leu Glu
    50                  55                  60
Leu Ala Met Thr His Pro Glu Phe Tyr Trp Tyr Val Asp Glu Gly Leu
65                  70                  75                  80
Ser Ala Asp Asn Leu Lys Ser Ser Leu Leu Arg Ser Glu Ile Leu Phe
                85                  90                  95
Gly Ala Pro Leu Pro Asn Tyr Tyr Ser Val Asp Asp Arg Trp Glu Glu
            100                 105                 110

Gln Arg Ala Lys Phe Gln Ser Phe Val Val Thr Tyr Val Ala Met Leu
        115                 120                 125
Ala Lys Gln Ser Thr Ser Lys Val Gln Val Leu Tyr Gly Gly Thr Asp
    130                 135                 140
Leu Phe Asp Tyr Glu Val Arg Arg Thr Phe Asn Asn Asp Met Leu Leu
145                 150                 155                 160
Ala Phe Ile Ser Ser Ser Cys Ile Ala Ala Leu Val Tyr Ile Leu Thr
                165                 170                 175
Ser Cys Ser Val Phe Leu Ser Phe Phe Gly Ile Ala Ser Ile Gly Leu
            180                 185                 190
Ser Cys Leu Val Ala Leu Phe Leu Tyr His Val Val Phe Gly Ile Gln
        195                 200                 205
Tyr Leu Gly Ile Leu Asn Gly Val Ala Ala Phe Val Ile Val Gly Ile
    210                 215                 220
Gly Val Asp Asp Val Phe Val Phe Ile Asn Thr Tyr Arg Gln Ala Thr
225                 230                 235                 240
His Leu Glu Asp Pro Gln Leu Arg Met Ile His Thr Val Gln Thr Ala
                245                 250                 255
Gly Lys Ala Thr Phe Phe Thr Ser Leu Thr Thr Ala Ala Ala Tyr Ala
            260                 265                 270
Ala Asn Val Phe Ser Gln Ile Pro Ala Val His Asp Phe Gly Leu Phe
        275                 280                 285
Met Ser Leu Ile Val Ser Cys Cys Trp Leu Ala Val Leu Val Thr Met

290                 295                 300

Pro Ala Ala Leu Gly Leu Trp Ser Leu Tyr Leu Ala Pro Leu Glu Ser

305                 310                 315                 320

Ser Cys Gln Thr Ser Cys His Gln Asn Cys Ser Arg Lys Thr Ser Leu

                325                 330                 335

His Phe Pro Gly Asp Val Phe Ala Thr Pro Glu Gln Val Gly Gly Ser

            340                 345                 350

Pro Ala Gln Gly Pro Ile Pro Tyr Leu Asp Asp Asp Ile Pro Leu Leu

        355                 360                 365

Glu Val Glu Glu Glu Pro Val Ser Leu Glu Leu Gly Asp Val Ser Leu

    370                 375                 380

Val Ser Val Ser Pro Glu Gly Leu Gln Pro Ala Ser Asn Thr Gly Ser

385                 390                 395                 400

Arg Gly His Leu Ile Val Gln Leu Gln Glu Leu Leu His His Trp Val

                405                 410                 415

Leu Trp Ser Ala Val Lys Ser Arg Trp Val Ile Val Ala Gly Ser Ala

            420                 425                 430

Pro Pro Ala Gly Pro Arg Tyr Ser Ser Gly Leu Ile Pro Thr Ser Arg

        435                 440                 445

Cys Cys Trp Thr Ser Ser Thr Thr

    450                 455

<210> 35

<211> 445

<212> PRT

<213> Human

<400> 35

Met Asp His Pro Gly Phe Arg Glu Phe Cys Trp Lys Pro His Glu Val

                5                   10                  15

Leu Lys Asp Leu Pro Leu Gly Ser Tyr Ser Tyr Cys Ser Pro Pro Ser

            20                  25                  30

Ser Leu Met Thr Tyr Phe Phe Pro Thr Glu Arg Gly Gly Lys Ile Tyr

        35                  40                  45

Tyr Asp Gly Met Gly Gln Asp Leu Ala Asp Ile Arg Gly Ser Leu Glu

    50                  55                  60

Leu Ala Met Thr His Pro Glu Phe Tyr Trp Tyr Val Asp Glu Gly Leu

65                  70                  75                  80

Ser Ala Asp Asn Leu Lys Ser Ser Leu Leu Arg Ser Glu Ile Leu Phe

                85                  90                  95

Gly Ala Pro Leu Pro Asn Tyr Tyr Ser Val Asp Asp Arg Trp Glu Glu

            100                 105                 110

Gln Arg Ala Lys Phe Gln Ser Phe Val Val Thr Tyr Val Ala Met Leu

        115                 120                 125

Ala Lys Gln Ser Thr Ser Lys Val Gln Val Leu Tyr Gly Gly Thr Asp

    130                 135                 140

Leu Phe Asp Tyr Glu Val Arg Arg Thr Phe Asn Asn Asp Met Leu Leu

145                 150                 155                 160

Ala Phe Ile Ser Ser Ser Cys Ile Ala Ala Leu Val Tyr Ile Leu Thr
                165                 170                 175
Ser Cys Ser Val Phe Leu Ser Phe Phe Gly Ile Ala Ser Ile Gly Leu
            180                 185                 190
Ser Cys Leu Val Ala Leu Phe Leu Tyr His Val Val Phe Gly Ile Gln
        195                 200                 205
Tyr Leu Gly Ile Leu Asn Gly Val Ala Ala Phe Val Ile Val Gly Ile
    210                 215                 220
Gly Val Asp Asp Val Phe Val Phe Ile Asn Thr Tyr Arg Gln Ala Thr
225                 230                 235                 240
His Leu Glu Asp Pro Gln Leu Arg Met Ile His Thr Val Gln Thr Ala
                245                 250                 255
Gly Lys Ala Thr Phe Phe Thr Ser Leu Thr Thr Ala Ala Ala Tyr Ala
            260                 265                 270
Ala Asn Val Phe Ser Gln Ile Pro Ala Val His Asp Phe Gly Leu Phe
        275                 280                 285
Met Ser Leu Ile Val Ser Cys Cys Trp Leu Ala Val Leu Val Thr Met
    290                 295                 300
Pro Ala Ala Leu Gly Leu Trp Ser Leu Tyr Leu Ala Pro Leu Glu Ser
305                 310                 315                 320
Ser Cys Gln Thr Ser Cys His Gln Asn Cys Ser Arg Lys Thr Ser Leu
                325                 330                 335
His Phe Pro Gly Asp Val Phe Ala Thr Pro Glu Gln Val Gly Gly Ser

            340                 345                 350
Pro Ala Gln Gly Pro Ile Pro Tyr Leu Asp Asp Asp Ile Pro Leu Leu
        355                 360                 365
Glu Val Glu Glu Glu Pro Val Ser Leu Glu Leu Gly Asp Val Ser Leu
    370                 375                 380
Val Ser Val Ser Pro Glu Gly Leu Gln Pro Ala Ser Asn Thr Gly Ser
385                 390                 395                 400
Arg Gly His Leu Ile Val Gln Leu Gln Glu Leu Leu His His Trp Val
                405                 410                 415
Leu Trp Ser Ala Val Lys Ser Arg Trp Val Ile Val Val Arg Leu Ala
            420                 425                 430
Leu Ser Ser Tyr Ser Val Ile Glu Asp Glu Asn Gly Gly
        435                 440                 445
<210> 36
<211> 25
<212> DNA
<213> Artificial Sequence
<220>
<223>
<400> 36
tactcttccg gcctgatacc aacat 25
<210> 37
<211> 22

<212> DNA
<213> Artificial Sequence
<220>
<223>
<400> 37
ccaaaggtgc aagtgtccag ag                                                22
<210> 38
<211> 27
<212> PRT
<213> Human
<400> 38
Asp Thr Val Ala Ile Leu Ser Pro Arg Leu Glu Tyr Ser Gly Thr Ile
                5                   10                  15
Ser Ala His Cys Asn Leu Tyr Leu Leu Asp Ser
            20                  25
<210> 39
<211> 81
<212> DNA
<213> Human
<400> 39
acacagttgc  cattctgtca  cccaggctgg  agtacagtgg  cacaatctcg  gctcactgca
60
acctctacct                          cctggattca                          g

81

<210> 40

<211> 1392

<212> PRT

<213> Human

<400> 40
<210> 41
<211> 4179
<212> DNA
<213> Human
<400> 41
atggacacgg aggatgaccc cttgctgcag gatgtgtggc tagaggagga gcaggaggag 60
gaagaagcaa cgggtgaaac ctttttaggg gcccagaagc cagggcccca acctggggca 120
gggggacagt gttgctggcg gcactggccc ctggcttccc gacccccagc ttcgggcttc 180
tggagtaccc tgggctgggc cttcaccaat ccgtgctgtg ctgggctggt gctcttcctg 240
ggctgcagca tccccatggc cctgtcagcc ttcatgttcc tttactaccc accgctggac 300
attgacatct cctacaacgc ctttgagatc cgcaaccacg aggcctcaca gcgtttcgac 360
gctctcactc tggcgcttaa gtcccagttt ggatcctggg ggcggaaccg gcgcgatttg 420
gccgacttca cctccgagac gcttcagcgc cttatctcag agcagctgca gcagctgcat 480
ctcggcaacc gctcgcggca agcctcccga gccccccgcg tcatccccgc ggcctcactc 540
ggtagcccag gcccttaccg ggacacttcc gcggctcaaa agcccacagc caatcggagc 600
gggcgacttc ggcgtgagac cccgcccctg gaggatctgg cagccaacca gagtgaagac 660
ccgcgaaacc agcggctgag caagaatggg cggtaccagc ccagcatccc gccccacgcg 720
gcagtcgcgg ccaatcagag ccgtgcccgc cgaggcgcct cgcgctggga ctactcgcgc 780
gcctatgtga gtgccaacac tcagacgcac gcgcactggc gcatcgagct catcttcctg 840
gcgcgcggcg acgcggagcg caacattttc accagtgagc gcctggtcac gatccatgag 900
atcgagcgca agatcatgga ccacccaggc ttccgggagt tctgctggaa gccccacgag 960
gtgctcaagg atctgccgct gggctcctac tcctactgct cgccccccag ctcgctcatg 1020
acctactttt ttcccaccga gaggggcggc aagatctact atgacggcat gggccaggac 1080
ctggcggaca tccggggctc cctggagctg gccatgactc accctgagtt ctactggtat 1140
gtggatgagg gcctctctgc agacaatctg aagagctccc tcctgcgcag tgagatcctg 1200

tttggagcac ccctgcccaa ctactactca gtagatgacc gctgggagga acaacgggct 1260
aagtttcaga gcttcgtggt cacctacgtg gccatgctgg ccaagcagtc taccagcaaa 1320
gtccaggttc tctatggggg gacagacctg tttgactatg aagtgcgcag gacgttcaac 1380
aatgacatgc tcctggcctt catcagcagc agctgcattg ctgccctggt ctacatcctc 1440
acctcctgct cagtgttcct gtccttcttt gggattgcca gcattggtct cagctgcctg 1500
gtggccctct tcctgtacca cgtggtcttt ggtatccagt acttgggcat cctgaatggg 1560
gtggccgcct tcgtgatcgt gggcattggt gtggacgatg tctttgtgtt catcaacacc 1620
taccgccagg ccacccacct ggaagaccca cagctgcgca tgatccacac cgtccaaact 1680
gcaggcaagg ccaccttctt cacctccctg accacagccg ccgcctacgc agctaacgtc 1740
ttctcccaga tcccagccgt ccacgacttt ggcctgttca tgtctctcat cgtgtcctgt 1800
tgctggctgg ccgtgcttgt caccatgcct gcagctctgg gcctctggag cctctacctg 1860
gcaccactgg agagctcctg ccagaccagc tgccaccaga attgcagccg gaagacctcc 1920
ctgcacttcc ccggagacgt gtttgccact cccgagcagg ttggaggcag ccctgcccag 1980
ggccccatac cctacctgga tgatgacatc cccttgctgg aggtcgagga agagccagtg 2040
tcactggagc tgggagacgt gtccctggtg tctgtgtccc ccgagggtct gcagccagcc 2100
tccaacacgg gcagccgcgg ccatctcatc gtgcagctgc aggagctgct gcaccactgg 2160
gtcctgtggt cagccgtcaa gagccgctgg gtgattgtgg ggctgttcgt ctccatcctc 2220
atcttgtccc tggtgttcgc cagccggctc cgccccgcca gccgggcccc gctactcttc 2280
cggcctgata ccaacatcca ggtgctgctg gacctcaagt acaacctgag cgccgagggc 2340
atctcctgca tcacctgttc aggtctgttc caggagaagc cccacagcct gcagaacaac 2400
atccggacgt ccctggagaa gaagaggcga ggctcagggg tcccctgggc tagccggcct 2460
gaggccaccc tgcaggattt cccaggcacc gtgtacatct ctaaagtgaa gagtcaaggc 2520
caccccgctg tctacaggct ctccctcaat gccagcctgc ctgctccttg gcaggctgtg 2580
tcgcctgggg atggagaggt gccctccttc caggtgtata gagcgccttt tggtaacttc 2640
accaagaagc tgaccgcttg tatgtctaca gtagggctgc tccaggcggc gagcccctcc 2700
cgcaagtgga tgctgacgac cttggcctgt gatgccaagc ggggctggaa gtttgacttc 2760
agcttctacg tggccaccaa ggagcagcag cacacccgga agctgtactt cgcccagtcc 2820
cacaagcccc ccttccacgg gcgcgtatgc atggcacccc ctggctgcct gcttagctcc 2880
agccccgatg ggcctaccaa aggcttcttc ttcgtgccta gtgagaaagt gcccaaggcc 2940
cgtctctcag ccaccttcgg cttcaacccc tgcgtgaaca cgggctgcgg gaagccggcg 3000
gtgcggccac tagtggatac cggggccatg gtctttgtgg tcttcggcat tattggcgtc 3060
aaccgcactc ggcaggtgga caaccacgtc attggagacc cgggtagtgt tgtctacgac 3120
agcagctttg acctcttcaa ggaaattggg cacctgtgtc acctctgcaa ggccatcgca 3180
gccaactccg agctggtgaa gccgggtggg gcccagtgcc tgccttcagg ctacagcatc 3240
tcctccttcc tgcagatgtt gcaccctgag tgcaaggagc tgcccgagcc caacctgctc 3300

ccggggcagc tgtcccacgg ggcagtgggc gtcagggagg gccgcgtgca gtggatctcc 3360
atggctttcg agtcgaccac gtacaagggc aaatcctcct tccagaccta ctcggactac 3420
ctgcgctggg agagcttcct ccagcagcag ctgcaggcct tgcccgaggg ctcagtcctg 3480
cgccggggct tccagacctg cgagcactgg aagcagatat tcatggaaat cgtaggggtg 3540
cagagcgccc tgtgcggcct ggtgctatcc ctgctcatct gcgtggccgc ggtggccgtg 3600
ttcaccaccc acatcctgct cctgctgccc gtgctcctca gcatcttggg catcgtgtgc 3660
ctggtggtga ccatcatgta ctggagcggc tgggagatgg gggctgtgga agccatctcc 3720
ctgtccatcc tcgttggctc ctccgtggat tactgcgtcc acctggtcga gggctacctg 3780
ctggctggag agaacctgcc cccccaccag gccgaggacg cccgaacgca gcgccagtgg 3840
cgtacgctgg aggccgtgcg gcacgtgggc gtggccatcg tctccagtgc cctcaccacg 3900
gtcatcgcca cagtgcccct cttcttctgc atcatcgccc catttgccaa gttcggcaag 3960
attgtggcac tcaacacggg cgtgtccatc ctctacacgc tgaccgtcag caccgccctg 4020
ctgggcatca tggcgcccag ctctttcact cggacccgga cttccttcct caaggccctg 4080
ggtgccgtgc tgctggcagg ggccctgggg ctgggtgcct gcctcgtgct cctgcagagc 4140
ggctataaga ttcccctgcc cgcaggggcc tccctatag 4179
<210> 42
<211> 5181
<212> DNA
<213> Human
<400> 42
gcggctccga gaagcttccc cctgcgactt ccgcgaggag acgagtctgc gcagcgtggt 60
ggccgccgcc ccccgaccct ctgcgcactc tctcccgcgc cggcggctca gcctagcccc 120
gttcggccgg ccgagactat ggacacggag gatgacccct tgctgcagga tgtgtggcta 180
gaggaggagc aggaggagga agaagcaacg ggtgaaacct ttttaggggc ccagaagcca 240
gggccccaac ctggggcagg gggacagtgt tgctggcggc actggcccct ggcttcccga 300
cccccagctt cgggcttctg gagtaccctg ggctgggcct tcaccaatcc gtgctgtgct 360
gggctggtgc tcttcctggg ctgcagcatc cccatggccc tgtcagcctt catgttcctt 420
tactacccac cgctggacat tgacatctcc tacaacgcct ttgagatccg caaccacgag 480

gcctcacagc gtttcgacgc tctcactctg gcgcttaagt cccagtttgg atcctggggg 540
cggaaccggc gcgatttggc cgacttcacc tccgagacgc ttcagcgcct tatctcagag 600
cagctgcagc agctgcatct cggcaaccgc tcgcggcaag cctcccgagc cccccgcgtc 660
atccccgcgg cctcactcgg tagcccaggc ccttaccggg acacttccgc ggctcaaaag 720
cccacagcca atcggagcgg gcgacttcgg cgtgagaccc cgcccctgga ggatctggca 780
gccaaccaga gtgaagaccc gcgaaaccag cggctgagca agaatgggcg gtaccagccc 840
agcatcccgc cccacgcggc agtcgcggcc aatcagagcc gtgcccgccg aggcgcctcg 900
cgctgggact actcgcgcgc ctatgtgagt gccaacactc agacgcacgc gcactggcgc 960
atcgagctca tcttcctggc gcgcggcgac gcggagcgca acattttcac cagtgagcgc 1020
ctggtcacga tccatgagat cgagcgcaag atcatggacc acccaggctt ccgggagttc 1080
tgctggaagc cccacgaggt gctcaaggat ctgccgctgg gctcctactc ctactgctcg 1140
ccccccagct cgctcatgac ctactttttt cccaccgaga ggggcggcaa gatctactat 1200
gacggcatgg gccaggacct ggcggacatc cggggctccc tggagctggc catgactcac 1260
cctgagttct actggtatgt ggatgagggc ctctctgcag acaatctgaa gagctccctc 1320
ctgcgcagtg agatcctgtt tggagcaccc ctgcccaact actactcagt agatgaccgc 1380
tgggaggaac aacgggctaa gtttcagagc ttcgtggtca cctacgtggc catgctggcc 1440
aagcagtcta ccagcaaagt ccaggttctc tatgggggga cagacctgtt tgactatgaa 1500
gtgcgcagga cgttcaacaa tgacatgctc ctggccttca tcagcagcag ctgcattgct 1560
gccctggtct acatcctcac ctcctgctca gtgttcctgt ccttctttgg gattgccagc 1620
attggtctca gctgcctggt ggccctcttc ctgtaccacg tggtctttgg tatccagtac 1680
ttgggcatcc tgaatggggt ggccgccttc gtgatcgtgg gcattggtgt ggacgatgtc 1740
tttgtgttca tcaacaccta ccgccaggcc acccacctgg aagacccaca gctgcgcatg 1800
atccacaccg tccaaactgc aggcaaggcc accttcttca cctccctgac cacagccgcc 1860

gcctacgcag ctaacgtctt ctcccagatc ccagccgtcc acgactttgg cctgttcatg 1920
tctctcatcg tgtcctgttg ctggctggcc gtgcttgtca ccatgcctgc agctctgggc 1980
ctctggagcc tctacctggc accactggag agctcctgcc agaccagctg ccaccagaat 2040
tgcagccgga agacctccct gcacttcccc ggagacgtgt ttgccactcc cgagcaggtt 2100
ggaggcagcc ctgcccaggg ccccataccc tacctggatg atgacatccc cttgctggag 2160
gtcgaggaag agccagtgtc actggagctg ggagacgtgt ccctggtgtc tgtgtccccc 2220
gagggtctgc agccagcctc caacacgggc agccgcggcc atctcatcgt gcagctgcag 2280
gagctgctgc accactgggt cctgtggtca gccgtcaaga gccgctgggt gattgtgggg 2340
ctgttcgtct ccatcctcat cttgtccctg gtgttcgcca gccggctccg ccccgccagc 2400
cgggccccgc tactcttccg gcctgatacc aacatccagg tgctgctgga cctcaagtac 2460
aacctgagcg ccgagggcat ctcctgcatc acctgttcag gtctgttcca ggagaagccc 2520
cacagcctgc agaacaacat ccggacgtcc ctggagaaga agaggcgagg ctcaggggtc 2580
ccctgggcta gccggcctga ggccaccctg caggatttcc caggcaccgt gtacatctct 2640
aaagtgaaga gtcaaggcca ccccgctgtc tacaggctct ccctcaatgc cagcctgcct 2700
gctccttggc aggctgtgtc gcctggggat ggagaggtgc cctccttcca ggtgtataga 2760
gcgccttttg gtaacttcac caagaagctg accgcttgta tgtctacagt agggctgctc 2820
caggcggcga gcccctcccg caagtggatg ctgacgacct tggcctgtga tgccaagcgg 2880
ggctggaagt ttgacttcag cttctacgtg gccaccaagg agcagcagca cacccggaag 2940
ctgtacttcg cccagtccca caagcccccc ttccacggcc gcgtatgcat ggcaccccct 3000
ggctgcctgc ttagctccag ccccgatggg cctaccaaag gcttcttctt cgtgcctagt 3060
gagaaagtgc ccaaggcccg tctctcagcc accttcggct tcaacccctg cgtgaacacg 3120
ggctgcggga agccggcggt gcggccacta gtggataccg gggccatggt ctttgtggtc 3180
ttcggcatta ttggcgtcaa ccgcactcgg caggtggaca accacgtcat tggagacccg 3240

ggtagtgttg tctacgacag cagctttgac ctcttcaagg aaattgggca cctgtgtcac 3300
ctctgcaagg ccatcgcagc caactccgag ctggtgaagc cgggtggggc ccagtgcctg 3360
ccttcaggct acagcatctc ctccttcctg cagatgttgc accctgagtg caaggagctg 3420
cccgagccca acctgctccc ggggcagctg tcccacgggg cagtgggcgt cagggagggc 3480
cgcgtgcagt ggatctccat ggctttcgag tcgaccacgt acaagggcaa atcctccttc 3540
cagacctact cggactacct gcgctgggag agcttcctcc agcagcagct gcaggccttg 3600
cccgagggct cagtcctgcg ccggggcttc cagacctgcg agcactggaa gcagatattc 3660
atggaaatcg taggggtgca gagcgccctg tgcggcctgg tgctatccct gctcatctgc 3720
gtggccgcgg tggccgtgtt caccacccac atcctgctcc tgctgcccgt gctcctcagc 3780
atcttgggca tcgtgtgcct ggtggtgacc atcatgtact ggagcggctg ggagatgggg 3840
gctgtggaag ccatctccct gtccatcctc gttggctcct ccgtggatta ctgcgtccac 3900
ctggtcgagg gctacctgct ggctggagag aacctgcccc cccaccaggc cgaggacgcc 3960
cgaacgcagc gccagtggcg tacgctggag gccgtgcggc acgtgggcgt ggccatcgtc 4020
tccagtgccc tcaccacggt catcgccaca gtgcccctct tcttctgcat catcgcccca 4080
tttgccaagt tcggcaagat tgtggcactc aacacgggcg tgtccatcct ctacacgctg 4140
accgtcagca ccgccctgct gggcatcatg gcgcccagct ctttcactcg gacccggact 4200
tccttcctca aggccctggg tgccgtgctg ctggcagggg ccctggggct gggtgcctgc 4260
ctcgtgctcc tgcagagcgg ctataagatt cccctgcccg caggggcctc cctatagccc 4320
gggacgggct ctggacactt gcacctttgg tcccatgggt gggggacagg agctgcttcc 4380
cagctcgact tcagctagct gtgtccccag gcctgggccc agggcgccct gcgggccagc 4440
gtggaggctg acacccacac agatggtgtg gaccatgctg ccttgtggag ctgggagttg 4500
gagacagccg ccaccccaca ggccgggcta ctggcagcca cactcggctt tttgcccagt 4560
ggcagaagag accagccctc ctcccatgcc cggtcaccat gggggtcagg ttatttttgt 4620

agggggtctc cctctcacac tgcctcagtg ctcacaacct tccagtgtgg atgttacagg 4680
gtggccccca ttctaccgat gtgaaaactg aggcgccagg acacagtggc tgccctgtcg 4740
ctggatcagt agcagagcca gagctgcctc cgagcgccat gccgccctcg ggaatcatac 4800
aggaagagca cagtggatcc agggtggggg cctctcaccc cctaaccccg cccccccgca 4860
accctcccct tcagctttac ggcggccagt gctgaatggc cctgtggccc tccctgggcc 4920
ttttggtctt ggccagagaa gacagaagga cccggcttgg gcttctgcat gtcctacccc 4980
tgaccccagc ctcaaggggc ccctcaaagg ccctctctgg gggctctggg gctcagcaca 5040
gctttcctca tggatctaag cccctgtctt tcccacctga ctctgagtgg atgttttggg 5100
ggatggcccc tgtggggagg agctgccatg ccggccgcct gctcacggca gaaggttgct 5160
attaaaatga cataggattg c 5181
<210> 43
<211> 21
<212> DNA
<213> Artificial Sequence
<220>
<223>
<400> 43
cttaagcgcc agagtgagag c 21
<210> 44
<211> 39
<212> DNA
<213> Artificial Sequence
<220>

<223>

<400> 44

aagcagtggt atcaacgcag agtggccatt atggccggg 39

<210> 45

<211> 23

<212> DNA

<213> Artificial Sequence

<220>

<223>

<400> 45

aagcagtggt atcaacgcag agt 23

<210> 46

<211> 19

<212> DNA

<213> Artificial Sequence

<220>

<223>

<400> 46

agcgccagag tgagagcgt 19

<210> 47

<211> 23

<212> DNA

<213> Artificial Sequence

<220>

<223>

<400> 47

tgttccttta ctacccaccg ctg                                                23

<210> 48

<211> 24

<212> DNA

<213> Artificial Sequence

<220>

<223>

<400> 48

gtcatcctcc gtgtccatag tctc                                               24

<210> 49

<211> 80

<212> DNA

<213> Artificial Sequence

<220>

<223>

<400> 49

ttgcgagctg aggactggga ttcgcgcgca gcttcccgcg gtctgcttgc cctggagcgg    60

agggggagcc ccagcctcct                                                    80

<210> 50

<211> 24

<212> DNA
<213> Artificial Sequence
<220>
<223>
<400> 50
gagactatgg acacggagga tgac 24
<210> 51
<211> 20
<212> DNA
<213> Artificial Sequence
<220>
<223>
<400> 51
aggccctcat ccacatacca 20
<210> 52
<211> 22
<212> DNA
<213> Artificial Sequence
<220>
<223>
<400> 52
ggagcgcaac attttcacca gt 22
<210> 53

<211> 22

<212> DNA

<213> Artificial Sequence

<220>

<223>

<400> 53

ccaaaggtgc aagtgtccag ag                                                22

<210> 54

<211> 22

<212> DNA

<213> Artificial Sequence

<220>

<223>

<400> 54

cactggatca ctcgaggtgt tg                                                22

<210> 55

<211> 19

<212> DNA

<213> Artificial Sequence

<220>

<223>

<400> 55

ccagtcccac gctgatgtg                                                    19

<210> 56

<211> 24

<212> DNA

<213> Artificial Sequence

<220>

<223>

<400> 56

ctgctgtttc tcgccctgtt cgga 24

<210> 57

<211> 21

<212> DNA

<213> Artificial Sequence

<220>

<223>

<400> 57

aatgacatgc tcctggcctt c 21

<210> 58

<211> 21

<212> DNA

<213> Artificial Sequence

<220>

<223>

<400> 58

atgctggcaa tcccaaaaga a                                                21

<210> 59

<211> 30

<212> DNA

<213> Artificial Sequence

<220>

<223>

<400> 59

tacatcctca cctcctgctc agtgttcctg                                       30

## Description

### TECHNICAL FIELD

The present invention relates to a novel SSD-containing protein derived from human liver, human testis and human brain, or salts thereof, and a DNA encoding the same.

### BACKGROUND ART

Cholesterol is a molecule rich in a plasma membrane of a eucaryotic cell, involving permeability, mechanical strength, durability and the like of the membrane, and having an important function in controlling the membrane, and it is also an essential lipid as various steroid hormone precursors. Animal cells have a complicated mechanism for controlling intracellular level of cholesterol which is one of main constituent lipids of a plasma membrane, for maintaining homeostasis in an organism. It is known that the cholesterol concentration in the plasma membrane and intracellular organelles is under strict control, and there is required a function to detect the concentration for this control. As a candidate for carrying out this function, SSD (sterol-sensing domain) is proposed [Current Opinion in Structural Biology, 8, 435-439 (1998)]. SSD was, at first, proposed as a five-transmembrane region, which is found conserved in HMG-CoA (3-hydroxy-3-methylglutaryl-coenzyme A) reducing enzyme, rate-determining enzyme in a cholesterol biosynthesis route, proteolysis of which is promoted by increase in intracellular cholesterol, and SCAP (SREBP cleavage activating protein) obtained as a molecule to activate processing of SREBP (sterol regulation element binding protein) by decrease in sterol level in a microsome, and thus which has a possibility of sensing the sterol concentration [Cell, 87, 415-426 (1996)]. The SSDs have neither so high homology in primary structure (identity: about 20%, possibility: about 40%) nor a consensus motif present. However, the SSDs are predicted to be well preserved in secondary structure because the hydrophobic plots thereof are extremely similar.

Niemann Pick disease type C is an autosomal recessive hereditary disease and is a metabolic disease having a deficiency in transportation of LDL-derived cholesterol from lysosome, leading to extraordinary accumulation of LDL cholesterol in a cell, and causing neuropathy and splenohepatomegaly. The causative gene thereof, NPC1 has been recently positional-cloned, teaching that its protein has SSD [Molecular Medicine Today, 4, 525-531 (1998)]. It is now suggested for NPC1 that its SSD detects cholesterol level of lysosome and when it reaches the threshold or more, NPC-granules are released as in budding of a vesicle to transport cholesterol to a microsome and plasma membrane [Current Opinion in Lipidology, 9, 131-135 (1998)], and this model is supported by numerous experimental facts [Proc. Natl. Acad. Sci. USA, 96, 805-810 (1999); J. Biol. Chem., 274, 9627-9635 (1999)]. NPC1 is only one identified as a sensor molecule in the intracellular lipid vesicle transportation system, however, there is a high possibility that the sensor transportation vesicle system is present also in other organelles.

Patched has been reported to be a candidate gene of basal cell nevus syndrome [Science, 272, 1668-1671 (1996)], and it functions as a secretory protein involving morphogenesis and as a receptor of Hedgehog, and Hedgehog receives the modification with cholesterol in embryonic neuroepithelium [Nature Genet., 15, 123-124 (1997)]. SSD is confirmed also in Patched [Cold Spring Harb. Symp. Quant. Biol., 62, 191-204 (1997); Science, 277, 228-231 (1997)]. Since NPC1 is supposed to function as a cholesterol sensor of lysosomes and Patched is supposed to function as a sensor for cholesterol modification of Hedgehog, all of four SSD-containing proteins found until now have a sterol sensor function. Recently, TRC8 [Proc. Natl. Acad. Sci. USA, 95, 9572-9577 (1998)], DHCR (7-dehydrocholesterol reductase)[J. Biol. Chem., 274, 14624-14631 (1999)], Dispatched [Cell, 99, 803-815 (1999)] and the like have been reported as a SSD-containing protein.

Recently, an idea has appeared that P glycoprotein ABC 1 is a receptor for cholesterol transportation [Nature Genetics, 22, 336-345 (1999); Nature Genetics, 22, 347-351 (1999); Nature Genetics, 22, 352-355 (1999)], there was, however, a report that a multi drug resistant (MDR) molecule involves the intracellular lipid transportation before [Cell, 87, 507-517 (1996)]. Interesting is an indication that a group of MDR inhibitors not only inhibit cholesterol transportation, but also cause lipid accumulation in lysosome as in NPC cell, and have teratogenicity, and the strengths thereof correlate with each other [Science, 280, 1603-1607 (1998)]. From these facts, it can be expected that MDR involving cholesterol transportation receives the activity regulation through cooperation or interaction with an SSD-containing protein, and ABC1 also has the same possibility. Active lipid transportation is present between cell membrane and microsome or Golgi apparatus, but a sensor molecule has not been identified yet. It is known that ACAT is distributed over the entire cytoplasm as if escaping from proteolysis, from a microsome at which ACAT originally is localized, with increase in the intracellular concentration of cholesterol, and this indicates the presence of a sensor molecule.

There are many intracellular and extracellular phenomena predicting the presence of a cholesterol sensor, which, however, cannot be explained using SSD-containing proteins found up to now. If a novel protein having SSD is found, the functional molecule may involve changed localization of macrophage ACAT due to cholesterol-loading, namely, transportation of a ACAT-containing vesicle, increased proteolysis of macrophage neutral cholesterol esterase (for example, hormone sensitive lipase) due to cholesterol-loading, a vesicle for transporting cholesterol from a microsome and Golgi apparatus to plasma membrane, a vesicle for transporting cholesterol from plasma membrane to a microsome, a rate-limiting enzyme at cholesterol synthesis system, a rate-limiting enzyme at bile acid synthesis system, a vesicle for transportation to mitochondria which is a site of steroid synthesis, and the like.

Thus, a SSD-containing protein has a high possibility of performing physiologically very important role, and discovery of a novel SSD-containing protein and
and elucidation of the mechanism has been desired.

The object of the present invention is to provide a novel SSD-containing protein or salts thereof, a DNA encoding the protein, a recombinant vector, a transformant, a method of producing the protein, a medicine containing the protein or DNA, an antibody to the protein, a method of screening a compound promoting or inhibiting the activity of the protein or salts thereof, a method of screening a compound changing the sterol-sensing ability of the protein or salts thereof, a compound or salt thereof obtainable by the screening methods, and the compound or salt thereof, and the like.

If a novel SSD-containing protein is found, it is possible to analyze much more a functional molecule which is related to changed localization of macrophage ACAT due to cholesterol-loading, namely, transportation of a ACAT-containing vesicle, increased proteolysis of macrophage neutral cholesterol esterase (for example, hormone sensitive lipase) due to cholesterol-loading, a vesicle for transporting cholesterol from a microsome and Golgi apparatus to plasma membrane, a vesicle for transporting cholesterol from plasma membrane to a microsome, a rate-limiting enzyme at cholesterol synthesis system, a rate-limiting enzyme at bile acid synthesis system, a vesicle for transportation to mitochondria which is a site of steroid synthesis, and to develop a new pharmaceutical inhibiting or promoting the activity of the protein, useful for prevention, diagnosis and treatment of various diseases related to lipid metabolic diseases, for example, diabetes mellitus, obesity, cancer, arterial sclerosis, hyperlipidemia or neurodegenerative diseases, neuropathy and the like.

### DISCLOSURE OF THE INVENTION

The present inventors have intensively studied and finally succeeded in cloning a cDNA having a novel nucleic acid sequence from a cDNA library derived from human liver, human brain, and human testis, respectively. Further, the present inventors have found that a protein encoded by the obtained cDNA is an SSD-containing protein. Based on this knowledge, the present inventors have further investigated and as a result, completed the present invention.
Thus, the present invention relates to:
(1) A protein or salt thereof, comprising the same or substantially the same amino acid sequence as that shown by SEQ ID NO: 16,
(2) The protein or salt thereof according to (1), comprising the same or substantially the same amino acid sequence as that shown by SEQ ID NO: 17,
(3) The protein or salt thereof according to (1), comprising the same or substantially the same amino acid sequence as that shown by SEQ ID NO: 34,
(4) The protein or salt thereof according to (1), comprising the same or substantially the same amino acid sequence as that shown by SEQ ID NO: 35,
(5) The protein or salt thereof according to (1), comprising the same or substantially the same amino acid sequence as that shown by SEQ ID NO: 40,
(6) A protein or salt thereof, comprising the same or substantially the same amino acid sequence as that shown by SEQ ID NO: 8, but not comprising the amino acid sequence shown by SEQ ID NO. 38,
(7) The protein or salt thereof according to (6), comprising the same or substantially the same amino acid sequence as that shown by SEQ ID NO: 9,
(8) DNA comprising DNA containing a nucleic acid sequence encoding (i) the protein of (1) or (ii) a protein comprising the same or substantially the same amino acid sequence as that shown by SEQ ID NO: 8, but not comprising the amino acid sequence shown by SEQ ID NO. 38,
(9) The DNA of (8), comprising (i) the nucleic acid sequence of 64-th to 3999-th bases of the nucleic acid sequence shown by SEQ ID NO: 7, (ii) the nucleic acid sequence shown by SEQ ID NO: 15, (iii) the nucleic acid sequence shown by SEQ ID NO: 32, (iv) the nucleic acid sequence shown by SEQ ID NO: 33, or (v) the nucleic acid sequence shown by SEQ ID NO: 41,
(10) A recombinant vector comprising DNA of (8),
(11) A transformant transformed with the recombinant vector of (10),
(12) A method of producing (i) the protein of (1) or (ii) a protein comprising the same or substantially the same amino acid sequence as that shown by SEQ ID NO: 8, but not comprising the amino acid sequence shown by SEQ ID NO, 38, or salt thereof, comprising culturing the transformant of (11) to produce the protein,
(13) An antibody to (i) the protein of (1) or (ii) a protein comprising the same or substantially the same amino acid sequence as that shown by SEQ ID NO: 8, but not comprising the amino acid sequence shown by SEQ ID NO. 38, or salt thereof,
(14) A medicine comprising (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof,
(15) A medicine comprising DNA of (8),
(16) The medicine of (14) or (15) which is a prophylactic and/or therapeutic agent for diabetes mellitus, obesity, cancer, arterial sclerosis, hyperlipidemia, neurodegenerative diseases or neuropathy,
(17) A diagnostic agent for diabetes mellitus, obesity, cancer, arterial sclerosis, hyperlipidemia, neurodegenerative diseases or neuropathy, comprising DNA of (8) or antibody of (13),
(18) A method of screening a compound or salt thereof promoting or inhibiting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, characterized by use of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof,
(19) A kit for screening a compound or salt thereof promoting or inhibiting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, the kit comprising (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof,
(20) A compound or salt thereof promoting or inhibiting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (18) or the screening kit of (19),
(21) A medicine comprising a compound or salt thereof promoting or inhibiting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (18) or the screening kit of (19),
(22) A prophylactic and/or therapeutic agent for diabetes mellitus, obesity, cancer, arterial sclerosis, hyperlipidemia, neurodegenerative diseases or neuropathy, comprising a compound or salt thereof promoting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (18) or the screening kit of (19),
(23) A method of screening a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, characterized by use of (i) the protein of (1) or (ii) a protein comprising the same or substantially the same amino acid sequence as that shown by SEQ ID NO: 8 or salt thereof,
(24) A kit for screening a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, the kit comprising (i) the protein of (1) or (ii) a protein comprising the same or substantially the same amino acid sequence as that shown by SEQ ID NO: 8 or salt thereof,
(25) A compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (23) or the screening kit of (24),
(26) A medicine comprising a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (23) or the screening kit of (24),
(27) A prophylactic and/or therapeutic agent for diabetes mellitus, obesity, cancer, arterial sclerosis, hyperlipidemia, neurodegenerative diseases or neuropathy, comprising a compound or salt thereof enhancing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (23) or the screening kit of (24),
(28) A method of quantifying mRNA of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized by use of DNA of (8) or a part thereof,
(29) A method of quantifying (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized by use of the antibody of (13),
(30) A method of diagnosing diseases related to the functions of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized by use of the quantifying method of (28) or (29),
(31) A method of screening a compound or salt thereof changing the expression amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized by use of the quantifying method of (28),
(32) A compound or salt thereof changing the expression amount of (i) the protein of (1) or (ii) a protein comprising the same or substantially the same amino acid sequence as that shown by SEQ ID NO: 8 (preferably a protein comprising the same or substantially the same amino acid sequence as that shown by SEQ ID NO: 8, but not comprising the amino acid sequence shown by SEQ ID NO. 38), obtainable using the screening method of (31),
(33) A medicine comprising a compound or salt thereof changing the expression amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (31),
(34) A prophylactic and/or therapeutic agent for diabetes mellitus, obesity, cancer, arterial sclerosis, hyperlipidemia, neurodegenerative diseases or neuropathy, comprising a compound or salt thereof increasing the expression amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (31),
(35) A method of screening a compound or salt thereof changing the the intracellular amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized by use of the quantifying method of (29),
(36) A compound or salt thereof changing the the intracellular amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (35),
(37) A medicine comprising a compound or salt thereof changing the the intracellular amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (35),
(38) A prophylactic and/or therapeutic agent for diabetes mellitus, obesity, cancer, arterial sclerosis, hyperlipidemia, neurodegenerative diseases or neuropathy, comprising a compound or salt thereof increasing the the intracellular amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (35),
(39) A prophylactic and/or therapeutic agent for diabetes mellitus, obesity, cancer, arterial sclerosis, hyperlipidemia, neurodegenerative diseases or neuropathy, comprising a compound or salt thereof having an action on a SSD-containing protein to manifest the regulation of intracellular cholesterol transport,
(40) A method of preventing and treating diabetes mellitus, obesity, cancer, arterial sclerosis, hyperlipidemia, neurodegenerative diseases or neuropathy, comprising administering to a mammal an effective amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof,
(41) A method of preventing and treating diabetes mellitus, obesity, cancer, arterial sclerosis, hyperlipidemia, neurodegenerative diseases or neuropathy, comprising administering to a mammal an effective amount of DNA of (8),
(42) A method of preventing and treating diabetes mellitus, obesity, cancer, arterial sclerosis, hyperlipidemia, neurodegenerative diseases or neuropathy, comprising administering to a mammal an effective amount of a compound or salt thereof promoting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (18) or the screening kit of (19),
(43) A method of preventing and treating diabetes mellitus, obesity, cancer, arterial sclerosis, hyperlipidemia, neurodegenerative diseases or neuropathy, comprising administering to a mammal an effective amount of a compound or salt thereof enhancing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (23) or the screening kit of (24),
(44) A method of preventing and treating diabetes mellitus, obesity, cancer, arterial sclerosis, hyperlipidemia, neurodegenerative diseases or neuropathy, comprising administering to a mammal an effective amount of a compound or salt thereof increasing the expression amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (31),
(45) A method of preventing and treating diabetes mellitus, obesity, cancer, arterial sclerosis, hyperlipidemia, neurodegenerative diseases or neuropathy, comprising administering to a mammal an effective amount of a compound or salt thereof having an action on a SSD-containing protein to manifest the regulation of intracellular cholesterol transport,
(46) Use of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, for producing a prophylactic and/or therapeutic agent for diabetes mellitus, obesity, cancer, arterial sclerosis, hyperlipidemia, neurodegenerative diseases or neuropathy,
(47) Use of DNA of (8), for producing a prophylactic and/or therapeutic agent for diabetes mellitus, obesity, cancer, arterial sclerosis, hyperlipidemia, neurodegenerative diseases or neuropathy,
(48) Use of a compound or salt thereof promoting the activity of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (18) or the screening kit of (19), for producing a prophylactic and/or therapeutic agent for diabetes mellitus, obesity, cancer, arterial sclerosis, hyperlipidemia, neurodegenerative diseases or neuropathy,
(49) Use of a compound or salt thereof enhancing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, obtainable using the screening method of (23) or the screening kit of (24), for producing a prophylactic and/or therapeutic agent for diabetes mellitus, obesity, cancer, arterial sclerosis, hyperlipidemia, neurodegenerative diseases or neuropathy,
(50) Use of a compound or salt thereof increasing the expression amount of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening method of (31), for producing a prophylactic and/or therapeutic agent for diabetes mellitus, obesity, cancer, arterial sclerosis, hyperlipidemia, neurodegenerative diseases or neuropathy,
(51) Use of a compound or salt thereof having an action on a SSD-containing protein to manifest the regulation of intracellular cholesterol transport, for producing a prophylactic and/or therapeutic agent for diabetes mellitus, obesity, cancer, arterial sclerosis, hyperlipidemia, neurodegenerative diseases or neuropathy.
   Further, the present invention provides:
(52) The protein or salt thereof according to (1) wherein the protein is a protein comprising (i) an amino acid sequence having deletion of 1 or more (preferably 1 to about 30, more preferably 1 to about 9, further preferably several (1 to 5)) amino acids in the amino acid sequence shown by SEQ ID NO: 16, (ii) an amino acid sequence having addition of 1 or more (preferably 1 to about 30, more preferably 1 to about 10, further preferably several (1 to 5)) amino acids in the amino acid sequence shown by SEQ ID NO: 16, (iii) an amino acid sequence having substitution of 1 or more (preferably 1 to about 30, more preferably 1 to about 10, further preferably several (1 to 5)) amino acids by other amino acids in the amino acid sequence shown by SEQ ID NO: 16 or (iv) an amino acid sequence having a combination thereof,
(53) The protein or salt thereof according to (6) wherein the protein is a protein comprising (i) an amino acid sequence having deletion of 1 or more (preferably 1 to about 30, more preferably 1 to about 9, further preferably several (1 to 5)) amino acids in the amino acid sequence shown by SEQ ID NO: 8, (ii) an amino acid sequence having addition of 1 or more (preferably 1 to about 30, more preferably 1 to about 10, further preferably several (1 to 5)) amino acids in the amino acid sequence shown by SEQ ID NO: 8, (iii) an amino acid sequence having substitution of 1 or more (preferably 1 to about 30, more preferably 1 to about 10, further preferably several (1 to 5)) amino acids by other amino acids in the amino acid sequence shown by SEQ ID NO: 8 or (iv) an amino acid sequence having a combination thereof, and not containing the amino acid sequence shown by SEQ ID NO: 38,
(54) A partial peptide of the protein of any of (1) to (3),
(55) The partial peptide according to (54) having the sterol-sensing ability,
(56) The screening method according to (23) comprising comparing a case (I) of contacting a sterol to (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, and a case (II) of contacting a sterol and a test compound to (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt protein thereof,
(57) A method of screening a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized in that, in a case (i) of contacting a sterol to a cell containing (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) and in a case (ii) of contacting a sterol and a test compound to a cell containing (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), cell-stimulating activities via the proteins are measured and compared,
(58) A method of screening a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), characterized in that, in a case of contacting a sterol to the protein expressed on cell membrane of the cultured transformant of (11), and in a case of contacting a sterol and a test compound to the protein expressed on cell membrane of the cultured transformant of (11), cell-stimulating activities via the proteins are measured and compared,
(59) A compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable by the screening method of (56) or (57),
(60) A medicine comprising a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable by the screening method of (57) or (58),
(61) The screening kit according to (24), comprising a cell containing (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38),
(62) The screening kit according to (24), comprising a membrane fraction of a cell containing (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38),
(63) The screening kit according to (24), comprising the protein expressed on cell membrane of the cultured transformant of (11),
(64) A compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening kit of (61) to (63),
(65) A medicine comprising a compound or salt thereof changing the sterol-sensing ability of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38), obtainable using the screening kit of (61) to (63),
(66) A method of quantifying (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or (iii) a partial peptide of the protein of (1) or (iv) a partial peptide of a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, characterized by contacting the antibody of (13) with (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or (iii) a partial peptide of the protein of (1) or (iv) a partial peptide of a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof,
(67) A method of quantifying (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or (iii) a partial peptide of the protein of (1) or (iv) a partial peptide of a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof in specimen liquid, characterized by reacting the antibody of (13) competitively with a test liquid and a labeled form of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or (iii) a partial peptide of the protein of (1) or (iv) a partial peptide of a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof, and measuring the ratio of labeled form of (i) the protein of (1) or (ii) a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or (iii) a partial peptide of the protein of (1) or (iv) a partial peptide of a protein comprising the same or substantially the same amino acid sequence as that shown by SEQ ID NO: 8 (preferably, a protein comprising the same or substantially the same amino acid sequence as that shown by SEQ ID NO: 8, but not comprising the amino acid sequence shown by SEQ ID NO. 38) or salt thereof which are bound to the antibody,
(68) A method of quantifying (i) the protein of (1) or (ii) a protein comprising the same or substantially the same amino acid sequence as that shown by SEQ ID NO: 8 (preferably, a protein comprising the same or substantially the same amino acid sequence i as that shown by SEQ ID NO: 8, but not comprising the amino acid sequence shown by SEQ ID NO. 38) or salt thereof in a test liquid, characterized by reacting a test liquid, simultaneously or continuously, with the antibody of (13) immobilized on a carrier and the labeled antibody of (13), and then measuring the activity of the label on the immobilized carrier,
   and the like.

### BRIEF DESCRIPTION OF DRAWINGS

Fig. 1 shows the result of analyzing tissue specific expression of SSP1 mRNA. The figure shows the results of heart, brain, placenta, lung, liver, skeletal muscle, kidney, pancreas, spleen, thymus, prostate, testis, ovary, small intestine, colon, PBL (peripheral blood monocyte), stomach, thyroid, spinal cord, trachea, adrenal gland and bone marrow.
Fig. 2 shows the result of analyzing tissue specific expression of SSP2 mRNA. The figure shows the results of heart, brain, placenta, lung, liver, skeletal muscle, kidney, pancreas, spleen, thymus, prostate, testis, ovary, small intestine, colon, PBL (peripheral blood monocyte), stomach, thyroid, spinal cord, trachea, adrenal gland and bone marrow.
Fig. 3 shows the analysis of SSP1 expression. In the figure, the abscissa shows the copy concentration of SSP1 cDNA in reaction liquid (copies/µl), and the ordinate shows the PCR cycle number required for attaining the detection region.
Fig. 4 shows the analysis of SSP2 expression. In the figure, the abscissa shows the copy concentration of SSP2 cDNA in reaction liquid (copies/µl), and the ordinate shows the PCR cycle number required for attaining the detection region.
Fig. 5 shows the result of investigating tissue specific gene expression of SSP1. In the figure, the ordinate shows Caco-2 (colon cancer-derived human cell strain, Caco-2), HepG2 (liver cancer-derived human cell strain, HepG2), M. Gland (mammary gland), B. Marrow (bone marrow), Adipocyte, Retina, Uterus, Prostate, Spleen, Pancreas, Fetus Brain, P. Gland (pituitary gland), Testis, Leukocyte, Colon, A. Gland (adrenal gland), Ovary, S. Muscle (smooth muscle), S. Intestine (small intestine), Lung, Liver, Kidney, Heart and Fetus. The abscissa shows the expression amount of a gene.
Fig. 6 shows the result of investigating the site-specific expression of SSP1 mRNA in regions of small intestine by northern blotting.
Fig. 7 shows the result of investigating the change of SSP1 gene expression by the intracellular cholesterol level in HepG2.
Fig. 8 shows the result of investigating neuroblastoma-specific expression of SSP2 gene among nerve cell lines and induction of SSP2 gene expression in IMR-32 cell in differentiation-dependent manner. The abscissa shows the expression amount of SSP2 gene.
Fig. 9 shows the result of investigating the amount of SSP1 protein produced by a stable SSP1-expression cell.
Fig. 10 shows the result of investigating the secretion amount of ApoB lipoprotein in a stable SSP1-overexpression HepG2 cell. The ordinate shows the secretion amount of ApoB lipoprotein.
Fig. 11 shows the results of investigating the site-specific expression of SSP2 mRNA in the brain of Alzheimer patients. The ordinate shows Cerebellum, Amygdalah, Hippocampus, Temporal Lobe, Parietal Lobe, Occipital Lobe and Frontal Lobe. The abscissa shows the expression amount of SSP2 mRNA.

### BEST MODES FOR CARRYING OUT THE INVENTION

The protein of the present invention is (1) a protein comprising the same or substantially the same amino acid sequence as that (SSD sequence) shown by SEQ ID NO: 16, or (2) a protein comprising the same or substantially the same amino acid sequence as that (SSD sequence) shown by SEQ ID NO: 8, but not comprising the amino acid sequence shown by SEQ ID NO. 38.

The protein of the present invention may be any protein derived from any cells (e.g., retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testis, ovary, placenta, uterus, bone, joint, skeletal muscle, etc. (especially brain or any of brain regions) from human and other mammalians (e.g., guinea pigs, rats, mice, rabbits, swine, sheep, bovine, monkeys, etc.). The protein may also be a synthetic protein.

The substantially same amino acid sequence as that shown by SEQ ID NO: 16 includes, for example, amino acid sequences having about 50% or more, preferably about 60% or more, more preferably about 70% or more, further preferably about 80% or more, specifically preferably about 90% or more, most preferably about 95% or more homology with an amino acid sequence shown by SEQ ID NO: 16.

As the protein of the present invention comprising substantially the same amino acid sequence as that shown by SEQ ID NO: 16, preferable are, for example, proteins comprising substantially the same amino acid sequence as that shown by SEQ ID NO: 16 and having substantially the same activity with that of the protein having the amino acid sequence shown by SEQ ID NO: 16.

Specific examples of the amino acid sequence shown by SEQ ID NO: 16 of the present invention include:
(1) proteins comprising the amino acid sequence shown by SEQ ID NO: 17 (for example, human testis-derived SSP2),
(2) proteins comprising the amino acid sequence shown by SEQ ID NO: 34 (for example, human testis-derived immature SSP2),
(3) proteins comprising the amino acid sequence shown by SEQ ID NO: 35 (for example, human testis-derived immature SSP2),
(4) proteins comprising the amino acid sequence shown by SEQ ID NO: 40 (for example, human brain-derived SSP2).

Further, the protein of the present invention also include proteins having substantially the same amino acid sequence as that shown by SEQ ID NO: 17, SEQ ID NO: 34, SEQ ID NO: 35 or SEQ ID NO: 40.

The substantially same amino acid sequence as that shown by SEQ ID NO: 17, SEQ ID NO: 34, SEQ ID NO: 35 or SEQ ID NO: 40 are, for example, amino acid sequences having about 50% or more, preferably about 60% or more, more preferably about 70% or more, further preferably about 80% or more, specifically preferably about 90% or more, most preferably about 95 % or more homology with the amino acid sequence shown by SEQ ID NO: 17, SEQ ID NO: 34, SEQ ID NO: 35 or SEQ ID NO: 40.

As the protein of the present invention comprising substantially the same amino acid sequence as that shown by SEQ ID NO: 17, SEQ ID NO: 34, SEQ ID NO: 35 or SEQ ID NO: 40, preferable are, for example, proteins comprising substantially the same amino acid sequence as that shown by SEQ ID NO: 17, SEQ ID NO: 34, SEQ ID NO: 35 or SEQ ID NO: 40 and having substantially the same activity with that of the protein having the amino acid sequence shown by SEQ ID NO: 17, SEQ ID NO: 34, SEQ ID NO: 35 or SEQ ID NO: 40.

The substantially same amino acid sequence as that shown by SEQ ID NO: 8 includes, for example, amino acid sequences having about 50% or more, preferably about 60% or more, more preferably about 70% or more, further preferably about 80% or more, specifically preferably about 90% or more, most preferably about 95% or more homology with the amino acid sequence shown by SEQ ID NO: 8.

As the protein of the present invention comprising substantially the same amino acid sequence as that shown by SEQ ID NO: 8, preferable are, for example, proteins comprising substantially the same amino acid sequence as that shown by SEQ ID NO: 8 and having substantially the same activity with that of the protein having the amino acid sequence shown by SEQ ID NO: 8.

The protein of the present invention comprising substantially the same amino acid sequence as that shown by SEQ ID NO: 8 and no amino acid sequence as that shown by SEQ ID NO: 38 includes, more specifically, proteins comprising (i) the 22-th to 1332-th residues of the amino acid sequence shown by SEQ ID NO: 9 or (ii) the amino acid sequence shown by SEQ ID NO: 9 (for example, human liver-derived SSP1).

Further, the protein of the present invention also include proteins comprising substantially the same amino acid sequence as (i) the 22-th to 1332-th residues of the amino acid sequence shown by SEQ ID NO: 9 or (ii) the amino acid sequence shown by SEQ ID NO: 9.

The substantially same amino acid sequence as (i) the 22-th to 1332-th residues of the amino acid sequence shown by SEQ ID NO: 9 or (ii) the amino acid sequence shown by SEQ ID NO: 9 includes, for example, amino acid sequences having about 50% or more, preferably about 60% or more, more preferably about 70% or more, further preferably about 80% or more, specifically preferably about 90% or more, most preferably about 95% or more homology with (i) the 22-th to 1332-th residues of the amino acid sequence shown by SEQ ID NO: 9 or (ii) the amino acid sequence shown by SEQ ID NO: 9.

As the protein of the present invention comprising substantially the same amino acid sequence as (i) the 22-th to 1332-th residues of the amino acid sequence shown by SEQ ID NO: 9 or (ii) the amino acid sequence shown by SEQ ID NO: 9, preferable are, for example, proteins comprising substantially the same amino acid sequence as (i) the 22-th to 1332-th residues of the amino acid sequence shown by SEQ ID NO: 9 or (ii) the amino acid sequence shown by SEQ ID NO: 9 and having substantially the same activity with that of the protein having (i) the 22-th to 1332-th residues of the amino acid sequence shown by SEQ ID NO: 9 or (ii) the amino acid sequence shown by SEQ ID NO: 9.

The activity in the term "substantially the same activity" includes the sterol (preferably, cholesterol)-sensing ability and the like. The term "substantially the same" is used in terms of property. Therefore, it is preferable that an activity such as the sterol-sensing ability are equivalent (for example, about 0.01 to 100 fold, preferably about 0.5 to 20 fold, more preferably about 0.5 to 2 fold), however, quantitative factors such as the activity level and the molecular weight of the proteins may be different.

Measurement of an activity such as the sterol-sensing ability can be carried out in accordance with a method of measuring cell response (cell-stimulating activity and the like) depending on the sterol concentration, or a modified method thereof, and for example, the screening method described later.

Also used as the protein of the present invention are proteins comprising (i) an amino acid sequence having deletion of 1 or more (preferably, 1 to about 30, more preferably, 1 to about 10, further preferably, several (1 to 5)) amino acids in the amino acid sequence shown by SEQ ID NO: 16, (ii) an amino acid sequence having addition of 1 or more (preferably, 1 to about 30, more preferably, 1 to about 10, further preferably, several (1 to 5)) amino acids in the amino acid sequence shown by SEQ ID NO: 16, (iii) an amino acid sequence having substitution of 1 or more (preferably, 1 to about 30, more preferably, 1 to about 10, further preferably, several (1 to 5)) amino acids by other amino acids in an amino acid sequence shown by SEQ ID NO: 16 or (iv) an amino acid sequence having a combination of the deletion, addition and substitution.

Further, also used as the protein of the present invention are proteins comprising (i) an amino acid sequence having deletion of 1 or more (preferably, 1 to about 30, more preferably, 1 to about 10, further preferably, several (1 to 5)) amino acids in the amino acid sequence shown by SEQ ID NO: 8, (ii) an amino acid sequence having addition of 1 or more (preferably, 1 to about 30, more preferably, 1 to about 10, further preferably, several (1 to 5)) amino acids in the amino acid sequence shown by SEQ ID NO: 8, (iii) an amino acid sequence having substitution of 1 or more (preferably, 1 to about 30, more preferably, 1 to about 10, further preferably, several (1 to 5)) amino acids by other amino acids in an amino acid sequence shown by SEQ ID NO: 8 or (iv) an amino acid sequence having a combination of the deletion, addition and substitution, and comprising no amino acid sequence shown by SEQ ID NO: 3.

Furthermore, also used as the protein of the present invention are proteins comprising (i) an amino acid sequence having deletion of 1 or more (preferably, 1 to about 30, more preferably, 1 to about 10, further preferably, several (1 to 5)) amino acids in the 22-th to 1332-th residues of the amino acid sequence shown by SEQ ID NO: 9, the amino acid sequence shown by SEQ ID NO: 9, or the amino acid sequence shown by SEQ ID NO: 17, SEQ ID NO: 34, SEQ ID NO: 35, or SEQ ID NO: 40, (ii) an amino acid sequence having addition of 1 or more (preferably, 1 to about 30, more preferably, 1 to about 10, further preferably, several (1 to 5)) amino acids in the 22-th to 1332-th residues of the amino acid sequence shown by SEQ ID NO: 9, the amino acid sequence shown by SEQ ID NO: 9, or the amino acid sequence shown by SEQ ID NO: 17 or SEQ ID NO: 40, (iii) an amino acid sequence having substitution of 1 or more (preferably, 1 to about 30, more preferably, 1 to about 10, further preferably, several (1 to 5)) amino acids by other amino acids in the 22-th to 1332-th residues of the amino acid sequence shown by SEQ ID NO: 9, the amino acid sequence shown by SEQ ID NO: 9, or the amino acid sequence shown by SEQ ID NO: 17 or SEQ ID NO: 40, or (iv) an amino acid sequence having a combination of the deletion, addition and substitution.

However, the protein of the present invention does not include proteins containing an amino acid sequence in which the amino acid sequence sown by SEQ ID NO: 38 is inserted into the amino acid sequence sown by SEQ ID NO: 9 (Genomics, 65, 137-145, 2000).

Preferably, the protein of the present invention excludes a protein having an amino acid sequence encoded by a DNA having the nucleic acid sequence sown by SEQ ID NO: 42.

Throughout the present specification, proteins are represented in accordance with the conventional way of describing peptides, that is, the N-terminus (amino terminus) at the left hand and the C-terminus (carboxyl terminus) at the right hand. In the proteins of the present invention including the protein comprising the amino acid sequence shown by SEQ ID NO:8, the C-terminus is usually in the form of a carboxyl group (-COOH) or a carboxylate (-COO⁻) but may be in the form of an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂-alkyl group, e.g., benzyl, phenethyl, etc., or an α-naphthyl-C₁₋₂-alkyl group such as α-naphthylmethyl, etc.; and the like. In addition, pivaloyloxymethyl or the like, which is used widely as an ester for oral administration, may also be used.

When the protein of the present invention contains a carboxyl group (or a carboxylate) at a position other than the C-terminus, it may be amidated or esterified and such an amide or ester is also included within the protein of the present invention. The ester group may be the same group as that described with respect to the C-terminus described above.

Furthermore, the proteins of the present invention include variants of the above-mentioned proteins, wherein the amino group at the N-terminal methionine residue of the protein supra is protected with a protecting group (for example, a C₁₋₆ acyl group such as a C₂₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₂₋₆ alkanoyl group, e.g., formyl group, acetyl group, etc.), or conjugated proteins such as glycoproteins bound to sugar chains.

As a partial peptide of the protein of the present invention (hereinafter sometimes referred to as the partial peptide), any partial peptide can be used so long as it can be a partial peptide of the above-mentioned protein. Of the protein of the present invention, a transmembrane region having the binding activity to a sterol can be used.

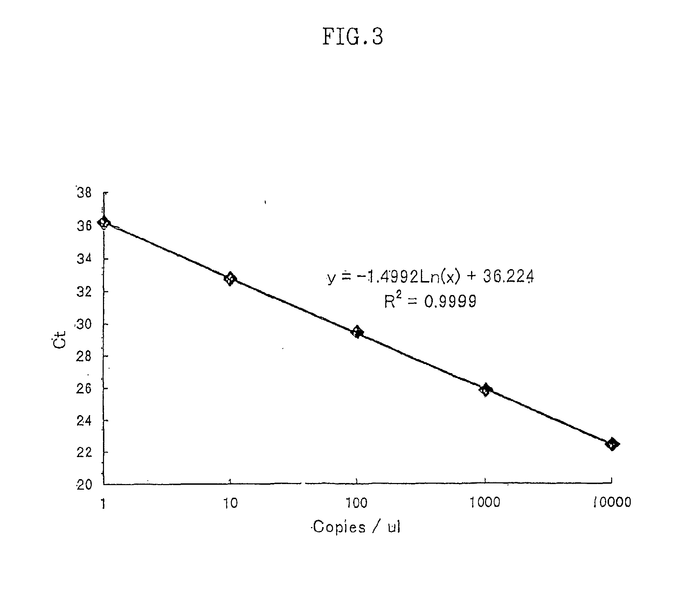

Specifically, an example of the partial peptide of (1) the protein having the amino acid sequence shown by SEQ ID NO: 16 or (2) the protein having the amino acid sequence shown by SEQ ID NO:8 but not having the amino acid sequence shown by SEQ ID NO:38 is a peptide containing a transmembrane region (hydrophobic region) deduced by the hydrophobic plotting analysis, which is shown in FIG. 3. The peptide containing a hydrophilic region in part can be used as well. In addition, the partial peptide may contain a single domain or plural domains together.

Preferred are partial peptides having at least 20, preferably at least 50, and more preferably at least 100 amino acids in the constituent amino acids of the protein of the present invention.

The substantially same amino acid sequence includes an amino acid sequence having at least about 50% homology, preferably at least about 70% homology, more preferably at least about 80% homology, much more preferably at least about 90% homology, and most preferably at least about 95% homology to these amino acid sequences.

Herein, the term "substantially the same activity" is as defined above. The "substantially the same activity" can be measured as described above.

The partial peptide of the present invention may include the above-mentioned amino acid sequence in which 1 or more (preferably approximately 1 to 10, more preferably several (1 to 5)) amino acids are deleted; to which 1 or more (preferably approximately 1 to 20, more preferably approximately 1 to 10, and most preferably several (1to 5)) amino acids are added; or in which 1 or more (preferably approximately 1 to 10, more preferably several and most preferably approximately 1 to 5) amino acids are substituted by other amino acids.

In the partial peptide of the present invention, the C-terminus is normally a carboxyl group (-COOH) or carboxylate (-COO⁻) but the C-terminus may be in the form of an amide (-CONH₂) or an ester (-COOR), as described in the protein of the present invention.

As described in the protein of the present invention, the partial peptide of the present invention also includes those in which the amino group of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycoproteins, to which sugar chains are bound.

In the partial peptide of the present invention, the C-terminus is normally a carboxyl group (-COOH) or carboxylate (-COO⁻) but the C-terminus may be in the form of an amide (-CONH₂) or an ester (-COOR), as described in the protein of the present invention.

A salt of the protein or the partial peptide of the present invention is a physiologically acceptable salt with an acid or base. Especially, a physiologically acceptable acid addition salt is preferred. Examples of the salt include, for example, a salt with an inorganic acid (e.g., hydrochloric acid, phosphoric acid, hydrobromic acid, sulfuric acid) or with an organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein of the present invention or salt thereof may be produced by a publicly known purification method from human or other mammalian cells or tissues as described above, or by culturing a transformant transformed by a DNA encoding the protein of the present invention, as later described. Furthermore, the protein or its salt may also be produced by the synthesizing method as described below or modified method thereof.

When the protein or its salt is produced from human or mammalian tissues or cells, after homogenization of human or mammalian tissues or cells, extraction with an acid or the like, and isolation and purification by a combination of chromatography techniques such as reverse phase chromatography, ion exchange chromatography, and the like are carried out.

To synthesize the protein of the present invention, a partial peptide, or a salt or amide thereof according to the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (remove) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide of the protein of the present invention or a salt thereof can be produced by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part. Where the product contains protecting groups, these protecting groups are removed to give the desired peptide. Publicly known methods for condensation and elimination of the protecting groups are described in 1) - 5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

Using a DNA encoding the protein of the present invention, mRNA of the protein of the present invention can be quantified by, for example, the publicly known method published in separate volume of *Jikken Igaku* 15(7) "New PCR and its application" (1997), or by its modifications.

The DNA encoding the protein of the present invention may be derived from any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the protein of the present invention may be any DNA having the nucleic acid sequence shown by SEQ ID NO:28 or SEQ ID NO:29, or the nucleic acid sequence hybridizable to the nucleic acid sequence shown by SEQ ID NO:28 or SEQ ID NO:29 under highly stringent conditions and encoding a protein having substantially the same activity as that of the protein of the present invention (e.g., the sterol (preferably choresterol) sensing ability, etc.).

Specific examples of the DNA hybridizable to the nucleic acid sequence shown by SEQ ID NO:28 or SEQ ID NO:29 include DNA containing a nucleic acid sequence having at least about 70% homology, preferably at least about 80% homology, more preferably at least about 90% homology and most preferably at least about 95% homology to the nucleic acid sequence shown by SEQ ID NO:28 or SEQ ID NO:29.

However, the DNA encoding the protein of the present invention does not include a DNA having a nucleic acid sequence in which the nucleic acid sequence sown by SEQ ID NO: 39 is inserted into the nucleic acid sequence sown by SEQ ID NO: 7 (Genomics, 65, 137-145, 2000).

Preferably, the DNA encoding the protein of the present invention excludes a DNA having the nucleic acid sequence sown by SEQ ID NO: 42.

The hybridization can be carried out by publicly known methods or by modifications thereof, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, the DNA encoding the protein comprising the amino acid sequence shown by SEQ ID NO: 8 includes the DNA comprising the nucleic acid sequence shown by SEQ ID NO:28; and the DNA encoding the protein comprising the amino acid sequence shown by SEQ ID NO: 16 includes the DNA comprising the nucleic acid sequence shown by SEQ ID NO:29.

Further, the DNA encoding the protein comprising the amino acid sequence shown by SEQ ID NO:8 but not comprising the amino acid sequence shown by SEQ ID NO:38 includes the DNA comprising the nucleic acid sequence shown by SEQ ID NO:28 but not the nucleic acid sequence shown by SEQ ID NO:39

In addition, (1) the DNA encoding the protein comprising the 22-th to 1332-th residues of the amino acid sequence shown by SEQ ID NO:9 includes the DNA comprising the 64-th to 3999-th bases of the nucleic acid sequence shown by SEQ ID NO:7; (2) the DNA encoding the protein comprising the amino acid sequence shown by SEQ ED NO:9 includes the DNA comprising the nucleic acid sequence shown by SEQ ID NO:7; (3) the DNA encoding the protein comprising the amino acid sequence shown by SEQ ID NO: 17 includes the DNA comprising the nucleic acid sequence shown by SEQ ID NO: 15; (4) the DNA encoding the protein comprising the amino acid sequence shown by SEQ ID NO:34 includes the DNA comprising the nucleic acid sequence shown by SEQ ID NO:32; (5) the DNA encoding the protein comprising the amino acid sequence shown by SEQ ID NO:35 includes the DNA comprising the nucleic acid sequence shown by SEQ ID NO:33; and (6) the DNA encoding the protein comprising the amino acid sequence shown by SEQ ID NO:40 includes the DNA comprising the nucleic acid sequence shown by SEQ ID NO:41.

For cloning of the DNA that completely encodes the protein of the present invention or a partial peptide thereof (hereinafter sometimes referred to as the protein of the present invention), the DNA may be either amplified by PCR using synthetic DNA primers containing a part of the nucleic acid sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using a commercially available library in accordance with the protocol described in the attached instruction.

Conversion of the DNA sequence can be effected in accordance with a publicly known method such as the Gupped duplex method or the Kunkel method or its modification, using a publicly known kit available as Mutan™-G or Mutan™-K (Takara Shuzo Co., Ltd.).

The cloned DNA encoding the protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the protein of the present invention can be produced, for example, by (a) excising the desired DNA fragment from the DNA encoding the protein of the present invention, and then (b) ligating the DNA fragment downstream of a promoter in an appropriate expression vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ-phage, etc., animal viruses such as retrovirus, vaccinia virus, baculovirus, etc. as well as pA1-11, pXTl, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc. Among them, CMV promoter or SRα promoter is preferably used.

When the host is bacteria of the genus Escherichia, preferred examples of the promoter include trp promoter, lac promoter, recA promoter, λP_{L} promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary and desired, a signal sequence that matches with a host is added to the N-terminus of the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr⁻) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the protein can be obtained.

When the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

When the bacteria belonging to the genus Escherichia are used as the host, the transformant is usually cultivated at about 15°C to about 43°C for about 3 hours to about 24 hours. If necessary and desired, the culture may be aerated or agitated.

When the bacteria belonging to the genus Bacillus are used as the host, the transformant is cultivated generally at about 30°C to about 40°C for about 6 hours to about 24 hours. If necessary and desired, the culture can be aerated or agitated.

When yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

When insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

When animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30° C to about 40° C for about 15 hours to about 60 hours and, if necessary and desired, the culture can be aerated or agitated.

As described above, the protein of the present invention can be produced inside or outside of the transformant cell, or in the plasma membrane.

The protein of the present invention can be separated and purified from the culture described above by the following procedures.

When the protein of the present invention is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™, etc. When the protein is secreted in the culture, after completion of the cultivation the supernatant can be separated from the transformants or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase or the like.

The activity of the thus produced protein of the present invention or salts thereof can be determined by a test binding to a labeled ligand, by an enzyme immunoassay using a specific antibody, or the like.

Antibodies to the protein of the present invention or salts thereof may be any of polyclonal antibodies and monoclonal antibodies, as long as they are capable of recognizing the protein of the present invention or salts thereof.

The antibodies to the protein of the present invention or salts thereof (hereinafter sometimes merely referred to as the protein of the present invention) may be manufactured by publicly known methods for manufacturing antibodies or antisera, using as an antigen the protein of the present invention.

### [Preparation of monoclonal antibody]

### (a) Preparation of monoclonal antibody-producing cells

The protein of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20° C to 40°C, preferably at about 37°C, for 5 days to 3 weeks, preferably 1 to 2 weeks. The cultivation can be conducted normally in 5% CO₂. The antibody titer of the culture supernatant of hybridomas can be determined as in the assay for the antibody titer in antisera described above.

### (b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, a complex of immunogen (a protein antigen) and a carrier protein is prepared, and a mammal is immunized with the complex in a manner similar to the method described above for the manufacture of monoclonal antibodies. The product containing the antibody to the protein of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

According to the present invention, an antisense polynucleotide that can inhibit the replication or expression of the gene encoding the protein can be designed and synthesized based on the base sequence information of the cloned or determined DNA encoding the protein. Such a polynucleotide is capable of hybridizing to mRNA of the gene to inhibit the synthesis or function of said RNA or capable of modulating or controlling the expression of the gene via interaction with the RNA encoding the protein. A DNA complementary to a selected sequence of the RNA encoding the protein and a DNA specifically hybridizable to the RNA encoding the protein are useful in modulating or controlling the expression of the gene encoding the protein in vivo and in vitro, and useful for the treatment or diagnosis of diseases. The term "corresponding" is used to mean homologous or complementary to a particular nucleotide sequence or nucleic acid sequence including a gene. The term "corresponding" between a nucleotide sequence or a nucleic acid sequence and a peptide (protein) usually means that amino acids of the peptide (protein) is under the order derived from the nucleotide (nucleic acid) sequence or its complement. In the gene, the 5'-end hairpin loop, 5'-end 6-base-pair repeats, 5'-end untranslated region, polypeptide translation initiation codon, protein coding region, ORF translation initiation codon, 3'-end untranslated region, 3'-end palindrome region, and 3'-end hairpin loop, may be selected as a preferred target region, though any other region may be selected as a target in the gene encoding the protein.

A polynucleotide complementary to at least a part of the target nucleic acid sequence, for example, a polynucleotides hybridizable to the target is supposed to be "antisense". Examples of the antisense polynucleotides include polydeoxynucleotides containing 2-deoxy-D-ribose, polydeoxynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

Examples of a nucleic acid sequence substantially complementary to the DNA of the present invention include nucleic acid sequences having at least about 70%, preferably at least about 80%, more preferably at least about 90% and most preferably at least about 95 % homology to the entire or a part of the nucleic acid sequence complementary to the DNA of the present invention (the complementary strand of the DNA of the present invention). Particularly preferred are antisense DNAs having at least about 70%, preferably at least about 80%, more preferably at least about 90% and most preferably at least about 95 % homology to a part of the whole nucleic acid sequence complementary to the DNA of the present invention, the part which encodes a N-terminal region of the protein of the present invention. These antisense DNAs can be produced a well-known DNA synthesizer, etc.

The inhibitory activity of the antisense nucleic acid can be examined using the transformant of the present invention, the gene expression system of the present invention in vivo and in vitro, or the translation system of the protein in vivo and in vitro. The nucleic acid can be applied to cells by a variety of publicly known methods.

The protein of the present invention (including a protein containing an amino acid sequence in which the amino acid sequence sown by SEQ ID NO: 38 is inserted into the amino acid sequence sown by SEQ ID NO: 9) and the DNA encoding the protein can be used for:
<1> a prophylactic and/or therapeutic agent for diseases associated with dysfunction of the protein of the present invention,
<2> an agent for genetic diagnosis,
<3> a method of screening a compound or salt thereof that changes the expression amount of the protein of the present invention,
<4> a prophylactic and/or therapeutic agent for various diseases comprising a compound or salt thereof that changes the expression amount of the protein of the present invention,
<5> a method of screening a compound or salt thereof that changes the sterol-sensing ability of the protein of the present invention,
<6> a prophylactic and/or therapeutic agent for various diseases comprising a compound or salt thereof that changes the sterol-sensing ability of the protein of the present invention,
<7> quantification of the protein of the present invention,
<8> neutralization by an antibody to the protein of the present invention, and
<9> preparation of a non-human animal carrying the DNA encoding the protein of the present invention.

In particular, using a screening method with the expression system of the recombinant protein of the present invention, a compound or salt thereof that changes the sterol-sensing ability of the protein of the present invention (hereinafter sometimes referred to as "the compound") can be selected, and then be used as a prophylactic and/or therapeutic agent for various diseases.

Hereinafter, use of the DNA encoding the protein of the present invention (hereinafter sometimes referred to as the DNA of the present invention) and the antibody to the protein of the present invention (hereinafter sometimes referred to as the antibody of the present invention) is specifically described.

### <1> A prophylactic and/or therapeutic agent for diseases associated with dysfunction of the protein of the present invention

### (i) The protein of the present invention or (ii) the DNA encoding the protein can be use as a medicine such as a prophylactic and/or therapeutic agent for diseases associated with dysfunction of the protein of the present invention.

For example, when there is a patient in which the sterol-sensing ability cannot be expected due to decrease in the protein of the present invention in vivo (deficiency in the protein), (i) the protein of the present invention can be administered to the patient to replenish the amount of the protein or (ii) the amount of the protein in the patient can be increased (a) by administering to the patient the DNA encoding the protein of the present invention and allowing DNA to express, or (b) by introducing the DNA encoding the protein of the present invention into the subject cell and allowing DNA to express, then, transplanting the cell to the patient, to sufficiently manifest the function of the protein. Thus, the DNA encoding the protein of the present invention is useful as a prophylactic and/or therapeutic agent for diseases associated with dysfunction of the protein of the present invention, which is safe and low toxic.

The protein of the present invention contains a partial sequence having about 20% or more homology with sequences of NPC 1 which is said to be a causative gene of Niemann-Pick disease type C; Patched which is said to be a signal molecule for development and differentiation; HMG-CoA Reductase which is a rate-limiting enzyme for the cholesterol biosynthesis system; and SCAP which is the main transcription control substance regulating the fatty acid synthesis system, cholesterol synthesis system and the catabolism system thereof, and sequences in the above-mentioned four proteins having homology with the partial sequence are considered to be a SSD sequence [Current Opinion in Structural Biology, 8, 435-439 (1998)]. From this, it is expected that the protein of the present invention has a function of sensing the sterol concentration, like the above-mentioned four proteins, and thus the protein of the present invention is useful for prevention and/or treatment of lipid metabolic diseases such as hyperlipidemia, arterial sclerosis and the like. Further, as the function of NPC1 having 40% or more homology with the protein of the present invention, control of lipid transportation in a cell is suggested [Current Opinion in Lipidology, 9, 131-135 (1998)]. Therefore, the protein of the present invention having homology with NPC1 is expected to control lipid transportation in a cell and useful for prevention and/or treatment of various lipid metabolism disorders, cardiovascular diseases, nerve-centric diseases, respiratory organ diseases, digestive organ diseases, live/gallbladder/pancreas diseases and endocrine diseases.

When the protein of the present invention is used as the prophylactic/therapeutic agents supra, such a pharmaceutical composition can be prepared in a conventional manner.

On the other hand, when the DNA encoding the protein of the present invention (hereinafter sometimes referred to as the DNA of the present invention) is used as the prophylactic/therapeutic agent described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered, as naked DNA or with an adjuvant to assist its uptake, by gene gun or through a catheter such as a catheter with a hydrogel.

For example, the protein of the present invention or the DNA of the present invention can be used orally, for example, in the form of tablet which may be sugar-coated if necessary and desired, capsule, elixir, microcapsule, etc., or parenterally in the form of injectable preparation such as a sterile solution and a suspension in water or with other pharmaceutically acceptable liquid. These preparations can be produced by mixing the protein of the present invention or the DNA of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is adjusted to such a dose that an appropriate dose is obtained within the specified range.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to a human or mammal (e.g., rat, rabbit, sheep, swine, bovine, cat, dog, monkey, etc.).

The dose of the protein of the present invention varies depending on subject to be administered, organs to be administered, conditions, routes for administration, etc. In oral administration, e.g., for a patient with hyperlipidemia, the dose is normally about 0.1 mg to about 100 mg, preferably about 1,0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for a patient with hyperlipidemia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, routes for administration, etc. In oral administration, e.g., for a patient with hyperlipidemia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for a patient with hyperlipidemia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### <2> An agent for genetic diagnosis

By using the DNA of the present invention as a probe, an abnormality (gene abnormality) of the DNA or mRNA encoding the protein of the present invention or its partial peptide can be detected in a human or mammal (e.g., rat, rabbit, sheep, swine, bovine, cat, dog, monkey, etc.). Therefore, the DNA of the present invention is useful as a gene diagnostic agent for detecting damage, mutation, decreased expression, increased expression, or overexpression of the DNA or mRNA.

The genetic diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

### <3> A method of screening a compound changing the expression amount of the protein of the present invention or a partial peptide thereof

The DNA of the present invention can be used as a probe for screening a compound changing the expression amount of the protein of the present invention or a partial peptide thereof.

Thus, the present invention provides a method of screening a compound changing the expression amount of the protein of the present invention or a partial peptide thereof, comprising measuring the amount of mRNA encoding the protein of the present invention or partial peptide thereof, which is contained in (i) (1) blood, (2) specific organ and (3) tissue or cells isolated from an organ, of non-human mammals, or (ii) a transformant and the like.

Measurement of the amount of mRNA of the protein of the present invention is carried out specifically as described below.
(i) To a normal or a disease-model non-human mammal (for example, mouse, rat, rabbit, sheep, pig, cow, cat, dog, monkey and the like; more specifically, dementia rat, obesity mouse, arterial sclerosis rabbit, gallbladder cancer mouse and the like), an agent (for example, anti-dementia agent, pressure drop agent, anti-cancer agent, anti-obesity agent and the like) or physical stress (for example, water immersion stress, electric shock, brightness and darkness, low temperature and the like) is imparted, and after a certain time, blood, specific organ (for example, brain, liver, kidney and the like), or tissue or cells isolated from an organ, and the like are harvested.
   The mRNA of the protein of the present invention contained in the obtained cell can be extracted from the cell and the like by a conventional method, and can be quantified using a method such as TaqManPCR, and can also be analyzed by northern blotting according to a known method.
(ii) A transformant expressing the protein of the present invention is produced according to the above-mentioned method, and mRNA of the protein of the present invention contained in the transformant can be quantified and analyzed in the same manner.

Screening of a compound changing the expression amount of the protein of the present invention can be carried out:
(i) by administering a test compound to a normal or disease-model non-human mammal at a certain time (30 minutes to 24 hours before, preferably, 30 minutes to 12 before, more preferably 1 hour to 6 hours before) before, or at a certain time (30 minutes to 3 days, preferably 1 hour to 2 days after, more preferably 1 hour to 24 hours) after imparting an agent or physical stress and the like, or simultaneously with an agent or physical stress; and at a certain time (30 minutes to 3 days, preferably 1 hour to 2 days, more preferably 1 hour to 24 hours) after the administration, quantifying and analyzing the amount of mRNA of the protein of the present invention contained in a cell; or
(ii) by adding a test compound to a medium in culturing a transformant according to a conventional method, and after culturing for a certain time (1 day to 7 days, preferably 1 day to 3 days, more preferably 2 days to 3 days), quantifying and analyzing the amount of mRNA of the protein of the present invention contained in the transformant.

The compound obtainable using the screening method of the present invention is capable of changing the expression amount of the protein of the present invention. Specifically, the compound includes (a) a compound that enhances the cell-stimulating activity mediated by the protein of the present invention (e.g., activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.) by increasing the expression amount of the protein of the present invention; and (b) a compound that reduces the cell-stimulating activity by decreasing the expression amount of the protein of the present invention.

The compound may be a peptide, protein, non-peptide compound, synthetic compound, and fermentation product, whether being novel or known.

The compound that enhances the cell-stimulating activity is useful as a safe and low-toxic pharmaceutical to enhance the physiological activity of the protein of the present invention.

The compound that reduces the cell-stimulating activity is useful as a safe and low-toxic pharmaceutical to reduce the physiological activity of the protein of the present invention.

When the compound obtainable by the screening method of the present invention is used as a pharmaceutical composition, such a composition can be prepared in a conventional manner. For example, the compound can be formulated into a tablet, capsule, elixir, microcapsule, aseptic solution, suspension, etc., as described for pharmaceuticals containing the protein of the present invention.

The composition thus obtained is safe and low-toxic, and can be administered to, for example, a human and mammal (e.g. rat, rabbit, sheep, swine, bovine, cat, dog, monkey, etc.).

The dose of the compound varies depending on subject to be administered, target organs, conditions, routes for administration, etc. In oral administration, e.g., for a patient with hyperlipidemia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for a patient with hyperlipidemia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### <4> A prophylactic and/or therapeutic agent for various diseases comprising a compound changing the expression amount of the protein of the present invention

The protein of the present invention is believed to perform some important role in an organism, for example, in metabolism, transportation and the like of intracellular lipid, as described above. Therefore, the compound changing the expression amount of the protein of the present invention can be used as a prophylactic and/or therapeutic agent for diseases associated with hyper function or dysfunction of the protein of the present invention. Particularly, the compound increasing the expression amount of the protein of the present invention can be used as a prophylactic and/or therapeutic agent for various diseases associated with lipid metabolism diseases, for example, diabetes mellitus, obesity, cancer, arterial sclerosis, hyperlipidemia, neuropathy and the like (particularly, arterial sclerosis, hyperlipidemia and the like).

When the compound is used as a pharmaceutical composition for diseases associated with hyper function or dysfunction of the protein of the present invention, such a composition can be prepared in a conventional manner.

For example, the compound can be used orally, for example, in the form of tablet which may be sugar-coated if necessary and desired, capsule, elixir, microcapsule etc., or parenterally in the form of injectable preparation such as a sterile solution and a suspension in water or with other pharmaceutically acceptable liquid. These preparations can be produced by mixing the compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is adjusted to such a dose that an appropriate dose is obtained within the specified range.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to a human or mammal (e.g., rat, rabbit, sheep, swine, bovine, cat, dog, monkey, etc.).

The dose of the compound varies depending on subject to be administered, organs to be administered, conditions, routes for administration, etc. In oral administration, e.g., for a patient with hyperlipidemia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for a patient with hyperlipidemia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### <5> A method of screening a compound changing the sterol-sensing ability of the protein of the present invention

A compound changing the sterol-sensing ability of the protein of the present invention (for example, peptide, protein, non-peptide compound, synthetic compound, fermentation product and the like) or salt thereof can be efficiently screened with a screening method using the protein of the present invention or the expression system of a recombinant protein.

Such a compound includes (a) a compound enhancing the cell stimulating activity mediated by the protein of the present invention (for example, activity to promote or inhibit arachidonic acid release, acetylcholine release, intracellular Ca²⁺ release, intracellular cAMP production, intracellular cGMP production, inositol phosphate production, cell membrane potential change, phosphorylation of intracellular proteins, activation of c-fos, decrease in pH, and the like), and (b) a compound reducing the cell stimulating activity, and the like.

Thus, the present invention provides a method of screening a compound changing the sterol-sensing ability of the protein of the present invention, comprising comparing a case (i) of contacting the protein of the present invention with a labeled sterol and a case (ii) of contacting the protein of the present invention with a labeled sterol and a test compound.

The screening method of the present invention is characterized in that the cell stimulating activity and the like of the protein and the like are measured in both the cases (i) and (ii), and the results are compared between the cases.

More specifically, the present invention provides:
(i) a method of screening a compound changing the sterol-sensing ability of the protein of the present invention, comprising measuring the cell stimulating activity mediated by the protein of the present invention (for example, activity to promote or inhibit arachidonic acid release, acetylcholine release, intracellular Ca²⁺ release, intracellular cAMP production, intracellular cGMP production, inositol phosphate production, cell membrane potential change, phosphorylation of intracellular proteins, activation of c-fos, decrease in pH, and the like) in a case of contacting a sterol with a cell containing the protein of the present invention and a case of contacting a sterol and a test compound with a cell containing the protein of the present invention, and comparing the results; and
(ii) a method of screening a compound changing the sterol-sensing ability of the protein of the present invention, comprising measuring the cell stimulating activity mediated by the protein of the present invention (for example, activity to promote or inhibit arachidonic acid release, acetylcholine release, intracellular Ca²⁺ release, intracellular cAMP production, intracellular cGMP production, inositol phosphate production, cell membrane potential change, phosphorylation of intracellular proteins, activation of c-fos, decrease in pH, and the like) in a case of contacting a sterol with the protein of the present invention expressed on cell membrane of a cultured transformant containing the DNA of the present invention and a case of contacting a sterol and a test compound with the protein of the present invention expressed on cell membrane of a cultured transformant containing the DNA of the present invention, and comparing the results.

The screening method of the present invention is described more specifically in the following.

At first, any materials which contain the protein of the present invention as described above can be used as the protein of the present invention for the screening method of the present invention, and preferred is a cell membrane fraction from a mammalian organ containing the protein of the present invention. However, because of extreme difficulty in obtaining a human organ in particular, it is preferable to use the human protein produced in a large amount using a recombinant for the screening.

To produce the protein of the present invention, the above-mentioned methods are used, and it is preferred to express the DNA of the present invention in mammalian and insect cells. For the DNA fragment encoding the target protein region, the complementary DNA, but not necessarily limited thereto, is employed. For example, the gene fragments and synthetic DNA may also be used. To introduce a DNA fragment encoding the protein of the present invention into host animal cells and efficiently express the DNA there, it is preferred to insert the DNA fragment downstream of a polyhedron promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRα promoter. The amount and quality of the expressed receptor are examined by a publicly known method, for example, the method described in the literature [Nambi, P. et al., The Journal of Biological Chemistry (J. Biol. Chem.), 267, 19555-19559, 1992].

Therefore, in the screening methods of the present invention, the material that contains the protein of the present invention may be the protein purified by a publicly known method, the cells containing the protein, or the cell membrane fraction containing the protein.

When the cells containing the protein of the present invention are used in the screening method of the present invention, the cells may be fixed with glutaraldehyde, formalin, etc. The fixation process is publicly known.

The cells containing the protein of the present invention are host cells expressing the protein. For the host cells, Escherichia coli, Bacillus subtilis, yeast, insect cells, animal cells and the like are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the protein expressed and membrane components such as cell-derived phospholipids and membrane proteins.

The amount of the protein in the cells containing the protein and in the membrane fraction is preferably 10³ to 10⁸ molecules per cell, more preferably 10⁵ to 10⁷ molecules per cell. As the amount of expression increases, the ligand binding activity per unit of membrane fraction (specific activity) increases so that not only the highly sensitive screening system can be constructed but also large quantities of samples can be assayed with the same lot.

To perform the screening method for a compound changing the sterol-sensing ability of the protein of the present invention, for example, an appropriate protein fraction is required.

The protein fraction is preferably a fraction of naturally occurring protein or a recombinant protein fraction having the same activity as that of the natural protein. Herein, the same activity is intended to mean a signal transduction activity or the like.

To perform the screening method for a compound changing the sterol-sensing ability of the protein of the present invention, the protein-mediated cell-stimulating activity (e.g., activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.) can be measured using a publicly known method or a commercially available kit.

Specifically, the cells containing the protein of the present invention are first cultured on a multi-well plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. When it is difficult to detect the production of the index substance (e.g., arachidonic acid) for the cell-stimulating activity due to a degrading enzyme contained in the cells, an inhibitor against such a degradation enzyme may be added prior to the assay. For detecting activities such as the cAMP production suppression activity, the baseline production in the cells is increased by forskolin or the like and the suppressing effect on the increased baseline production may then be detected.

The screening by assaying the cell-stimulating activity requires the cells expressing an appropriate protein. For the cells expressing the protein of the present invention, the cell line possessing the native protein of the present invention, the cell line expressing the recombinant protein described above, and the like are desirable.

For the test compound, for example, peptides, proteins, non-peptide compounds, synthetic compounds, fermentation products, cell extracts, plant extracts, and animal tissue extracts can be used. These compounds may be novel or known.

The screening kit for a compound changing the sterol-sensing ability of the protein of the present invention comprises the protein of the present invention, the cells containing the protein of the present invention, or the cell membrane fraction containing the protein of the present invention.

An example of the screening kit of the present invention is as follows:
1. Reagents for the screening
   (i) Buffer for measurement and washing Hanks' balanced salt solution (Gibco Co.) supplemented with 0.05% bovine serum albumin (Sigma Co.).
      The solution is sterilized by filtration through a 0.45 µm filter, and stored at 4°C or may be prepared at use.
   (ii) Standard protein of the present invention CHO cells expressing the protein of the present invention are sub-cultured in a 12-well plate at a density of 5 × 10⁵ cells/well followed by culturing at 37°C under 5% CO₂ and 95% air for 2 days.

The compound obtainable using the screening method or the screening kit of the present invention is capable of changing the sterol-sensing ability of the protein of the present invention. Specifically, the compound includes (a) a compound that has the cell-stimulating activity mediated by the protein of the present invention (e.g., activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.); and (b) a compound that reduces the cell-stimulating activity.

The compound may be a peptide, protein, non-peptide compound, synthetic compound, and fermentation product, whether being novel or known.

The compound that enhances the sterol-sensing ability of the protein of the present invention is capable of enhancing the physiological activity of the protein of the present invention, and thus is useful as a safe and low-toxic pharmaceutical to enhance the activity of the protein of the present invention.

The compound that reduces the sterol-sensing ability of the protein of the present invention is capable of inhibiting the physiological activity of the protein of the present invention, and thus is useful as a safe and low-toxic pharmaceutical to inhibit the activity of the protein of the present invention.

When the compound obtainable by the screening method or the screening kit of the present invention is used as a pharmaceutical composition, such a composition can be prepared in a conventional manner. For example, the compound can be formulated into a tablet, capsule, elixir, microcapsule, aseptic solution, suspension, etc., as described for pharmaceuticals containing the protein of the present invention.

The composition thus obtained is safe and low-toxic, and can be administered to, for example, a human and mammal (e.g. rat, rabbit, sheep, swine, bovine, cat, dog, monkey, etc.).

The dose of the compound varies depending on subject to be administered, target organs, conditions, routes for administration, etc. In oral administration, e.g., for a patient with hyperlipidemia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for a patient with hyperlipidemia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### <6> A prophylactic and/or therapeutic agent for various diseases comprising a compound changing the sterol-sensing ability of the protein of the present invention

The protein of the present invention is believed to perform some important role in an organism, for example, in metabolism, transportation and the like of intracellular lipid, as described above. Therefore, the compound changing the sterol-sensing ability of the protein of the present invention can be used as a prophylactic and/or therapeutic agent for diseases associated with hyper function or dysfunction of the protein of the present invention.

When the compound is used as a pharmaceutical composition for diseases associated with dysfunction of the protein of the present invention, such a composition can be prepared in a conventional manner.

For example, the compound can be used orally, for example, in the form of tablet which may be sugar-coated if necessary and desired, capsule, elixir, microcapsule etc., or parenterally in the form of injectable preparation such as a sterile solution and a suspension in water or with other pharmaceutically acceptable liquid. These preparations can be produced by mixing the compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is adjusted to such a dose that an appropriate dose is obtained within the specified range.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to a human or mammal (e.g., rat, rabbit, sheep, swine, bovine, cat, dog, monkey, etc.).

The dose of the compound varies depending on subject to be administered, organs to be administered, conditions, routes for administration, etc. In oral administration, e.g., for a patient with hyperlipidemia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for a patient with hyperlipidemia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### <7> Quantification of the protein of the present invention

The antibodies of the present invention are capable of specifically recognizing the protein of the present invention. Therefore, the antibodies can be used to quantify the protein of the present invention in a test fluid, especially for quantification by the sandwich immunoassay. Thus, the present invention provides, for example, the following quantification methods:
(i) a method of quantifying the protein of the present invention in a test fluid, which comprises competitively reacting the antibody of the present invention with the test fluid and the labeled protein, and measuring the ratio of the labeled protein bound to the antibody; and
(ii) a method of quantifying the protein of the present invention in a test fluid, which comprises reacting the test fluid with the antibody of the present invention immobilized on a carrier and the labeled antibody of the present invention simultaneously or sequentially, and measuring the activity of the label on the immobilized carrier.

In (ii) described above, it is preferred that one antibody recognizes the N-terminal region of the protein of the present invention, and another antibody reacts with the C-terminal region of the protein of the present invention.

Using monoclonal antibodies to the protein of the present invention (hereinafter sometimes referred to as the monoclonal antibodies of the present invention), the protein of the present invention can be assayed and also detected by tissue staining or the like. For this purpose, an antibody molecule itself may be used, or F(ab')₂, Fab' or Fab fractions of the antibody molecule may also be used. Assay methods using antibodies to the protein of the present invention are not particularly limited. Any assay method can be used, so long as the amount of antibody, antigen, or antibody-antigen complex corresponding to the amount of antigen (e.g., the amount of the protein) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H] and [¹⁴C] are used. As the enzyme described above, stable enzymes with high specific activity are preferred; for example, β-galactosidase, β-glucosidase, alkaline phosphatase, peroxidase, malate dehydrogenase and the like are used. Example of the fluorescent substance used are fluorescamine and 5 fluorescein isothiocyanate are used. For the luminescent substance, for example, luminol, luminol derivatives, luciferin, and lucigenin. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like. are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above.

In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein are preferably used as the 30 monoclonal antibodies of the present invention for the primary and secondary reactions. That is, in the antibodies used for the primary and secondary reactions are, for example, when the antibody used in the secondary reaction recognizes the C-terminal region of the protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts. [For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention or its salts can be quantified with high sensitivity, using the antibodies of the present invention.

By quantifying the protein of the present invention or its salts in vivo using the antibodies of the present invention, diagnosis can be made on various diseases associated with dysfunction of the protein of the present invention.

The antibodies of the present invention can also be used for specifically detecting the protein of the present invention present in test samples such as body fluids or tissues. The antibodies may also be used for preparation of antibody columns for purification of the protein of the present invention, for detection of the protein of the present invention in each fraction upon purification, and for analysis of the behavior of the protein of the present invention in the test cells.

### <8> Neutralization by an antibody to the protein of the present invention

The neutralizing activity of antibodies to the protein of the present invention refers to an activity of inactivating the signal transduction function involving the protein. Therefore, when the antibody has the neutralizing activity, the antibody can inactivate the signal transduction in which the protein participates, for example, inactivate the protein-mediated cell-stimulating activity (e.g., activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.). Therefore, the antibody can be used for the prevention and/or treatment of diseases caused by overexpression of the protein.

### <9> Preparation of an animal carrying the DNA encoding the protein of the present invention

Using the DNA of the present invention, transgenic animals expressing the protein of the present invention can be prepared. Examples of the animals include mammals (e.g., rats, mice, rabbits, sheep, swine, bovine, cats, dogs, monkeys, etc.) (hereinafter merely referred to as animals) can be used, with mice and rabbits being particularly appropriate.

To transfer the DNA of the present invention to target animals, it is generally advantageous to use the DNA in a gene construct ligated downstream of a promoter that can express the DNA in animal cells. For example, when the DNA of the present invention derived from rabbit is transferred, e.g., the gene construct, in which the DNA is ligated downstream of a promoter that can expresses the DNA of the present invention derived from animals containing the DNA of the present invention highly homologous to the rabbit-derived DNA, is microinjected to rabbit fertilized ova; thus, the DNA-transferred animal, which is capable of producing a high level of the protein of the present invention, can be produced. Examples of the promoters that are usable include virus-derived promoters and ubiquitous expression promoters such as metallothionein promoter, but promoters of NGF gene and enolase that are specifically expressed in the brain are preferably used.

The transfer of the DNA of the present invention at the fertilized egg cell stage secures the presence of the DNA in all germ and somatic cells in the produced animal. The presence of the protein of the present invention in the germ cells in the DNA-transferred animal means that all germ and somatic cells contain the protein of the present invention in all progenies of the animal. The progenies of the animal that took over the gene contain the protein of the present invention in all germ and somatic cells.

The DNA-transferred animals of the present invention can be maintained and bled in the conventional environment as animals carrying the DNA after confirming the stable retention of the gene in the animals through mating. Furthermore, mating male and female animals containing the objective DNA results in acquiring homozygote animals having the transferred gene on both homologous chromosomes. By mating the male and female homozygotes, bleeding can be performed so that all progenies contain the DNA.

Since the protein of the present invention is highly expressed in the animals in which the DNA of the present invention has been transferred, the animals are useful for screening of a compound changing the sterol-sensing ability of the protein of the present invention.

The animals in which the DNA of the present invention has been transferred can also be used as cell sources for tissue culture. The protein of the present invention can be analyzed by, for example, directly analyzing the DNA or RNA in tissues from the mouse in which the DNA of the present invention has been transferred, or by analyzing tissues containing the protein expressed from the gene. Cells from tissues containing the protein of the present invention are cultured by the standard tissue culture technique. Using these cells, for example, the function of tissue cells such as cells derived from the brain or peripheral tissues, which are generally difficult to culture, can be studied. Using these cells, for example, it is possible to select pharmaceuticals that increase various tissue functions. When a highly expressing cell line is available, the protein of the present invention can be isolated and purified from the cell line.

### <10> A compound having an action on a SSD-containing protein to manifest the regulation of intracellular cholesterol transport

Further, a compound having an action on an SSD-containing protein, such as the protein of the present invention, to manifest the regulation of intracellular cholesterol transport can regulate the synthesis and absorption of cholesterol, or secretion of cholesterol in the form of lipoprotein, uptake into cells, accumulation, storage, or release of cholesterol. Therefore, a pharmaceutical composition comprising said compound can be used as a prophylactic and/or therapeutic agent for various diseases associated with diseases of lipid metabolism, for example, diabetic mellitus, obesity, cancer, arterial sclerosis, hyperlipidemia, or neuropathy (particularly, arterial sclerosis, hyperlipidemia).

In the specification and drawings, abbreviations of bases and amino acids are based on the abbreviations of the IUPAC-IUB Commission on Biochemical Nomenclature or the conventional abbreviations used in the art, examples of which are shown below. An amino acid that has an optical isomer takes its L form unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid
- Me: : methyl
- Et: : ethyl
- Bu: : butyl
- Ph: : phenyl
- TC: : thiazolidine-4(R)-carboxamide

The substituents, protective groups and reagents, which are frequently used throughout the specification, are shown by the following abbreviations.
- Tos: : p-toluenesulfonyl
- CHO: : formyl
- Bzl: : benzyl
- Cl₂Bl: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyloxycarbonyl
- Boc: : t-butoxycarbonyl
- DNP: : dinitrophenol
- Trt: : trityl
- Bum: : t-butoxymethyl
- Fmoc: :N-9-fluorenylmethoxycarbonyl
- HOBt: : 1-hydroxybenztriazole
- HOOBt: : 3,4-dihydro-3-hydroxy-4-oxo-1,2,3-benzotriazine
- HONB: : 1-hydroxy-5-norbornene-2,3-dicarboximide
- DCC: : N,N'-dicyclohexylcarbodiimide

Each SEQ ID NO (sequence identification number) in the Sequence Listing of the specification indicates the following sequence, respectively.

### [SEQ ID NO: 1]

This shows the nucleic acid sequence of a primer used for cloning cDNA encoding the human SSP1 protein of the present invention.

### [SEQ ID NO: 2]

This shows the nucleic acid sequence of a primer used for cloning cDNA encoding the human SSP1 protein of the present invention.

### [SEQ ID NO: 3]

This shows the nucleic acid sequence of a primer used for cloning cDNA encoding the human SSP1 protein of the present invention.

### [SEQ ID NO: 4]

This shows the nucleic acid sequence of a primer used for cloning cDNA encoding the human SSP1 protein of the present invention.

### [SEQ ID NO: 5]

This shows the nucleic acid sequence of a primer used for cloning cDNA encoding the human SSP1 protein of the present invention.

### [SEQ ID NO: 6]

This shows the nucleic acid sequence of a primer used for cloning cDNA encoding the human SSP1 protein of the present invention.

### [SEQ ID NO: 7]

This shows the nucleic acid sequence of cDNA encoding the human SSP1 protein of the present invention having the amino acid sequence of SEQ ID NO: 9.

### [SEQ ID NO: 8]

This shows the amino acid sequence of SSD present in the human SSP1 protein of the present invention.

### [SEQ ID NO: 9]

This shows the amino acid sequence of the human liver-derived SSP 1 protein of the present invention.

### [SEQ ID NO: 10]

This shows the nucleic acid sequence of a primer used for cloning cDNA encoding the human SSP2 protein of the present invention.

### [SEQ ID NO: 11]

This shows the nucleic acid sequence of a primer used for cloning cDNA encoding the human SSP2 protein of the present invention.

### [SEQ ID NO: 12]

This shows the nucleic acid sequence of a primer used for cloning cDNA encoding the human SSP2 protein of the present invention.

### [SEQ ID NO: 13]

This shows the nucleic acid sequence of a primer used for cloning cDNA encoding the human SSP2 protein of the present invention.

### [SEQ ID NO: 14]

This shows the nucleic acid sequence of a primer used for cloning cDNA encoding the human SSP2 protein of the present invention.

### [SEQ ID NO: 15]

This shows the nucleic acid sequence of cDNA encoding the human testis-derived SSP2 protein of the present invention having the amino acid sequence of SEQ ID NO: 17.

### [SEQ ID NO: 16]

This shows the amino acid sequence of SSD present in the human SSP2 protein of the present invention.

### [SEQ ID NO: 17]

This shows the amino acid sequence of the human testis-derived SSP2 protein of the present invention.

### [SEQ ID NO: 18]

This shows the nucleic acid sequence of a primer used for preparing a probe for Norther analysis of the human SSP1 of the present invention.

### [SEQ ID NO: 19]

This shows the nucleic acid sequence of a primer used for preparing a probe for Norther analysis of the human SSP1 of the present invention.

### [SEQ ID NO: 20]

This shows the nucleic acid sequence of a primer used for preparing a probe for Norther analysis of the human SSP1 of the present invention.

### [SEQ ID NO: 21]

This shows the nucleic acid sequence of a primer used for preparing a probe for Norther analysis of the human SSP1 of the present invention.

### [SEQ ID NO: 22]

This shows the nucleic acid sequence of a primer used for cloning cDNA encoding the human SSP1 protein of the present invention.

### [SEQ ID NO: 23]

This shows the nucleic acid sequence of a primer used for cloning cDNA encoding the human SSP2 protein of the present invention.

### [SEQ ID NO: 24]

This shows the nucleic acid sequence of a primer used for cloning cDNA encoding the human SSP2 protein of the present invention.

### [SEQ ID NO: 25]

This shows the nucleic acid sequence of a primer used for cloning cDNA encoding the human SSP2 protein of the present invention.

### [SEQ ID NO: 26]

This shows the nucleic acid sequence of a primer used for cloning cDNA encoding the human SSP2 protein of the present invention.

### [SEQ ID NO: 27]

This shows the nucleic acid sequence of a primer used for cloning cDNA encoding the human SSP2 protein of the present invention.

### [SEQ ID NO: 28]

This shows the nucleic acid sequence of cDNA encoding SSD which is present in the human SSP1 protein of the present invention and has the amino acid sequence of SEQ ID NO: 8.

### [SEQ ID NO: 29]

This shows the nucleic acid sequence of cDNA encoding SSD which is present in the human SSP2 protein of the present invention and has the amino acid sequence of SEQ ID NO: 16.

### [SEQ ID NO: 30]

This shows the nucleic acid sequence of a primer used for cloning cDNA encoding the human SSP2 protein of the present invention.

### [SEQ ID NO: 31]

This shows the nucleic acid sequence of a primer used for cloning cDNA encoding the human SSP2 protein of the present invention.

### [SEQ ID NO: 32]

This shows the nucleic acid sequence of cDNA encoding the human testis-derived SSP2-V1 protein of the present invention having the amino acid sequence of SEQ ID NO: 34.

### [SEQ ID NO: 33]

This shows the nucleic acid sequence of cDNA encoding the human testis-derived SSP2-V2 protein of the present invention having the amino acid sequence of SEQ ID NO: 35.

### [SEQ ID NO: 34]

This shows the amino acid sequence of the human testis-derived SSP2-V1 protein of the present invention.

### [SEQ ID NO: 35]

This shows the amino acid sequence of the human testis-derived SSP2-V2 protein of the present invention.

### [SEQ ID NO:36]

This shows the nucleic acid sequence of a primer used for cloning cDNA encoding the human SSP2 protein of the present invention.

### [SEQ ID NO:37]

This shows the nucleic acid sequence of a primer used for cloning cDNA encoding the human SSP2 protein of the present invention.

### [SEQ ID NO: 38]

This shows the amino acid sequence which is inserted into the amino acid sequence described in Genomics, 65, 137-145, 2000 and is not contained in the amino acid sequence of SEQ ID NO: 9.

### [SEQ ID NO: 39]

This shows the nucleic acid sequence of cDNA encoding the amino acid sequence of SEQ ID NO: 38.

### [SEQ ID NO: 40]

This shows the amino acid sequence of the human brain-derived SSP2 protein of the present invention.

### [SEQ ID NO: 41]

This shows the nucleic acid sequence of cDNA encoding the human brain-derived SSP2 protein of the present invention having the amino acid sequence of SEQ ID NO: 40.

### [SEQ ID NO: 42]

This shows the nucleic acid sequence of KIAAI377 (DNA Res., 7, 65-73 (2000)).

### [SEQ ID NO: 43]

This shows the nucleic acid sequence of a primer used for cloning cDNA encoding the human brain-derived SSP2 protein of the present invention.

### [SEQ ID NO: 44]

This shows the nucleic acid sequence of a primer used for cloning cDNA encoding the human brain-derived SSP2 protein of the present invention.

### [SEQ ID NO: 45]

This shows the nucleic acid sequence of a primer used for cloning cDNA encoding the human brain-derived SSP2 protein of the present invention.

### [SEQ ID NO: 46]

This shows the nucleic acid sequence of a primer used for cloning cDNA encoding the human brain-derived SSP2 protein of the present invention.

### [SEQ ID NO: 47]

This shows the nucleic acid sequence of a region amplified by PCR for cloning cDNA encoding the human brain-derived SSP2 protein of the present invention.

### [SEQ ID NO: 48]

This shows the nucleic acid sequence of a region amplified by PCR for cloning cDNA encoding the human brain-derived SSP2 protein of the present invention.

### [SEQ ID NO: 49]

This shows the nucleic acid sequence of a unknown region which is present in the cDNA encoding the human brain-derived SSP2 protein of the present invention.

### [SEQ ID NO: 50]

This shows the nucleic acid sequence of a region amplified by PCR for cloning cDNA encoding the human brain-derived SSP2 protein of the present invention.

### [SEQ ID NO: 51]

This shows the nucleic acid sequence of a region amplified by PCR for cloning cDNA encoding the human brain-derived SSP2 protein of the present invention.

### [SEQ ID NO: 52]

This shows the nucleic acid sequence of a region amplified by PCR for cloning cDNA encoding the human brain-derived SSP2 protein of the present invention.

### [SEQ ID NO: 53]

This shows the nucleic acid sequence of a region amplified by PCR for cloning cDNA encoding the human brain-derived SSP2 protein of the present invention.

### [SEQ ID NO: 54]

This shows the nucleic acid sequence of a probe used for analysis of expression of the human SSP1 gene of the present invention.

### [SEQ ID NO: 55]

This shows the nucleic acid sequence of a probe used for analysis of expression of the human SSP1 gene of the present invention.

### [SEQ ID NO: 56]

This shows the nucleic acid sequence of a probe used for analysis of expression of the human SSP1 gene of the present invention.

### [SEQ ID NO: 57]

This shows the nucleic acid sequence of a probe used for analysis of expression of the human SSP2 gene of the present invention.

### [SEQ ID NO: 58]

This shows the nucleic acid sequence of a probe used for analysis of expression of the human SSP2 gene of the present invention.

### [SEQ ID NO: 59]

This shows the nucleic acid sequence of a probe used for analysis of expression of the human SSP2 gene of the present invention.

A transformant *Escherichia coli* DH5α/pTB2080 obtained in Example 1 is on deposit with the Ministry of International Trade and Industry, Agency of Industrial Science and Technology, National Institute of Bioscience and Human Technology (NIBH; 1-1-3, Higashi, Tsukuba-shi, Ibaraki, Japan) as the Accession Number FERM BP-7015 since February 2, 2000 and with Institute for Fermentation (IFO; 2-17-85, Juso Honcho, Yodogawa-ku, Osaka-shi, Osaka, Japan) as the Accession Number IFO 16351 since January 13, 2000.

A transformant *Escherichia coli* DH5α/pTB2081 obtained in Example 2 was on deposit with the Ministry of International Trade and Industry, Agency of Industrial Science and Technology, National Institute of Bioscience and Human Technology (NIBH; 1-1-3, Higashi, Tsukuba-shi, Ibaraki, Japan) as the Accession Number FERM BP-7016 since February 2, 2000 and with Institute for Fermentation (IFO; 2-17-85, Juso Honcho, Yodogawa-ku, Osaka-shi, Osaka, Japan) as the Accession Number IFO 16352 since January 13, 2000.

A transformant *Escherichia coli* DH5α/pTB2221 obtained in Example 5 is on deposit with the Ministry of International Trade and Industry, Agency of Industrial Science and Technology, National Institute of Bioscience and Human Technology (NIBH; 1-1-3, Higashi, Tsukuba-shi, Ibaraki, Japan) as the Accession Number FERM BP-7502 since March 14, 2001 and with Institute for Fermentation (IFO; 2-17-85, Juso Honcho, Yodogawa-ku, Osaka-shi, Osaka, Japan) as the Accession Number IFO 16580 since March 8, 2001.

### EXAMPLES

The present invention is described in more detail with reference to the following examples, being not intended to limit the scope of the present invention thereto. The genetic procedures using Escherichia coli were performed according to methods described in the "Molecular Cloning".

### Example 1: Cloning of cDNA encoding the human liver-derived SSP1 protein

The cDNA encoding human SSP1 protein was cloned by conducting PCR using a human liver-derived cDNA library according to the following procedure. PCR was carried out using the oligo DNA shown by SEQ ID NO: 1 as a sense chain primer and the oligo DNA shown by SEQ ID NO: 2 as an anti-sense chain primer, and using human live-derived Gene Pool cDNA (Invitrogen) and cDNA obtained by RT-PCR using a random primer from mRNA purified from HepG2 cell to obtain a 5'-upstream sequence containing each primer as a starting point. In the same way, PCR was carried out using the oligo DNAs shown by SEQ ID NO: 3 and SEQ ID NO: 4 as a sense chain primer and an anti-sense primer, respectively, to obtain a sequence containing each primer as a starting point, downstream of the above sequence. Further, PCR was carried out using the oligo DNAs shown by SEQ ID NO: 5 and SEQ ID NO: 6 as a sense chain primer and an anti-sense primer, respectively, to obtain a 3'-downstream sequence containing each primer as a starting point. The nucleic acid sequences of 3 double-stranded DNAs thus obtained were determined to find the presence of a common sequence over-lapping. This teaches that these sequences are derived from the same gene. These three cDNA fragments obtained by PCR were ligated after treatment with a restriction enzyme in the common sequence portion to finally obtain a cDNA fragment having a full length of 3,999 base pairs (bp) as ORF shown by SEQ ID NO: 7. This cDNA fragment encodes a novel human SSP1 protein composed of 1332 amino acids shown by SEQ ID NO: 9, which contains the SSD sequence of 184 amino acid residues shown by SEQ ID NO: 7. A nucleic acid sequence encoding the SSD sequence shown by SEQ ID NO: 8 is shown by SEQ ID NO: 28. This human SSP1 protein has the highest homology with the human NPC 1 protein, and has 8 cysteine residues as located in the N terminal NPC Domain. The homology between them is 42.0% in terms of amino acid sequence.

The cDNA (SEQ ID NO: 7) encoding the human liver SSP1 protein of the present invention was introduced into pUC 118 (Takara Shuzo), and the resulting plasmid pTB2080 was introduced into Escherichia coli DH5α according to a known method to obtain a transformant: Escherichia coli DH5α/pTB2080.

### Example 2: Cloning of cDNA encoding the human testis-derived SSP2 protein

The cDNA encoding human SSP2 protein was cloned by conducting PCR using a human testis-derived cDNA library according to the following procedure. 5'-RACE was carried out using the oligo DNA shown by SEQ ID NO: 10 as an anti-sense chain primer, and human testis-derived Marathon-Ready cDNA (CLONTECH) to obtain a 5'-upstream sequence containing each primer as a starting point. In the same manner as the case of cloning cDNA encoding the human SSP1 protein, PCR was carried out using the oligo DNAs shown by SEQ ID NO: 11 and SEQ ID NO: 12 as a sense chain primer and an anti-sense primer, respectively, to obtain a sequence containing each primer as a starting point, downstream of the above sequence. Further, PCR was carried out using the oligo DNAs shown by SEQ ID NO: 13 and SEQ ID NO: 14 as a sense chain primer and an anti-sense primer, respectively, to obtain a 3'-downstream sequence containing each primer as a starting point. The nucleic acid sequences thus obtained of three double-stranded DNAs were determined to find the presence of a common sequence over-lapping. This teaches that these sequences are derived from the same gene. The cDNA fragment resulting from connection in the common sequence portion has ORF as cDNA having a full length of 3,264 base pairs (bp) shown by SEQ ID NO: 15. The cDNA fragment encodes a novel human SSP2 protein composed of 1,087 amino acids shown by SEQ ID NO: 17, containing the SSD sequence of 200 amino acid residues shown by SEQ ID NO: 16. A nucleic acid sequence encoding the SSD sequence shown by SEQ ID NO: 16 is shown by SEQ ID NO: 29.

For obtaining cDNA (SEQ ID NO: 15) encoding the human testis-derived SSP2 protein of the present invention, PCR was further carried out for the sequence region using human testis-derived Marathon-Ready cDNA (CLONTECH). PCR was carried out using the oligo DNAs shown by SEQ ID NO: 30 and SEQ ID NO: 31 as a sense chain primer and an anti-sense primer, respectively, to obtain a sequence of 5'-upstream containing each primer as a starting point. In this procedure, cDNA fragments were obtained having a nucleic acid sequence, other than the intended sequence, shown by SEQ ID NO: 32 and SEQ ID NO: 33 as ORF, believed to be an alternative splicing variant. All the fragments contain a part encoding the SSD region shown by SEQ ID NO: 16, but they encode an immature SSP2 protein due to insertion of a stop codon into the ORF sequence by frame shift caused by insertion and deletion of bases. The cDNA shown by SEQ ID NO: 32 encodes SSP2-V1 composed of 456 amino acids shown by SEQ ID NO: 34, and the cDNA shown by SEQ ID NO: 33 encodes SSP2-V2 composed of 445 amino acids shown by SEQ ID NO: 35.

Further, PCR was carried out using the oligo DNAs shown by SEQ ID NO: 36 and SEQ ID NO: 37 as a sense chain primer and an anti-sense primer, respectively, to obtain a 3'-downstream sequence containing each primer as a starting point. These cDNA fragments obtained by PCR were ligated by treatment with a restriction enzyme in the common sequence portion, and the resulting cDNA fragment shown by SEQ ID NO: 15 was introduced into pT7 (Novagene) to obtain a plasmid pTB2081. The plasmid pTB2081 was introduced into Escherichia coli DH5α according to a known method to obtain a transformant: Escherichia coli DH5α/pTB2081.

### Example 3: Analysis of SSP1 expression in human tissues and its tissue specificity

A probe used for northern analysis was prepared by effecting PCR according to the following procedure using a human fetus-derived cDNA library. PCR was carried out using the oligo DNA shown by SEQ ID NO: 18 as a sense chain primer and the oligo DNA shown by SEQ ID NO: 19 as an anti-sense chain primer, and using human fetus-derived Marathon Ready cDNA (CLONTECH). Then, using the resulting amplified product, PCR was carried out with the oligo DNA shown by SEQ ID NO: 20 as a sense chain primer and the oligo DNA shown by SEQ ID NO: 21 as an anti-sense chain primer to obtain the cDNA shown by SEQ ID NO: 22.

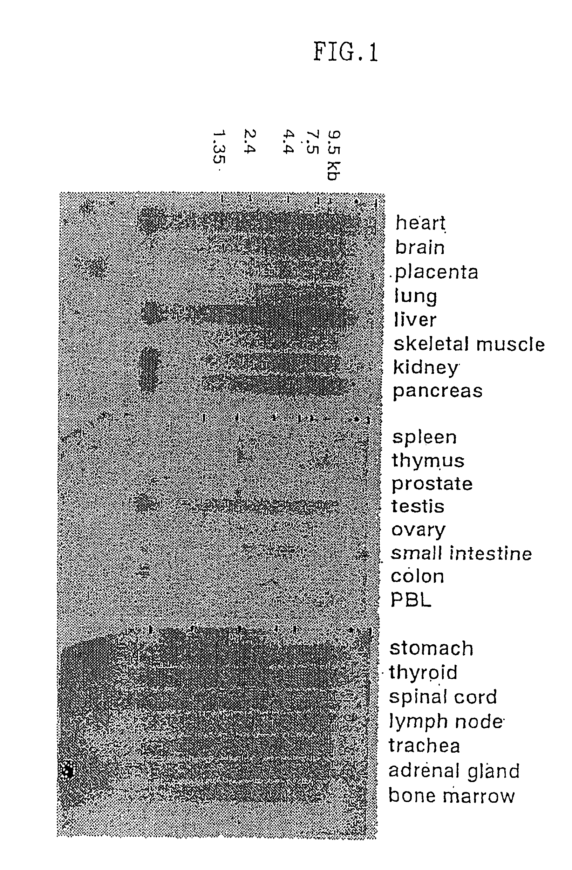

The tissue specificity of SSP1mRNA expression was analyzed (Fig. 1) by using the cDNA shown by SEQ ID NO: 22 as a probe and hybridizing it to Multiple Tissue Northern Blots (CLONTECH), Multiple Tissue Northern Blots II (CLONTECH), and Multiple Tissue Northern Blots III (CLONTECH). As shown in Fig. 1, SSP1 was expressed specifically in liver.

### Example 4: Analysis of SSP2 expression in human tissues and its tissue specificity

A probe used for northern analysis was carried out by effecting PCR according to the following procedure using a human fetus-derived cDNA library. PCR was carried out using the oligo DNA shown by SEQ ID NO: 23 as a sense chain primer and the oligo DNA shown by SEQ ID NO: 24 as an anti-sense chain primer, and using human fetus-derived Marathon Ready cDNA (CLONTECH). Then, using the resulting amplified product, PCR was carried out with the oligo DNA shown by SEQ ID NO: 25 as a sense chain primer and the oligo DNA shown by SEQ ID NO: 26 as an anti-sense chain primer to obtain the cDNA shown by SEQ ID NO: 27.

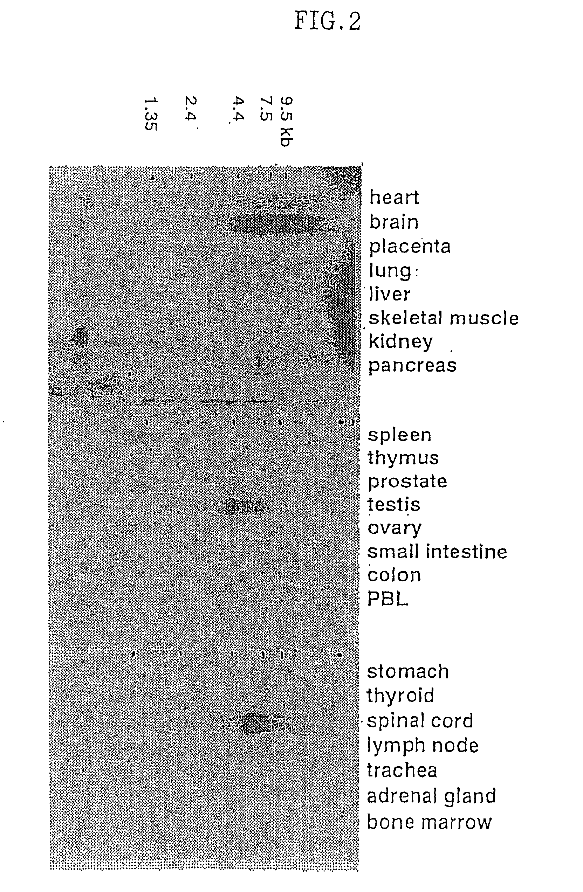

The tissue specificity of SSP2mRNA expression was analyzed (Fig. 2) by using the cDNA shown by SEQ ID NO: 27 as a probe and hybridizing it to Multiple Tissue Northern Blots (CLONTECH), Multiple Tissue Northern Blots II (CLONTECH), and Multiple Tissue Northern Blots III (CLONTECH). As shown in Fig. 2, SSP2 was expressed as mRNA of about 6 kb in brain and spinal cord and expressed as mRNA of about 4 kb in testis.

### Example 5: Cloning of cDNA encoding the human brain-derived SSP2 protein

The mRNA of SSP2 gene expressed in brain and spinal cord had a length of about 6 kbp, longer than the mRNA expressed in testis, and thus a transcription from further upstream was possible. The sequence of a human brain-derived EST clone reported as KIAA1377 (DNA Res., 7, 65-73 (2000)) contained the sequence of the human testis-derived SSP2 obtained above, and further contained an upstream region of about 1 kbp (SEQ ID NO: 42). However, this KIAA1377 did not specify the 5'-terminal well. Thus, the structure of the 5'-terminal of mRNA of SSP2 gene expressed in human brain was determined.
More specifically, a reverse transcription reaction from human brain poly(A) + RNA was performed using a mixture of the primer shown by SEQ ID NO: 43 and SMART III oligo shown by SEQ ID NO: 44 (Clontech) to make conversion of the elongated chain. Using the product as a template, PCR was carried out with the 5'-phosphorylated primers shown by SEQ ID NO: 45 and SEQ ID NO: 46 to amplify the 5'-terminal region of SSP2. After the amplification product was purified, a ligation was carried out to cause cyclization and concatemerization of the product, and using it as a template, a region between the nucleic acid sequence shown by SEQ ID NO: 47 and the sequence shown by SEQ ID NO: 48 was amplified to obtain an amplified fragment containing the unknown 5'-terminal region. The sequence of the unknown region is shown by SEQ ID NO: 49. Since this fragment also contains a stop codon downstream of the unknown region in a translation frame of SSP2 gene, it was clear that the SSP2 gene shown by SEQ ID NO: 41 encoded a protein having the amino acid sequence shown by SEQ ID NO: 40. From the information, a region between the nucleic acid sequence shown by SEQ ID NO: 50 and the nucleic acid sequence shown by SEQ ID NO: 51 was obtained by PCR. A region between the nucleic acid sequence shown by SEQ ID NO: 52 and the nucleic acid sequence shown by SEQ ID NO: 53 was obtained by PCR from SSP2 gene expressed in testis, shown by SEQ ID NO: 15. The two above-mentioned PCR products were cut with a restriction enzyme and ligated, and using it as a template, PCR was carried out with the primers shown by SEQ ID NO: 50 and SEQ ID NO: 53 to obtain a gene containing the entire length of SSP2 gene expressed in brain. The entire length of gene was sub-cloned to pcDNA3.1 (+) to obtain a plasmid pTB2221. The plasmid pTB2221 was introduced into Escherichia coli DH5α according to a known method to obtain a transformant: Escherichia coli DH5α/pTB2221.

### Example 6: Setting of an analysis system of the expression using TaqMan PCR

For conducting more detailed analysis of SSP 1 expression, TaqMan PCR conditions were set. The primers used were Upper Primer (SEQ ID NO: 54) and Lower Primer (SEQ ID NO: 55), and the probe used for detection was shown by SEQ ID NO: 56. In TaqMan PCR, 250 nM of Upper Primer, 250 nM of Lower Primer, 75 nM of the probe and template DNA were mixed with TaqMan Universal Mixture, and SSP1 gene was quantified by real time PCR of 40 cycle of 15 seconds at 95°C and 60 seconds at 60°C. A typical calibration curve pattern is shown in Fig. 3.

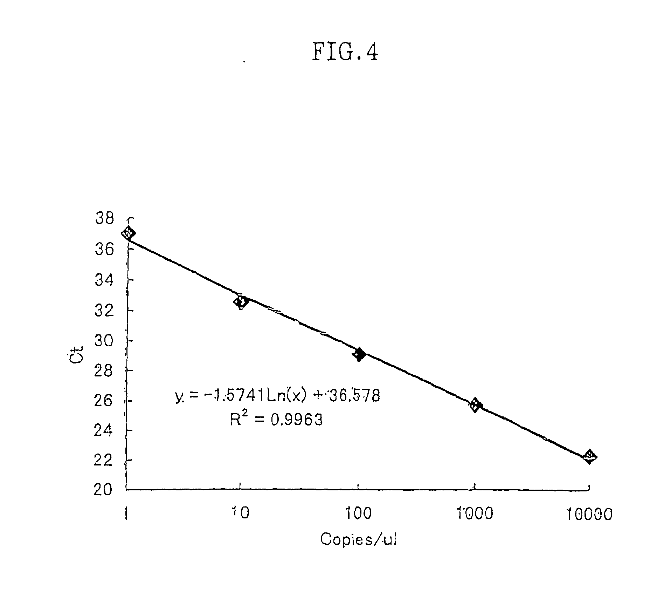

For conducting more detailed analysis of SSP2 expression, TaqMan PCR conditions were set. The primers used were Upper Primer (SEQ ID NO: 57) and Lower Primer (SEQ ID NO: 58), and the probe used for detection was shown by SEQ ID NO: 59. In TaqMan PCR, 250 nM of Upper Primer, 250 nM of Lower Primer, 75 nM of the probe and template DNA were mixed with TaqMan Universal Mixture, and SSP2 gene was quantified by real time PCR of 40 cycle of 15 seconds at 95°C and 60 seconds at 60°C. A typical calibration curve pattern is shown in Fig. 4.

### Example 7: Analysis of tissue specificity of SSP1 gene expression by TaqMan PCR method

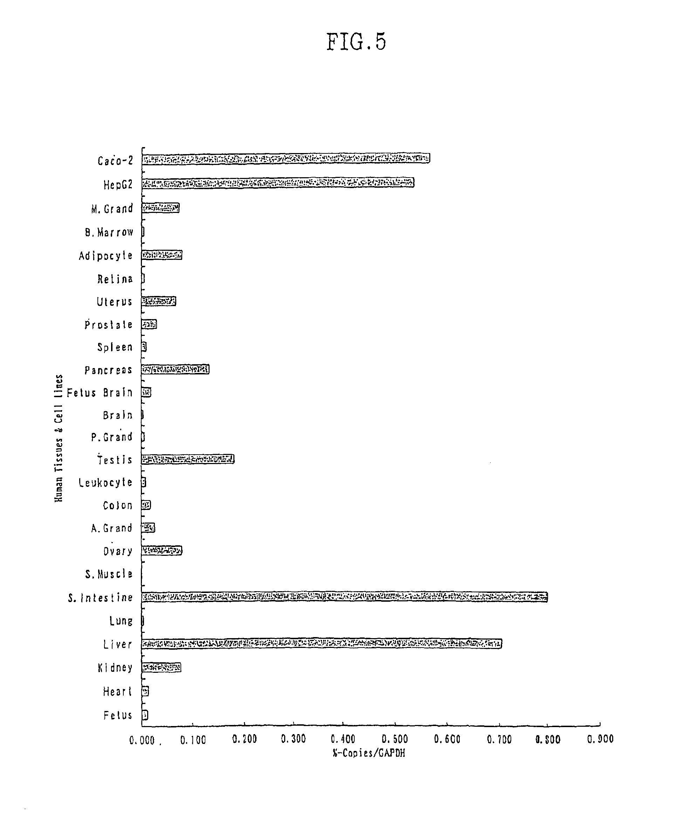

Using the quantification system of SSP1 gene expression by TaqMan PCR, made in Example 6, the expression amount of SSP1 mRNA was quantified on the basis of various Marathon Ready cDNAs and cDNAs obtained by a reverse transcription using oligo-dT as a primer from RNAs extracted from HepG2 cell and Caco-2 cell. The results are shown in Fig. 5.

### Example 8: Analysis of the site-specific expression of SSP1 mRNA in small intestine by northern blotting

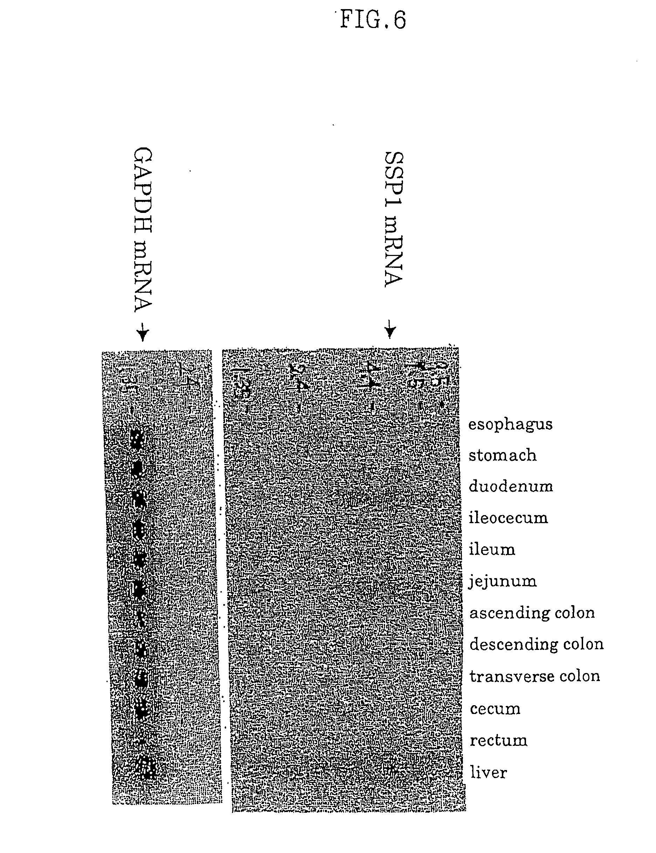

The site specificity of SSP1 mRNA expression in small intestine tissue was analyzed (Fig. 6) by using the cDNA shown by SEQ ID NO: 22 as a probe and hybridizing it to Multiple Tissue Northern Blots (CLONTECH), Multiple Tissue Northern Blots II (CLONTECH), and Multiple Tissue Northern Blots III (CLONTECH). As shown in Fig. 6, SSP1 was expressed specifically in duodenum and jejunum.

### Example 9: Change of SSP1 gene expression depending on intracellular cholesterol level

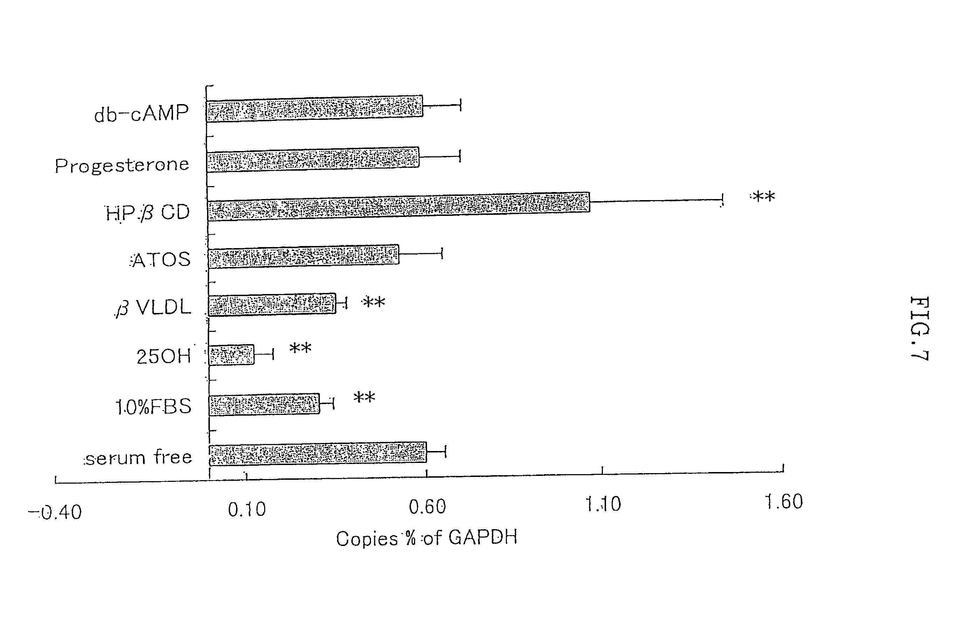

After HepG2 cell was treated as shown in Fig.7 for one day, RNA was extracted to perform a reverse transcription using oligo-dT as a primer. Then, change in the expression amount of SSP1 mRNA was analyzed using the quantification system of expression amount of SSP1 mRNA as shown in Example 6. Specifically, HepG2 cells were cultured on a 24-well plate, and incubated for 24 hours in a serum free D-MEM medium in the presence of 10% FBS (10% FBS), 5 µg/ml of 25-hydroxycholesterol (25-OH), 100 µg-TC/ml of rabbit βVLDL (βVLDL), 1 µM of Atrvastatin (ATOS), 5 mM hydroxypropyl-β-cyclodextrin (HPβCD), 10 µM progesterone (Progesterone) or 100 µM dibutyl-cyclic AMP (db-cAMP). The expression amount of SSP1 mRNA in RNA extracted from these cells was measured as a rate (%) to GAPDH mRNA measured as the internal standard. The results are shown in Fig. 7. As is known from these results, SSP1 mRNA showed decrease in its expression under cholesterol-loading condition (βVLDL, 25OH, 10% FBS) and in contrast, showed increase in its expression under serum free condition or under condition in which cholesterol was further decreased by HPβCD. From these results, it was shown that SSP1 mRNA receives expression control depending on sterol level.

### Example 10: Neuroblastoma-specific expression of SSP2 gene among nerve cell lines and differentiation-dependent induction of SSP2 gene expression in IMR-32 cell, analyzed by TaqMan PCR

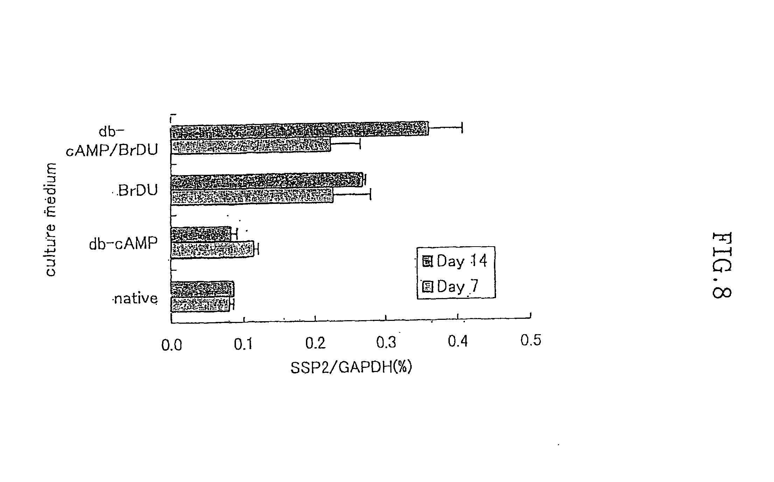

IMR-32 cells were cultured overnight at 2.5 × 10e-5 cells/well (6 well plate), and cultured for 14 days under four different conditions of 1 mM dibutyl cycle AMP (db-cAMP), 10 µM Bromo deoxyuridine (BrDU), 1 mM db-cAMP/10 µM BrDU and no differentiation-inducing agent. RNA was extracted from cells at 7 days and 14 days, then, SSP2 mRNA was quantified by TaqMan PCR. Induction of expression of SSP2 mRNA was recognized in cells which were differentiated to neuron in terms of morphology by 10 µM BrDU (Fig. 8).

### Example 11: Acquisition of a stable SSP1-overexpressing HepG2 cell

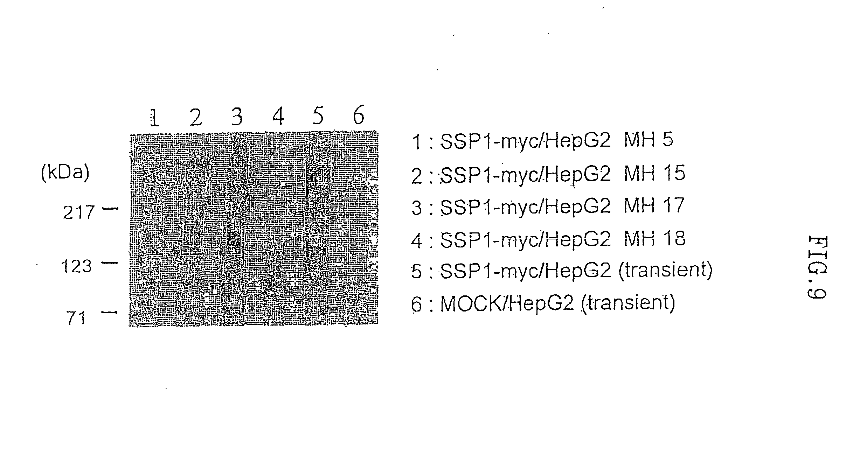

SSP1 gene was subcloned into pcDNA3.1(+)-myc vector (Invitrogen), and the thus obtained expression plasmid for eucaryotic cell having the C-terminal Myc-tag was transfected into HepG2 cells by a lipofection method, and these cells were cultured in the presence of 500 ug/ml G418 to obtain G418-resistant strains. From these strains, 4 stable cell strains highly expressing SSP1 mRNA were obtained using the quantification of expression amount of SSP1 mRNA by TaqMan PCR. The expression amounts of SSP1 mRNA in these cells are shown in the table below. A gene encoding amino acid no. 33-273 of SSP1 protein was subcloned into pET 21(+) vector (Novagen), and using the SSP1 partial protein produced in Escherichia coli as an antigen, a polyclonal antibody was prepared according to an ordinary method. The amino acid sequence of the antigen protein is shown by SEQ ID NO: (antigen AP-1). Using this antibody (AP-1), the expression of SSP1 protein in cell-extract of the four stable SSP1-expressing cell strains was investigated by a Western blotting method. As a result, production of SSP1 protein was recognized corresponding to SSP1 mRNA level of the stable expression cells (Fig. 9 and Table 1).

**[Table 1]**

| Copies % of GAPDH | MH5 | MH15 | MH17 | MH18 | Hep G2 |
|---|---|---|---|---|---|
| mean | 7.9 | 23.9 | 50.9 | 3.2 | 1.4 |
| SD | 0.5 | 2.8 | 3.0 | 0.3 | 0.2 |

### Example 12: Decrease in ApoB lipoprotein secretion in the stable SSP1-overexpressing HepG2 cell

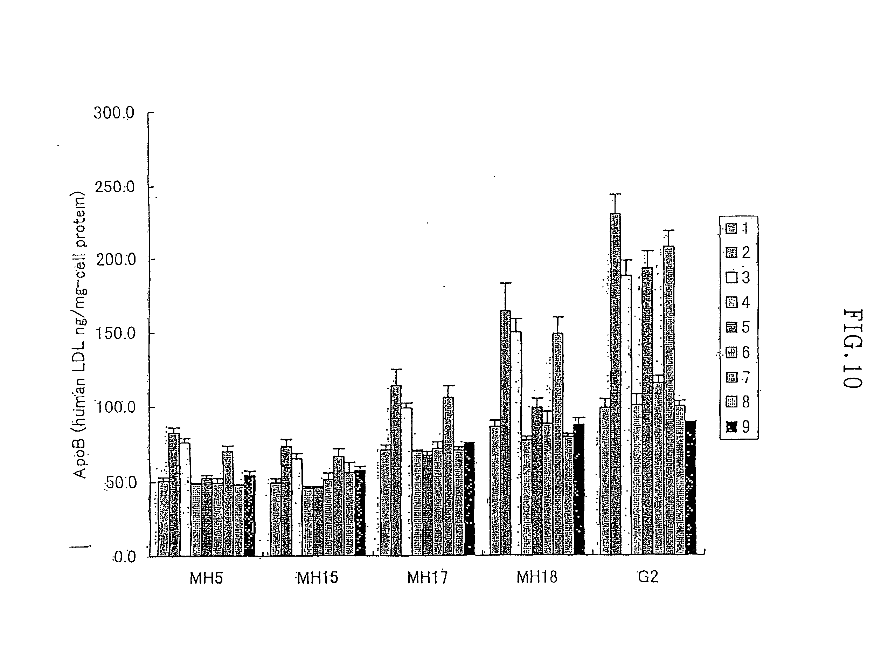

The stable expression cells thus obtained were compared with a parent strain, HepG2 cell for ApoB lipoprotein production for 24 hours. For quantification of ApoB lipoprotein, a sandwich EIA method using an anti-human apoB goat polyclonal antibody and an anti-human apoB mouse monoclonal antibody was carried out. In the quantification, human LDL was used as a standard substance, and the concentration of ApoB lipoprotein secreted in the supernatant was represented as an equivalent human LDL value. As a result, decrease in ApoB production and secretion was observed in the SSP1 stable expression cells. Further, in these cell strains, an activity to promote apoB lipoprotein secretion by addition of oleic acid disappeared (Fig. 10 and Table 2).

**[Table 2]**

| Conditions | |
|---|---|
| 1 | 5% LPDS |
| 2 | 5% LPDS + 25-hydroxycholesterol |
| 3 | 5% LPDS + 25-hydroxycholesterol + CI-976 |
| 4 | 5% LPDS + Atrvastatin |
| 5 | 5% LPDS + oleate (BSA complex) |
| 6 | 5% LPDS + β VLDL |
| 7 | 5% LPDS + 25-hydroxycholesterol β VLDL |
| 8 | Serum free |
| 9 | 5% FBS |

### Example 13: Induction of the site-specific expression of SSP2 mRNA in brain of an alzheimer patient

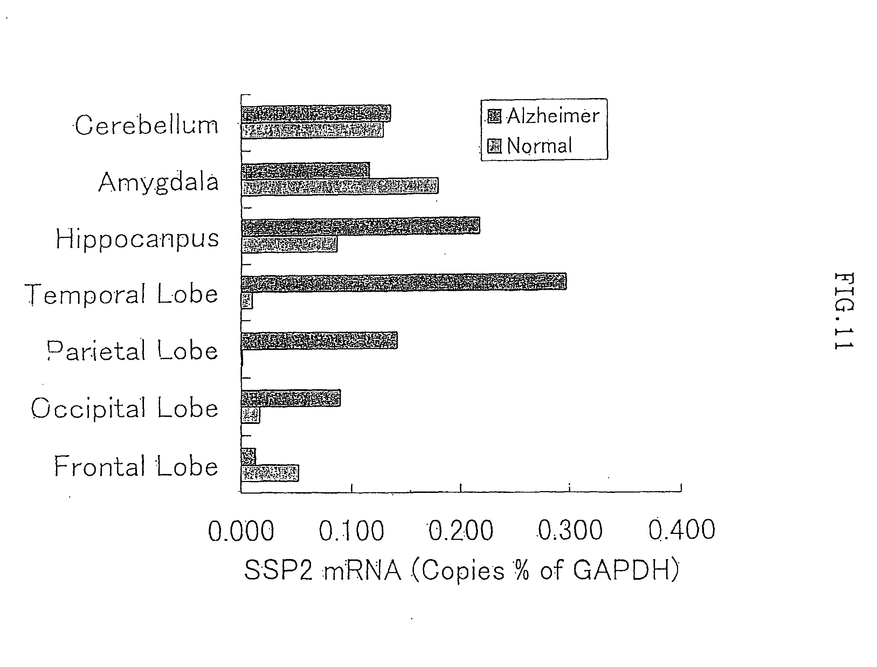

Using TaqMan PCR, the SSP2 mRNA expression level was quantified in each brain regions of a normal person and Alzheimer patient. cDNA derived from each brain regions used for the experiment was purchased from BioChain. As a result of quantification, SSP2 mRNA is expressed in cerebellum, hippocampus, amygdala and the like in a normal person, while it is also expressed in cerebral hemisphere regions, such as parietal lobe, occipital lobe, temporal lobe and the like in an Alzheimer patient (Fig. 11).

### INDUSTRIAL APPLICABILITY

The protein of the present invention has the sterol-sensing ability and plays some important role in an organism such as in metabolism and transportation of intracellular lipids, and thus can be used as a prophylactic and/or therapeutic agent for various diseases associated with diseases of lipid metabolism, for example, diabetic mellitus, obesity, cancer, arterial sclerosis, hyperlipidemia, neuropathy (particularly, arterial sclerosis, hyperlipidemia), and also be used for screening a compound changing the sterol-sensing ability.

## Claims

1. A protein or salt thereof, comprising the same or substantially the same amino acid sequence as that shown by SEQ ID NO: 16.

2. The protein or salt thereof according to claim 1, comprising the same or substantially the same amino acid sequence as that shown by SEQ ID NO: 17.

3. The protein or salt thereof according to claim 1, comprising the same or substantially the same amino acid sequence as that shown by SEQ ID NO: 34.

4. The protein or salt thereof according to claim 1, comprising the same or substantially the same amino acid sequence as that shown by SEQ ID NO: 35.

5. The protein or salt thereof according to claim 1, comprising the same or substantially the same amino acid sequence as that shown by SEQ ID NO: 40.

6. A protein or salt thereof, comprising the same or substantially the same amino acid sequence as that shown by SEQ ID NO: 8, but not comprising the amino acid sequence shown by SEQ ID NO. 38.

7. The protein or salt thereof according to claim 6, comprising the same or substantially the same amino acid sequence as the 22-th to 1332-th residues of the amino acid sequence shown by SEQ ID NO: 9.

8. DNA comprising DNA containing a nucleic acid sequence encoding (i) the protein of claim 1 or (ii) a protein comprising the same or substantially the same amino acid sequence as that shown by SEQ ID NO: 8, but not comprising the amino acid sequence shown by SEQ ID NO. 38.

9. The DNA of claim 8, comprising (i) the nucleic acid sequence of 64-th to 3999-th bases of the nucleic acid sequence shown by SEQ ID NO: 7, (ii) the nucleic acid sequence shown by SEQ ID NO: 15, (iii) the nucleic acid sequence shown by SEQ ID NO: 32, (iv) the nucleic acid sequence shown by SEQ ID NO: 33, or (v) the nucleic acid sequence shown by SEQ ID NO: 41.

10. A recombinant vector comprising DNA of claim 8.

11. A transformant transformed with the recombinant vector of claim 10.

12. A method of producing (i) the protein of claim 1 or (ii) a protein comprising the same or substantially the same amino acid sequence as that shown by SEQ ID NO: 8, but not comprising the amino acid sequence shown by SEQ ID NO. 38, or salt thereof, comprising culturing the transformant of claim 11 to produce the protein.

13. An antibody to (i) the protein of claim 1 or (ii) a protein comprising the same or substantially the same amino acid sequence as that shown by SEQ ID NO: 8, but not comprising the amino acid sequence shown by SEQ ID NO. 38, or salt thereof.

14. A medicine comprising (i) the protein of claim 1 or (ii) a protein comprising the same or substantially the same amino acid sequence as that shown by SEQ ID NO: 8, or salt thereof.

15. A medicine comprising DNA of claim 8.

16. The medicine of claim 14 or 15 which is a prophylactic and/or therapeutic agent for diabetes mellitus, obesity, cancer, arterial sclerosis, hyperlipidemia, neurodegenerative diseases or neuropathy.

17. A diagnostic agent for diabetes mellitus, obesity, cancer, arterial sclerosis, hyperlipidemia, neurodegenerative diseases or neuropathy, comprising DNA of claim 8 or antibody of claim 13.

18. A method of screening a compound or salt thereof promoting or inhibiting the activity of (i) the protein of claim 1 or (ii) a protein comprising the same or substantially the same amino acid sequence as that shown by SEQ ID NO: 8, or salt thereof, **characterized by** use of (i) the protein of claim 1 or (ii) a protein comprising the same or substantially the same amino acid sequence as that shown by SEQ ID NO: 8, or salt thereof.

19. A kit for screening a compound or salt thereof promoting or inhibiting the activity of (i) the protein of claim 1 or (ii) a protein comprising the same or substantially the same amino acid sequence as that shown by SEQ ID NO: 8, or salt thereof, the kit comprising (i) the protein of claim 1 or (ii) a protein comprising the same or substantially the same amino acid sequence as that shown by SEQ ID NO: 8, or salt thereof.

20. A compound or salt thereof promoting or inhibiting the activity of (i) the protein of claim 1 or (ii) a protein comprising the same or substantially the same amino acid sequence as that shown by SEQ ID NO: 8, or salt thereof, obtainable using the screening method of claim 18 or the screening kit of claim 19.

21. A medicine comprising a compound or salt thereof promoting or inhibiting the activity of (i) the protein of claim 1 or (ii) a protein comprising the same or substantially the same amino acid sequence as that shown by SEQ ID NO: 8, or salt thereof, obtainable using the screening method of claim 18 or the screening kit of claim 19.

22. A prophylactic and/or therapeutic agent for diabetes mellitus, obesity, cancer, arterial sclerosis, hyperlipidemia, neurodegenerative diseases or neuropathy, comprising a compound or salt thereof promoting the activity of (i) the protein of claim 1 or (ii) a protein comprising the same or substantially the same amino acid sequence as that shown by SEQ ID NO: 8, or salt thereof, obtainable using the screening method of claim 18 or the screening kit of claim 19.

23. A method of screening a compound or salt thereof changing the sterol-sensing ability of (i) the protein of claim 1 or (ii) a protein comprising the same or substantially the same amino acid sequence as that shown by SEQ ID NO: 8, or salt thereof, **characterized by** use of (i) the protein of claim 1 or (ii) a protein comprising the same or substantially the same amino acid sequence as that shown by SEQ ID NO: 8, or salt thereof.

24. A kit for screening a compound or salt thereof changing the sterol-sensing ability of (i) the protein of claim 1 or (ii) a protein comprising the same or substantially the same amino acid sequence as that shown by SEQ ID NO: 8, or salt thereof, the kit comprising (i) the protein of claim 1 or (ii) a protein comprising the same or substantially the same amino acid sequence as that shown by SEQ ID NO: 8, or salt thereof.

25. A compound or salt thereof changing the sterol-sensing ability of (i) the protein of claim 1 or (ii) a protein comprising the same or substantially the same amino acid sequence as that shown by SEQ ID NO: 8, or salt thereof, obtainable using the screening method of claim 23 or the screening kit of claim 24.

26. A medicine comprising a compound or salt thereof changing the sterol-sensing ability of (i) the protein of claim 1 or (ii) a protein comprising the same or substantially the same amino acid sequence as that shown by SEQ ID NO: 8, or salt thereof, obtainable using the screening method of claim 23 or the screening kit of claim 24.

27. A prophylactic and/or therapeutic agent for diabetes mellitus, obesity, cancer, arterial sclerosis, hyperlipidemia, neurodegenerative diseases or neuropathy, comprising a compound or salt thereof enhancing the sterol-sensing ability of (i) the protein of claim 1 or (ii) a protein comprising the same or substantially the same amino acid sequence as that shown by SEQ ID NO: 8, or salt thereof, obtainable using the screening method of claim 23 or the screening kit of claim 24.

28. A method of quantifying mRNA of (i) the protein of claim 1 or (ii) a protein comprising the same or substantially the same amino acid sequence as that shown by SEQ ID NO: 8, **characterized by** use of DNA of claim 8 or a part thereof.

29. A method of quantifying (i) the protein of claim 1 or (ii) a protein comprising the same or substantially the same amino acid sequence as that shown by SEQ ID NO: 8, **characterized by** use of the antibody of claim 13.

30. A method of diagnosing diseases related to the functions of (i) the protein of claim 1 or (ii) a protein comprising the same or substantially the same amino acid sequence as that shown by SEQ ID NO: 8, **characterized by** use of the quantifying method of claim 28 or 29.

31. A method of screening a compound or salt thereof changing the expression amount of (i) the protein of claim 1 or (ii) a protein comprising the same or substantially the same amino acid sequence as that shown by SEQ ID NO: 8, **characterized by** use of the quantifying method of claim 28.

32. A compound or salt thereof changing the expression amount of (i) the protein of claim 1 or (ii) a protein comprising the same or substantially the same amino acid sequence as that shown by SEQ ID NO: 8, obtainable using the screening method of claim 31.

33. A medicine comprising a compound or salt thereof changing the expression amount of (i) the protein of claim 1 or (ii) a protein comprising the same or substantially the same amino acid sequence as that shown by SEQ ID NO: 8, obtainable using the screening method of claim 31.

34. A prophylactic and/or therapeutic agent for diabetes mellitus, obesity, cancer, arterial sclerosis, hyperlipidemia, neurodegenerative diseases or neuropathy, comprising a compound or salt thereof increasing the expression amount of (i) the protein of claim 1 or (ii) a protein comprising the same or substantially the same amino acid sequence as that shown by SEQ ID NO: 8, obtainable using the screening method of claim 31.

35. A method of screening a compound or salt thereof changing the intracellular amount of (i) the protein of claim 1 or (ii) a protein comprising the same or substantially the same amino acid sequence as that shown by SEQ ID NO: 8, **characterized by** use of the quantifying method of claim 29.

36. A compound or salt thereof changing the intracellular amount of (i) the protein of claim 1 or (ii) a protein comprising the same or substantially the same amino acid sequence as that shown by SEQ ID NO: 8, obtainable using the screening method of claim 35.

37. A medicine comprising a compound or salt thereof changing the intracellular amount of (i) the protein of claim 1 or (ii) a protein comprising the same or substantially the same amino acid sequence as that shown by SEQ ID NO: 8, obtainable using the screening method of claim 35.

38. A prophylactic and/or therapeutic agent for diabetes mellitus, obesity, cancer, arterial sclerosis, hyperlipidemia, neurodegenerative diseases or neuropathy, comprising a compound or salt thereof increasing the intracellular amount of (i) the protein of claim 1 or (ii) a protein comprising the same or substantially the same amino acid sequence as that shown by SEQ ID NO: 8, obtainable using the screening method of claim 35.

39. A prophylactic and/or therapeutic agent for diabetes mellitus, obesity, cancer, arterial sclerosis, hyperlipidemia, neurodegenerative diseases or neuropathy, comprising a compound or salt thereof having an action on an SSD-containing protein to manifest the regulation of intracellular cholesterol transport.

40. A method of preventing and treating diabetes mellitus, obesity, cancer, arterial sclerosis, hyperlipidemia, neurodegenerative diseases or neuropathy, comprising administering to a mammal an effective amount of (i) the protein of claim 1 or (ii) a protein comprising the same or substantially the same amino acid sequence as that shown by SEQ ID NO: 8, or salt thereof.

41. A method of preventing and treating diabetes mellitus, obesity, cancer, arterial sclerosis, hyperlipidemia, neurodegenerative diseases or neuropathy, comprising administering to a mammal an effective amount of DNA of claim 8.

42. A method of preventing and treating diabetes mellitus, obesity, cancer, arterial sclerosis, hyperlipidemia, neurodegenerative diseases or neuropathy, comprising administering to a mammal an effective amount of a compound or salt thereof promoting the activity of (i) the protein of claim 1 or (ii) a protein comprising the same or substantially the same amino acid sequence as that shown by SEQ ID NO: 8, or salt thereof, obtainable using the screening method of claim 18 or the screening kit of claim 19.

43. A method of preventing and treating diabetes mellitus, obesity, cancer, arterial sclerosis, hyperlipidemia, neurodegenerative diseases or neuropathy, comprising administering to a mammal an effective amount of a compound or salt thereof enhancing the sterol-sensing ability of (i) the protein of claim 1 or (ii) a protein comprising the same or substantially the same amino acid sequence as that shown by SEQ ID NO: 8, or salt thereof, obtainable using the screening method of claim 23 or the screening kit of claim 24.

44. A method of preventing and treating diabetes mellitus, obesity, cancer, arterial sclerosis, hyperlipidemia, neurodegenerative diseases or neuropathy, comprising administering to a mammal an effective amount of a compound or salt thereof increasing the expression amount of (i) the protein of claim 1 or (ii) a protein comprising the same or substantially the same amino acid sequence as that shown by SEQ ID NO: 8, obtainable using the screening method of claim 31.

45. A method of preventing and treating diabetes mellitus, obesity, cancer, arterial sclerosis, hyperlipidemia, neurodegenerative diseases or neuropathy, comprising administering to a mammal an effective amount of a compound or salt thereof having an action on an SSD-containing protein to manifest the regulation of intracellular cholesterol transport.

46. Use of (i) the protein of claim 1 or (ii) a protein comprising the same or substantially the same amino acid sequence as that shown by SEQ ID NO: 8, or salt thereof, for producing a prophylactic and/or therapeutic agent for diabetes mellitus, obesity, cancer, arterial sclerosis, hyperlipidemia, neurodegenerative diseases or neuropathy.

47. Use of DNA of claim 8, for producing a prophylactic and/or therapeutic agent for diabetes mellitus, obesity, cancer, arterial sclerosis, hyperlipidemia, neurodegenerative diseases or neuropathy.

48. Use of a compound or salt thereof promoting the activity of (i) the protein of claim 1 or (ii) a protein comprising the same or substantially the same amino acid sequence as that shown by SEQ ID NO: 8, or salt thereof, obtainable using the screening method of claim 18 or the screening kit of claim 19, for producing a prophylactic and/or therapeutic agent for diabetes mellitus, obesity, cancer, arterial sclerosis, hyperlipidemia, neurodegenerative diseases or neuropathy.

49. Use of a compound or salt thereof enhancing the sterol-sensing ability of (i) the protein of claim 1 or (ii) a protein comprising the same or substantially the same amino acid sequence as that shown by SEQ ID NO: 8, or salt thereof, obtainable using the screening method of claim 23 or the screening kit of claim 24, for producing a prophylactic and/or therapeutic agent for diabetes mellitus, obesity, cancer, arterial sclerosis, hyperlipidemia, neurodegenerative diseases or neuropathy.

50. Use of a compound or salt thereof increasing the expression amount of (i) the protein of claim 1 or (ii) a protein comprising the same or substantially the same amino acid sequence as that shown by SEQ ID NO: 8, obtainable using the screening method of claim 31, for producing a prophylactic and/or therapeutic agent for diabetes mellitus, obesity, cancer, arterial sclerosis, hyperlipidemia, neurodegenerative diseases or neuropathy.

51. Use of a compound or salt thereof having an action on an SSD-containing protein to manifest the regulation of intracellular cholesterol transport, for producing a prophylactic and/or therapeutic agent for diabetes mellitus, obesity, cancer, arterial sclerosis, hyperlipidemia, neurodegenerative diseases or neuropathy.
